(19) Europäisches Patentamt
European Patent Office
Office européen des brevets

(11) EP 3 576 757 B1

(12) EUROPEAN PATENT SPECIFICATION

(45) Date of publication and mention of the grant of the patent:
**09.06.2021 Bulletin 2021/23**

(21) Application number: **18747182.6**

(22) Date of filing: **05.02.2018**

(51) Int Cl.:
***A61K 35/15*** *(2015.01)* ***A61K 39/395*** *(2006.01)*
***G01N 33/50*** *(2006.01)*

(86) International application number:
**PCT/US2018/016896**

(87) International publication number:
**WO 2018/145023 (09.08.2018 Gazette 2018/32)**

# (54) METHOD OF PREDICTING RESPONSE TO IMMUNOTHERAPY

VERFAHREN ZUR VORHERSAGE DES ANSPRECHENS AUF EINE IMMUNTHERAPIE

PROCÉDÉ DE PRÉDICTION DE LA RÉPONSE À UNE IMMUNOTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2017 US 201762591057 P**
**06.02.2017 US 201762455337 P**

(43) Date of publication of application:
**11.12.2019 Bulletin 2019/50**

(73) Proprietor: **Novartis AG**
**4056 Basel (CH)**

(72) Inventors:
- **BORDEAUX, Jennifer**
**Carlsbad**
**California 92008 (US)**
- **DAKAPPAGARI, Naveen**
**Carlsbad**
**California 92008 (US)**
- **KIM, Ju Young**
**Carlsbad**
**California 92008 (US)**

(74) Representative: **Nederlandsch Octrooibureau**
**P.O. Box 29720**
**2502 LS The Hague (NL)**

(56) References cited:
**WO-A1-2012/160235** **WO-A1-2016/073760**
**WO-A1-2016/100975** **WO-A2-2016/081947**
**US-A1- 2016 123 964** **US-A1- 2016 222 118**
**US-A1- 2016 362 472**

- **DOUGLAS B. JOHNSON ET AL: "Quantitative Spatial Profiling of PD-1/PD-L1 Interaction and HLA-DR/IDO-1 Predicts Improved Outcomes of anti-PD-1 Therapies in Metastatic Melanoma", CLINICAL CANCER RESEARCH, 18 July 2018 (2018-07-18), XP055671064, US ISSN: 1078-0432, DOI: 10.1158/1078-0432.CCR-18-0309**
- **HERBST et al.: "Predictive correlates of response to the anti-PD-L1 antibody NIPDL-3280A in 1 cancer patients", Nature, vol. 515, 27 November 2014 (2014-11-27), pages 563-567, XP055262130,**
- **OHMATSU et al.: "IL -32 induces indoleamine 2,3-dioxygenase+ CD 1c+ dendritic cells and 1 indoleamine 2,3-dioxygenase+ CD 163+ macrophages: Relevance to mycosis fungoides progression", OncoImmunology, vol. 6, no. 2, 1 January 2017 (2017-01-01) , pages 1-11, XP055617168, DOI: 10.1080/2162402X.2016.1181237**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 576 757 B1

## Description

CROSS-REFERENCE TO RELATED APPLICATIONS

This application claims the benefit of the priority dates of U.S. Provisional Application No. 62/455,337, filed February 6, 2017, and U.S. Provisional Application No. 62/591,057, filed November 27, 2017

BACKGROUND

The present invention relates generally to the field of cancer treatment. All appearances of the term "embodiment" refer to embodiments of the description, unless they are part of the claimed invention. WO 2016/073760 A1 discloses that pre-treatment samples obtained from responding patients showed higher numbers of CD8, PD1, and PD-L1 expressing cells at the invasive tumour margin and inside tumours, with close proximity between PD-1 and PD-L1.

SUMMARY

Disclosed herein, in one aspect, are methods of predicting a likelihood that a cancer patient will respond positively to immunotherapy, the methods comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all HLA-DR positive cells in a field of view of the sample expressing HLA-DR$^+$IDO-1$^+$, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy.

In some embodiments, the cancer patient is likely to respond positively to immunotherapy if either the interaction score is greater than or equal to the first threshold value or the value for PBP is greater than or equal to the second threshold value. In some embodiments, the cancer patient is likely to respond positively to immunotherapy if the interaction score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

Disclosed herein, in another aspect, are methods of predicting a likelihood that a cancer patient will respond positively to immunotherapy, the methods comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR+ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

In some embodiments, the cancer patient is likely to respond positively to immunotherapy if either the spatial proximity score is greater than or equal to the first threshold value or the value for PBP is greater than or equal to the second threshold value. In some embodiments, the cancer patient is likely to respond positively to immunotherapy if the spatial proximity score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, anti-IDO-1 treatment, or combinations thereof. In some embodiments, the total area of the fourth mask and the total area of the sixth mask from step (B) are measured in pixels. In some embodiments, the spatial proximity is assessed on a pixel scale. In some embodiments, each of the fluorescence tags is directed to a specific biomarker. In some embodiments, the plurality of fluorescence tags comprises a first fluorescence tag for PD-1 and a second fluorescence tag for PD-L1. In some embodiments, the margin ranges from about 1 to about 100 pixels. In some embodiments, the proximally located cells expressing PD-L1 are within about 0.5 to about 50 $\mu$m of a plasma membrane of the cells that express PD-1. In some embodiments, the first total area for all cells from each of the selected fields of view from step (A) is measured in pixels. In some embodiments, the normalization factor is a second total area for all non-tumor cells from each of the selected fields of view. In some embodiments, the predetermined factor is $10^4$. In some embodiments, the first threshold value ranges from about 500 to about 5000. In some embodiments, the first threshold value is about 900 plus or minus 100. In some embodiments, the spatial proximity score (SPS) is determined by the following equation:

$$SPS = \frac{A_I}{A_C} \times 10^4$$

wherein $A_I$ is a total interaction area (total area of cells expressing PD-1 and encompassed by dilated fluorescence signals attributable to cells expressing PD-L1) and $A_C$ is the total area of cells that have a capacity to express the PD-1. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

Disclosed herein, in another aspect, are methods of predicting a likelihood that a cancer patient will respond positively to immunotherapy, the methods comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;
for each of the selected fields of view, dilating fluorescence signals attributable to PD-1 by a margin sufficient to encompass proximally located cells expressing PD-L1;
dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and
recording the spatial proximity score;

(B) deriving a value for % biomarker positivity (PBP) for all HLA-DR+ cells present in a field of view expressing $HLA\text{-}DR^+IDO\text{-}1^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express $HLA\text{-}DR^+$;
constructing a third mask of third fluorescence signals representative of all areas present in the field of view, which express $IDO\text{-}1^+$;
combining said first and second masks in a manner that provides a fourth mask comprising fluorescence signals representative of all cells in the field of view, which also express $HLA\text{-}DR^+$;
combining said first and third masks in a manner that provides a fifth mask comprising fluorescence signals representative of all cells in the field of view, which also express $IDO\text{-}1^+$;
combining said fourth and fifth masks in a manner that provides a sixth mask comprising fluorescence signals representative of all cells in the field of view, which express $HLA\text{-}DR^+$ and $IDO\text{-}1^+$;
deriving the value for PBP for all cells present in the field of view expressing $HLA\text{-}DR^+IDO\text{-}1^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

In some embodiments, the cancer patient is likely to respond positively to immunotherapy if either the spatial proximity score is greater than or equal to the first threshold value or the value for PBP is greater than or equal to the second threshold value. In some embodiments, the cancer patient is likely to respond positively to immunotherapy if the spatial proximity score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, anti-IDO-1 treatment, or combinations thereof. In some embodiments, the total area of the fourth mask and the total area of the sixth mask from step (B) are measured in pixels. In some embodiments, the spatial proximity is assessed on a pixel scale. In some embodiments, each of the fluorescence tags is directed to a specific biomarker. In some embodiments, the plurality of fluorescence tags comprises a first fluorescence tag for PD-1 and a second fluorescence tag for PD-L1. In some embodiments, the margin ranges from about 1 to about 100 pixels. In some embodiments, the proximally located cells expressing PD-L1 are within about 0.5 to about 50 μm of a plasma membrane of the cells that express PD-1. In some embodiments, the first total area for all cells from each of the selected fields of view from step (A) is measured in pixels. In some embodiments, the normalization factor is a second total area for all non-tumor cells from each of the selected fields of view. In some embodiments, the predetermined factor is $10^4$. In some embodiments, the first threshold value ranges from about 500 to about 5000. In some embodiments, the first threshold value is about 900 plus or minus 100. In some embodiments, the spatial proximity score (SPS) is determined by the following equation:

$$SPS = \frac{A_I}{A_C} \times 10^4$$

wherein $A_I$ is a total interaction area (total area of cells expressing PD-L1 and encompassed by dilated fluorescence signals attributable to cells expressing PD-1) and $A_C$ is the total area of cells that have a capacity to express the PD-L1. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

Disclosed herein, in another aspect, are methods of predicting a likelihood that a cancer patient will respond positively to immunotherapy, the methods comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy.

In some embodiments, the cancer patient is likely to respond positively to immunotherapy if either the interaction score is greater than or equal to the first threshold value or the value for PBP is greater than or equal to the second threshold value. In some embodiments, the cancer patient is likely to respond positively to immunotherapy if the interaction score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

Disclosed herein, in another aspect, are methods of predicting a likelihood that a cancer patient will respond positively to adjuvant chemotherapy, the methods comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to adjuvant chemotherapy.

In some embodiments, the cancer patient is likely to respond positively to adjuvant chemotherapy if either the interaction score is greater than or equal to the first threshold value or the value for PBP is greater than or equal to the second threshold value. In some embodiments, the cancer patient is likely to respond positively to adjuvant chemotherapy if the interaction score is greater than or equal to the first threshold value and the value for PBP is greater than or equal to the second threshold value. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

Disclosed herein, in another aspect, are methods of treating cancer in a patient in need thereof, the methods comprising

(A) predicting a likelihood that the patient will respond positively to immunotherapy using a method disclosed herein; and

(B) if the patient is likely to respond positively to immunotherapy, then administering immunotherapy to the patient.

Disclosed herein, in another aspect, are methods of treating cancer in a patient in need thereof, the methods comprising

(A) predicting a likelihood that the patient will respond positively to adjuvant chemotherapy using a method disclosed herein; and

(B) if the patient is likely to respond positively to adjuvant chemotherapy, then administering adjuvant chemotherapy to the patient.

Disclosed herein, in another aspect, are methods of selecting a cancer patient who is likely to benefit from an immunotherapy, the methods comprising:

(A) scoring a sample comprising tumor tissue taken from the cancer patient comprising:

using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all HLA-DR positive cells in a field of view of the sample expressing HLA-$DR^+$IDO-$1^+$, and recording the value for PBP; and

(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from the immunotherapy.

In some embodiments, the cancer patient is likely to benefit from the immunotherapy if either the interaction score is greater than or equal to the first threshold value or the value for PBP is greater than or equal to the second threshold value. In some embodiments, the cancer patient is likely to benefit from the immunotherapy if the interaction score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient.

Disclosed herein, in another aspect, are methods of selecting a cancer patient who is likely to benefit from an immunotherapy, the methods comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;

for each of the selected fields of view, dilating fluorescence signals attributable to PD-L1 by a margin sufficient to encompass proximally located cells expressing PD-1;

dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR+ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1$^+$;

deriving the value for PBP for all cells present in the field of view expressing HLA-DR$^+$IDO-1$^+$ by dividing the total area of the sixth mask by the total area of the fourth mask; and

recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to benefit from the immunotherapy.

In some embodiments, the cancer patient is likely to benefit from the immunotherapy if either the spatial proximity score is greater than or equal to the first threshold value or the value for PBP is greater than or equal to the second threshold value. In some embodiments, the cancer patient is likely to benefit from the immunotherapy if the spatial proximity score is greater than or equal to the first threshold value and the value for PBP is greater than or equal to the second threshold value. In some embodiments, the immunotherapy targets the PD-1 and/or PD-L1 axis. In some embodiments, the immunotherapy comprises anti-PD-1 treatment, anti-PD-L1 treatment, anti-IDO-1 treatment, or combinations thereof. In some embodiments, the total area of the fourth mask and the total area of the sixth mask from step (B) are measured in pixels. In some embodiments, the spatial proximity is assessed on a pixel scale. In some embodiments, each of the fluorescence tags is directed to a specific biomarker. In some embodiments, the plurality of fluorescence tags comprises a first fluorescence tag for PD-1 and a second fluorescence tag for PD-L1. In some embodiments, the margin ranges from about 1 to about 100 pixels. In some embodiments, the proximally located cells expressing PD-L1 are within about 0.5 to about 50 μm of a plasma membrane of the cells that express PD-1. In some embodiments, the first total area for all cells from each of the selected fields of view from step (A) is measured in pixels. In some embodiments, the normalization factor is a second total area for all non-tumor cells from each of the selected fields of view. In some embodiments, the predetermined factor is $10^4$. In some embodiments, the first threshold value ranges from about 500 to about 5000. In some embodiments, the first threshold value is about 900 plus or minus 100. In some embodiments, the spatial proximity score (SPS) is determined by the following equation:

$$SPS = \frac{A_I}{A_C} \times 10^4$$

wherein $A_I$ is a total interaction area (total area of cells expressing PD-1 and encompassed by dilated fluorescence signals attributable to cells expressing PD-L1) and $A_C$ is the total area of cells that have a capacity to express the PD-1. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient.

Disclosed herein, in another aspect, are methods of selecting a cancer patient who is likely to benefit from an adjuvant chemotherapy, the methods comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from adjuvant chemotherapy.

In some embodiments, the cancer patient is likely to benefit from adjuvant chemotherapy if either the interaction score is greater than or equal to the first threshold value or the value for PBP is greater than or equal to the second threshold value. In some embodiments, the cancer patient is likely to benefit from adjuvant chemotherapy if the interaction score is greater than or equal to the first threshold value and the value for PBP is greater than or equal to the second threshold value. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

Disclosed herein, in another aspect, are methods of predicting a likelihood that a cancer patient will respond positively to adjuvant chemotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $CD25^+FoxP3^+$, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to adjuvant chemotherapy.

Disclosed herein, in another aspect, are methods of selecting a cancer patient who is likely to benefit from an adjuvant chemotherapy, the methods comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $CD25^+FoxP3^+$, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from adjuvant chemotherapy.

Disclosed herein, in another aspect, are methods of treating cancer in a patient in need thereof, the method comprising

(A) predicting a likelihood that the patient will respond positively to immunotherapy using the method of any one of claims 1-67; and
(B) if the patient is likely to respond positively to immunotherapy, then administering immunotherapy to the patient; or
(C) if the patient is unlikely to respond positively to immunotherapy, then administering to the patient (1) targeted therapy if a BRAF mutation is present, or (2) palliative surgery and/or radiation therapy and best supportive care if BRAF mutation is absent.

In some embodiments, the targeted therapy if a BRAF mutation is present comprises, consists of, or consists essentially of Vemurafenib, dabrafenib, or a combination thereof.

Disclosed herein, in another aspect, are methods of treating cancer in a patient in need thereof, the method comprising

(A) predicting a likelihood that the patient will respond positively to adjuvant chemotherapy using the method of any one of claims 68-81; and
(B) if the patient is likely to respond positively to adjuvant chemotherapy, then administering adjuvant chemotherapy to the patient; or
(C) if the patient is unlikely to respond positively to adjuvant chemotherapy, then proceeding to mutation testing and (1) administering to the patient targeted therapy if the mutation testing is positive or (2) administering to the patient best supportive care if the mutation testing is negative.

In some embodiments, if mutation testing is positive and the patient is EGFR positive, the targeted therapy comprises, consists of, or consists essentially of Erlotinib. In some embodiments, if mutation testing is positive and the patient is ALK positive, the targeted therapy comprises, consists of, or consists essentially of Crizotinib.

Disclosed herein, in another aspect, are methods of predicting a likelihood that a melanoma patient will respond positively to immunotherapy, the method comprising:

(A) deriving a value for % biomarker positivity (PBP) for all HLA-DR positive cells in a field of view of the sample expressing HLA-DR$^+$IDO-1$^+$, and recording the value for PBP; and
(B) comparing the value for PBP to a threshold value;

wherein if the value for PBP is greater than or equal to the threshold value, then the melanoma patient is likely to respond positively to immunotherapy.

Disclosed herein, in another aspect, are methods of predicting a likelihood that a melanoma patient will respond positively to immunotherapy, the method comprising:

(A) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(B) comparing the value for PBP to a threshold value;

wherein if the value for PBP is greater than or equal to the threshold value, then the melanoma patient is likely to respond positively to immunotherapy.

Disclosed herein, in another aspect, are methods of predicting a likelihood that a non-small cell lung cancer patient will respond positively to adjuvant chemotherapy, the method comprising:

(A) deriving a value for % biomarker positivity (PBP) for all T cells (CD4$^+$ or CD8$^+$) in a field of view of the sample expressing CD25$^+$FoxP3$^+$, and recording the value for PBP; and
(B) comparing the value for PBP to a threshold value;

wherein if the value for PBP is greater than or equal to the threshold value, then the non-small cell lung cancer patient is likely to respond positively to adjuvant chemotherapy.

Disclosed herein, in another aspect, are methods of selecting a non-small cell lung cancer patient who is likely to benefit from an adjuvant chemotherapy, the method comprising:

(A) deriving a value for % biomarker positivity (PBP) for all T cells ($CD4^+$ or $CD8^+$) in a field of view of the sample expressing $CD25^+FoxP3^+$, and recording the value for PBP; and
(B) comparing the value for PBP to a threshold value;

wherein if the value for PBP is greater than or equal to the threshold value, then the non-small cell lung cancer patient is likely to benefit from adjuvant chemotherapy.

Disclosed herein, in another aspect, are methods of predicting a likelihood that a non-small cell lung cancer patient will respond positively to adjuvant chemotherapy, the method comprising:

(A) deriving a value for % biomarker positivity (PBP) for all T cells ($CD4^+$ or $CD8^+$) in a field of view of the sample expressing $Ki67^+$, and recording the value for PBP; and
(B) comparing the value for PBP to a threshold value;

wherein if the value for PBP is less than the threshold value, then the non-small cell lung cancer patient is likely to respond positively to adjuvant chemotherapy.

Disclosed herein, in another aspect, are methods of selecting a non-small cell lung cancer patient who is likely to benefit from an adjuvant chemotherapy, the method comprising:

(A) deriving a value for % biomarker positivity (PBP) for all T cells ($CD4^+$ or $CD8^+$) in a field of view of the sample expressing $Ki67^+$, and recording the value for PBP; and
(B) comparing the value for PBP to a threshold value;

wherein if the value for PBP is less than the threshold value, then the non-small cell lung cancer patient is likely to benefit from adjuvant chemotherapy.

Disclosed herein, in another aspect, are methods of predicting a likelihood that a cancer patient will respond positively to adjuvant chemotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $Ki67^+$, and recording the value for PBP; and
(C) comparing the interaction score to a first threshold value and comparing the value for PBP to a second threshold value;

wherein if (1) either the interaction score is greater than or equal to the first threshold value or the value for PBP is less than the second threshold value, or (2) the interaction score is greater than or equal to the first threshold value and the value for PBP is less than the second threshold value, then the cancer patient is likely to respond positively to adjuvant chemotherapy.

Disclosed herein, in another aspect, are methods of selecting a cancer patient who is likely to benefit from an adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $Ki67^+$, and recording the value for PBP; and
(C) comparing the interaction score to a first threshold value and comparing the value for PBP to a second threshold value;

wherein if (1) either the interaction score is greater than or equal to the first threshold value or the value for PBP is less than the second threshold value, or (2) the interaction score is greater than or equal to the first threshold value and the value for PBP is less than the second threshold value, then the cancer patient is likely to benefit from adjuvant chemotherapy.

## BRIEF DESCRIPTION OF THE DRAWINGS

**FIG. 1** shows a non-limiting example of an overview of antibodies and detection reagents used in the preparation of tissue samples for imaging and analysis.
**FIG. 2a** shows a non-limiting example of all nuclei detected with DAPI within an image.
**FIG. 2b** shows a non-limiting example of a dilated binary mask of all cells within the image of **FIG 2a.**

**FIG. 3a** shows a non-limiting example of an image of S100 detected with 488 dye.

**FIG. 3b** shows a non-limiting example of a binary mask of all tumor area within the image of **FIG 3a.**

**FIG. 3c** shows a non-limiting example of a mask of all tumor cells within the image of **FIG 3a.**

**FIG. 3d** shows a non-limiting example of a mask of all non-tumor cells within the image of **FIG 3a.**

**FIG. 4a** shows a non-limiting example of an image of PD-L1 detected with Cy® 5.

**FIG. 4b** shows a non-limiting example of a binary mask of all PD-L1-positive cells within the image of **FIG 4a.**

**FIG. 5a** shows a non-limiting example of an image of PD-1 detected with Cy® 3.5.

**FIG. 5b** shows a non-limiting example of a binary mask of all PD-1-positive non-tumor cells within the image of **FIG 5a.**

**FIG. 6a** shows a non-limiting example of an interaction mask of all PD-L1-positive cells and the nearest neighbor cells.

**FIG. 6b** shows a non-limiting example of an interaction compartment of the PD-1-positive cells in close proximity to the PD-L1-positive cells.

**FIG. 7a** shows a non-limiting example of interaction scores from 24 melanoma patients.

**FIG. 7b** shows a non-limiting example of interaction scores from 142 melanoma patients.

**FIG. 7c** shows a non-limiting example of percent biomarker positivity (PBP) for IDO-$1^+$HLA-$DR^+$ expression in 24 melanoma patients according to response status to anti-PD-1 therapies.

**FIG. 7d** shows a non-limiting example of percent biomarker positivity (PBP) for IDO-$1^+$HLA-$DR^+$CD11$b^-$ expression in 24 melanoma patients according to response status to anti-PD-1 therapies.

**FIG. 8a** shows a non-limiting example of percent biomarker positivity (PBP) combinations for CD11b, HLA-DR, and IDO-1 expression in 24 melanoma patients according to response status to anti-PD-1 therapies. IDO-$1^+$HLA-$DR^+$ PBP demonstrated the most statistically significant difference in expression between responders and non-responders.

**FIG. 8b** shows the ability for PD-1/PD-L1 interaction score, IDO-$1^+$HLA-$DR^+$ PBP or the combination thereof to predict patients who will respond to anti-PD-1 therapies in 24 melanoma patients. The combination correctly identifies the greatest number of responders.

**FIG. 8c** shows the ability for PD-1/PD-L1 interaction score, IDO-$1^+$HLA-$DR^+$ PBP or the combination thereof to predict patients who will respond to anti-PD-1 therapies in 142 melanoma patients. The combination correctly identifies the greatest number of responders.

**FIG. 8d** shows the highest prediction of response to anti-PD-1 therapy for patients positive for both the PD-1/PD-L1 interaction score and IDO-$1^+$HLA-$DR^+$ PBP compared to positive for only PD-1/PD-L1 interaction score or IDO-$1^+$HLA-$DR^+$ PBP in 166 melanoma patients (combining data shown in **FIG. 8b** and **FIG. 8c**).

**FIG. 9a** shows a non-limiting example where the combined test (PD-1/PD-L1 interaction score and IDO-$1^+$HLA-$DR^+$ PBP) is able to identify melanoma patients with statistically significant improved progression free survival (PFS).

**FIG. 9b** shows a non-limiting example where the combined test (PD-1/PD-L1 interaction score and IDO-$1^+$HLA-$DR^+$ PBP) is able to identify melanoma patients with statistically significant improved overall survival (OS).

**FIG. 10** shows a non-limiting example where the combined test (PD-1/PD-L1 interaction score and IDO-$1^+$HLA-$DR^+$ PBP) is able to identify melanoma patients (from combined cohorts: 166 total patients) with statistically significant improved overall survival (OS) compared to patients negative for both the PD-1/PD-L1 interaction score and IDO-$1^+$HLA-$DR^+$PBP.

**FIG. 11** is a flowchart of a process for deriving a value of biomarker positivity, according to an exemplary embodiment.

**FIG. 12** is a flowchart of a process for deriving a value of biomarker positivity, according to a second exemplary embodiment.

**FIG. 13** is a block diagram of a controller configured to derive a value of biomarker positivity, according to an exemplary embodiment.

**FIG. 14** is a flow diagram of the image processing steps used to derive a value of biomarker positivity, according to an exemplary embodiment.

**FIG. 15** is a flowchart of a process for scoring a sample comprising tumor tissue, according to an exemplary embodiment.

**FIG. 16** is a flowchart of a process for scoring a sample comprising tumor tissue, according to a second exemplary embodiment.

**FIG. 17** is a block diagram of a controller configured to score a sample comprising tumor tissue taken from a cancer patient, according to an exemplary embodiment.

**FIG. 18** is a flow diagram of the image processing steps used to score a sample comprising tumor tissue, according to an exemplary embodiment.

**FIG. 19** shows the correlation of IDO-$1^+$HLA-$DR^+$ PBP calculated from alternative sample preparation methods in 29 melanoma samples.

**FIG. 20a** shows a non-limiting example where the PD-L1 tumor expression above 5% is not able to identify melanoma patients with statistically significant improved PFS.

**FIG. 20b** shows a non-limiting example where the PD-L1 tumor expression above 5% is not able to identify melanoma patients with statistically significant improved OS.

**FIG. 21a** shows a non-limiting example of high PD-1/PD-L1 interaction score predicting non-small cell lung cancer (NSCLC) patients with improved OS following adjuvant chemotherapy treatment.

**FIG. 21b** shows a non-limiting example of how high PD-1/PD-L1 interaction score is unable to distinguish non-small cell lung cancer (NSCLC) patients with improved OS without adjuvant chemotherapy treatment.

**FIG. 22** shows a non-limiting example where adjuvant chemotherapy treatment does not improve OS in non-small cell lung cancer (NSCLC) patients.

**FIG. 23a** shows a non-limiting example of where PBP for $CD25^+FoxP3^+$ expression of all T cells ($CD4^+$ or $CD8^+$) in 114 NSCLC patients treated with adjuvant chemotherapy is able to identify patients with statistically significant improved PFS.

**FIG. 23b** shows a non-limiting example of where PBP for $CD25^+FoxP3^+$ expression of all T cells ($CD4^+$ or $CD8^+$) in 114 NSCLC patients treated with adjuvant chemotherapy is able to identify patients with statistically significant improved OS.

**FIG. 24a** shows a non-limiting example of where the combined test (PD-1/PD-L1 interaction score and PBP for $CD25^+FoxP3^+$ expression of all T cells ($CD4^+$ or $CD8^+$)) in 114 NSCLC patients treated with adjuvant chemotherapy is able to identify patients with statistically significant improved PFS.

**FIG. 24b** shows a non-limiting example of where the combined test (PD-1/PD-L1 interaction score and PBP for $CD25^+FoxP3^+$ expression of all T cells ($CD4^+$ or $CD8^+$)) in 114 NSCLC patients treated with adjuvant chemotherapy is able to identify patients with statistically significant improved OS.

**FIG. 25a** shows a non-limiting example of where PBP for $CD25^+FoxP3^+$ expression of all T cells ($CD4^+$ or $CD8^+$) in 328 NSCLC patients who did not receive adjuvant chemotherapy does not demonstrate statistically significant improved PFS.

**FIG. 25b** shows a non-limiting example of where PBP for $CD25^+FoxP3^+$ expression of all T cells ($CD4^+$ or $CD8^+$) in 328 NSCLC patients who did not receive adjuvant chemotherapy does not demonstrate statistically significant improved OS.

**FIG. 26a** shows a non-limiting example of where the combined test (PD-1/PD-L1 interaction score and PBP for $CD25^+FoxP3^+$ expression of all T cells ($CD4^+$ or $CD8^+$)) in 328 NSCLC patients who did not receive adjuvant chemotherapy does not demonstrate statistically significant improved PFS.

**FIG. 26b** shows a non-limiting example of where the combined test (PD-1/PD-L1 interaction score and PBP for $CD25^+FoxP3^+$ expression of all T cells ($CD4^+$ or $CD8^+$)) in 328 NSCLC patients who did not receive adjuvant chemotherapy does not demonstrate statistically significant improved OS.

**FIG. 27a** shows a non-limiting example of where PBP for $Ki67^+$ expression of all T cells ($CD4^+$ or $CD8^+$) in 114 NSCLC patients treated with adjuvant chemotherapy is able to identify patients with statistically significant improved PFS.

**FIG. 27b** shows a non-limiting example of where PBP for $Ki67^+$ expression of all T cells ($CD4^+$ or $CD8^+$) in 114 NSCLC patients treated with adjuvant chemotherapy is able to identify patients with statistically significant improved OS.

**FIG. 28a** shows a non-limiting example of where the combined test (PD-1/PD-L1 interaction score and PBP for $Ki67^+$ expression of all T cells ($CD4^+$ or $CD8^+$)) in 114 NSCLC patients treated with adjuvant chemotherapy is able to identify patients with statistically significant improved PFS.

**FIG. 28b** shows a non-limiting example of where the combined test (PD-1/PD-L1 interaction score and PBP for $Ki67^+$ expression of all T cells ($CD4^+$ or $CD8^+$)) in 114 NSCLC patients treated with adjuvant chemotherapy is able to identify patients with statistically significant improved OS.

**FIG. 29a** shows a non-limiting example of where PBP for $Ki67^+$ expression of all T cells ($CD4^+$ or $CD8^+$) in 328 NSCLC patients who did not receive adjuvant chemotherapy does not demonstrate statistically significant improved PFS.

**FIG. 29b** shows a non-limiting example of where PBP for $Ki67^+$ expression of all T cells ($CD4^+$ or $CD8^+$) in 328 NSCLC patients who did not receive adjuvant chemotherapy does not demonstrate statistically significant improved OS.

**FIG. 30a** shows a non-limiting example of where the combined test (PD-1/PD-L1 interaction score and PBP for $Ki67^+$ expression of all T cells ($CD4^+$ or $CD8^+$)) in 328 NSCLC patients who did not receive adjuvant chemotherapy does not demonstrate statistically significant improved PFS.

**FIG. 30b** shows a non-limiting example of where the combined test (PD-1/PD-L1 interaction score and PBP for $Ki67^+$ expression of all T cells ($CD4^+$ or $CD8^+$)) in 328 NSCLC patients who did not receive adjuvant chemotherapy does not demonstrate statistically significant improved OS.

## DETAILED DESCRIPTION

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein. All methods described herein can be performed in any suitable order unless otherwise indicated herein or otherwise clearly contradicted by context. The use of any and all examples, or exemplary language (e.g., "such as") provided herein, is intended merely to better illuminate the embodiments and does not pose a limitation on the scope of the claims unless otherwise stated. No language in the specification should be construed as indicating any non-claimed element as essential.

The term "treating" or "treatment" refers to administering a therapy in an amount, manner, or mode effective to improve a condition, symptom, or parameter associated with a disorder or to prevent progression of a disorder, to either a statistically significant degree or to a degree detectable to one skilled in the art. An effective amount, manner, or mode can vary depending on the subject and may be tailored to the patient.

The term "best supportive care" refers to care that focuses on relieving symptoms of cancer and/or cancer treatment to help the patient feel more comfortable.

Tumors may be classified based on their immune contexture as "hot" (inflamed) or "cold" (non-inflamed). While patients bearing hot tumors may be expected to respond to certain immunotherapies and potentially live longer than patients bearing cold tumors, it has been previously unclear to those skilled in the art as to which biomarkers correlate with response and survival.

To address this issue, some embodiments of the methods described herein aid in the identification of cancer patients who will respond to one or more immunotherapies via expression of immune exhaustion biomarkers (*e.g.,* PD-1 and PD-L1) and cancer patients who will not respond (*i.e.,* non-responders) via the presence of cell types known to cause immune suppression (*e.g.,* CD11b, HLA-DR, IDO-1, ARG1) or highly proliferating tumor cells devoid of MHC class I expression (*e.g.,* $Ki67^+$, $B2M^-$). In some embodiments, the methods described herein comprise use of multiplex immunohistochemistry assays (*e.g.,* multiplex FIHC assays) based on specific immune suppression or activation signatures.

In one aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all cells in a field of view of the sample expressing a biomarker of interest, and recording the value for PBP; and (C) comparing the score to a first threshold value and comparing the value for PBP to a second threshold value; wherein if (1) either the score is greater than or equal to the first threshold value or the value for PBP is greater than or equal to the second threshold value, or (2) the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all cells in a field of view of the sample expressing a biomarker of interest, and recording the value for PBP; and (C) comparing the score to a first threshold value and comparing the value for PBP to a second threshold value; wherein if either the score is greater than or equal to the first threshold value or the value for PBP is greater than or equal to the second threshold value, then the cancer patient is likely to respond positively to immunotherapy.

In one aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all cells in a field of view of the sample expressing a biomarker of interest, and recording the value for PBP; and (C) comparing the score to a first threshold value and comparing the value for PBP to a second threshold value; wherein if the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all HLA-DR positive ($HLA\text{-}DR^+$) cells in a field of view of the sample expressing $HLA\text{-}DR^+IDO\text{-}1^+$, and recording the value for PBP; and (C) comparing the score to a first threshold value and comparing the value for PBP to a second threshold value; wherein if (1) either the score is greater than or equal to the first threshold value or the value for PBP is greater than or equal to the second threshold value, or (2) the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all HLA-DR positive ($HLA\text{-}DR^+$) cells in a field of view of the sample expressing HLA-DR$^+$IDO-1$^+$, and recording the value for PBP; and (C) comparing the score to a first threshold value and comparing the value for PBP to a second threshold value; wherein if either the score is greater than or equal to the first threshold value or the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all HLA-DR positive (HLA-DR$^+$) cells in a field of view of the sample expressing HLA-DR$^+$IDO-1$^+$, and recording the value for PBP; and (C) comparing the score to a first threshold value and comparing the value for PBP to a second threshold value; wherein if the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing CD25$^+$FoxP3$^+$, and recording the value for PBP; and (C) comparing the score to a first threshold value and comparing the value for PBP to a second threshold value; wherein if (1) either the score is greater than or equal to the first threshold value or the value for PBP is greater than or equal to the second threshold value or (2) the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing CD25$^+$FoxP3$^+$, and recording the value for PBP; and (C) comparing the score to a first threshold value and comparing the value for PBP to a second threshold value; wherein if the score is greater than or equal to the first threshold value or the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing CD25$^+$FoxP3$^+$, and recording the value for PBP; and (C) comparing the score to a first threshold value and comparing the value for PBP to a second threshold value; wherein if the score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing Ki67$^+$, and recording the value for PBP; and (C) comparing the score to a first threshold value and comparing the value for PBP to a second threshold value; wherein if (1) either the score is greater than or equal to the first threshold value or the value for PBP is less than the second threshold value or (2) the score is greater than or equal to the first threshold value and the value for PBP is less than the second threshold value, then the cancer patient is likely to respond positively to immunotherapy..

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing Ki67$^+$, and recording the value for PBP; and (C) comparing the score to a first threshold value and comparing the value for PBP to a second threshold value; wherein if the score is greater than or equal to the first threshold value or the value for PBP is less than the second threshold value, then the cancer patient is likely to respond positively to immunotherapy.

In another aspect, provided herein are methods of predicting a likelihood that a cancer patient will respond positively to immunotherapy, the method comprising (A) scoring a sample comprising tumor tissue taken from the cancer patient; (B) deriving a value for % biomarker positivity (PBP) for all T cells in a field of view of the sample expressing Ki67$^+$, and recording the value for PBP; and (C) comparing the score to a first threshold value and comparing the value for PBP to a second threshold value; wherein if the score is greater than or equal to the first threshold value and the value for PBP is less than the second threshold value, then the cancer patient is likely to respond positively to immunotherapy.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all HLA-DR positive cells in a field of view of the sample expressing HLA-DR+IDO-1+, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a metastatic melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all HLA-DR positive cells in a field of view of the sample expressing HLA-DR+IDO-1+, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a metastatic melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all HLA-DR positive cells in a field of view of the sample expressing HLA-DR+IDO-1+, and recording the value for PBP; and (C) comparing the interaction score to a first threshold value and comparing the value for PBP to a second threshold value; wherein if the interaction score is greater than or equal to the first threshold value and the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a metastatic melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all biomarker positive cells in a field of view of the sample, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all biomarker positive cells in a field of view of the sample, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof.In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all biomarker positive cells in a field of view of the sample, and recording the value for PBP; and (C) comparing the interaction score to a first threshold value and comparing the value for PBP to a second threshold value; wherein if the interaction score is greater than or equal to the first threshold value and the value for PBP is greater than or equal to the second threshold value, then the cancer patient is likely to respond positively to immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8 and a combination of two or more thereof.In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if the interaction score is greater than or equal to the first threshold value and the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to immunotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-Ll; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if the interaction score is greater than or equal to the first threshold value and the value for PBP is greater than or equal to the second threshold value, then the cancer patient is likely to respond positively to immunotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;

for each of the selected fields of view, dilating fluorescence signals attributable to PD-L1 by a margin sufficient to encompass proximally located cells expressing PD-1;

dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if the spatial proximity score is greater than or equal to the first threshold value and the value for PBP is greater than or equal to the second threshold value, then the cancer patient is likely to respond positively to immunotherapy.

In another aspect, provided herein are methods of predicting a likelihood that a melanoma cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1 $^+$;

deriving the value for PBP for all cells present in the field of view expressing HLA-DR$^+$IDO-1$^+$ by dividing the total area of the sixth mask by the total area of the fourth mask; and

recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

In another aspect, provided herein are methods of predicting a likelihood that a melanoma cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;

for each of the selected fields of view, dilating fluorescence signals attributable to PD-L1 by a margin sufficient to encompass proximally located cells expressing PD-1;

dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1 $^+$;

deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to respond positively to immunotherapy.

In another aspect, provided herein are methods of predicting a likelihood that a melanoma cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if the spatial proximity score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;
for each of the selected fields of view, dilating fluorescence signals attributable to PD-1 by a margin sufficient to encompass proximally located cells expressing PD-L1;
dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;
for each of the selected fields of view, dilating fluorescence signals attributable to PD-1 by a margin sufficient to encompass proximally located cells expressing PD-L1;
dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$

by dividing the total area of the sixth mask by the total area of the fourth mask; and
recording the value for PBP; and

(C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to respond positively to immunotherapy.

In another aspect, provided herein are methods of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;
for each of the selected fields of view, dilating fluorescence signals attributable to PD-1 by a margin sufficient to encompass proximally located cells expressing PD-L1;
dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if the spatial proximity score is greater than or equal to the first threshold value and the value for PBP is greater than or equal to the second threshold value, then the cancer patient is likely to respond positively to immunotherapy.

In another aspect, provided herein are methods of selecting a cancer patient who is likely to benefit from an immunotherapy, the method comprising (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all HLA-DR positive cells in a field of view of the sample expressing HLA-DR$^+$IDO-1$^+$, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from the immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In another aspect, provided herein are methods of selecting a cancer patient who is likely to benefit from an immunotherapy, the method comprising (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all HLA-DR positive cells in a field of view of the sample expressing HLA-DR$^+$IDO-1$^+$, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from the immunotherapy. In some embodiments, the immunotherapy targets the PD-1 and/or PD-L1 axis. In some embodiments, the immunotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In another aspect, provided herein are methods of selecting a cancer patient who is likely to benefit from an immunotherapy, the method comprising (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all HLA-DR positive cells in a field of view of the sample expressing HLA-DR$^+$IDO-1$^+$, and recording the value for PBP; and (C) comparing the interaction score to a first threshold value and comparing the value for PBP to a second threshold value; wherein if the interaction score is greater than or equal to the first threshold value and the value for PBP is greater than or equal to the second threshold value, then the cancer patient is likely to benefit from the immunotherapy. In some embodiments, the immunotherapy targets the PD-1 and/or PD-L1 axis. In some embodiments, the immunotherapy comprises anti-PD-1 treatment, anti-PD-LI treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 900 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In another aspect, provided herein are methods of selecting a cancer patient who is likely to benefit from an immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;

for each of the selected fields of view, dilating fluorescence signals attributable to PD-L1 by a margin sufficient to encompass proximally located cells expressing PD-1;

dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1$^+$;

deriving the value for PBP for all cells present in the field of view expressing HLA-DR$^+$IDO-1$^+$ by dividing the total area of the sixth mask by the total area of the fourth mask; and

recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to benefit from the immunotherapy.

In another aspect, provided herein are methods of selecting a cancer patient who is likely to benefit from an immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;

for each of the selected fields of view, dilating fluorescence signals attributable to PD-L1 by a margin sufficient to encompass proximally located cells expressing PD-1;

dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to benefit from the immunotherapy.

In another aspect, provided herein are methods of selecting a cancer patient who is likely to benefit from an immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if the spatial proximity score is greater than or equal to the first threshold value and the value for PBP is greater than or equal to the second threshold value, then the cancer patient is likely to benefit from the immunotherapy.

In another aspect, provided herein are methods of predicting a likelihood that a cancer patient will respond positively to adjuvant chemotherapy, the method comprising (A) scoring a sample comprising tumor tissue taken from the cancer patient; (B) deriving a value for % biomarker positivity (PBP) for all cells in a field of view of the sample expressing a biomarker of interest, and recording the value for PBP; and (C) comparing the score to a first threshold value and comparing the value for PBP to a second threshold value; wherein if either the score is greater than or equal to the first threshold value or the value for PBP is greater than or equal to the second threshold value, then the cancer patient is likely to respond positively to adjuvant chemotherapy.

In some embodiments, the method of predicting a likelihood that a cancer patient will respond positively to adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all biomarker positive cells in a field of view of the sample, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to adjuvant chemotherapy. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

In some embodiments, the method of predicting a likelihood that a cancer patient will respond positively to adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 $\mu$m to about 50 $\mu$m. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a cancer patient will respond positively to adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of predicting a likelihood that a non-small cell lung cancer patient will respond positively to adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%.

In some embodiments, the method of predicting a likelihood that a non-small cell lung cancer patient will respond positively to adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to respond positively to adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%.

In another aspect, provided herein are methods of selecting a cancer patient who is likely to benefit from adjuvant chemotherapy, the method comprising (A) scoring a sample comprising tumor tissue taken from the cancer patient; (B) deriving a value for % biomarker positivity (PBP) for all cells in a field of view of the sample expressing a biomarker of interest, and recording the value for PBP; and (C) comparing the score to a first threshold value and comparing the value for PBP to a second threshold value; wherein if either the score is greater than or equal to the first threshold value or the value for PBP is greater than or equal to the second threshold value, then the cancer patient is likely to benefit from the adjuvant chemotherapy.

In some embodiments, the method of selecting a cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all biomarker positive cells in a field of view of the sample, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from the adjuvant chemotherapy. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof. In some embodiments, the biomarker of step (B) is selected from CD11b, HLA-DR, Arginase 1, IDO-1, CD25, FoxP3, Granzyme B, CD56, CD68, CD163, Ki67, Tim3, Lag3, and a combination of two or more thereof.

In some embodiments, the method of selecting a cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of selecting a cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%. In some embodiments, the cancer patient is a melanoma cancer patient or a lung cancer patient. In some embodiments, the cancer patient is a melanoma cancer patient. In some embodiments, the cancer patient is a non-small cell lung cancer patient.

In some embodiments, the method of selecting a non-small cell lung cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, CD4, CD8, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%.

In some embodiments, the method of selecting a non-small cell lung cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for all cells in a field of view of the sample expressing a biomarker of interest, and recording the value for PBP; and (C) comparing the interaction score to a first threshold value and comparing the value for PBP to a second threshold value; wherein if either the interaction score is greater than or equal to the first threshold value or the value for PBP is greater than or equal to the second threshold value, then the cancer patient is likely to benefit from adjuvant chemotherapy; and the biomarker of interest comprises the expression and/or lack of expression of CD11b, HLA-DR, Arginase 1, IDO-1, CD25, FoxP3, Granzyme B, CD56, CD68, CD163, Ki67, Tim3, Lag3, or a combination of two or more thereof. In some embodiments, the adjuvant chemotherapy targets the PD-1 and/or PD-L1 axis. In some embodiments, the adjuvant chemotherapy comprises anti-PD-1 treatment, anti-PD-L1 treatment, IDO-1 inhibiting treatment, or combinations thereof. In some embodiments, the spatial proximity is assessed on a pixel scale. In some embodiments, the spatial proximity between the at least one pair of cells ranges from about 1 pixel to about 100 pixels. In some embodiments, the spatial proximity between the at least one pair of cells ranges from about 0.5 μm to about 50 μm. In some embodiments, the first threshold value ranges from about 500 to about 5000. In some embodiments, the first threshold value is about 700 plus or minus 100. In some embodiments, the second threshold value ranges from about 2% to about 10%. In some embodiments, the second threshold value is about 5% plus or minus 1%.

In some embodiments, the method of selecting a non-small cell lung cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for for all T cells in a field of view of the sample expressing $CD25^+FoxP3^+$, and recording the value for PBP; and (C) comparing the interaction score to a first threshold value and comparing the value for PBP to a second threshold value; wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from adjuvant chemotherapy.

In some embodiments, the method of selecting a non-small cell lung cancer patient who is likely to benefit from adjuvant chemotherapy comprises (A) scoring a sample comprising tumor tissue taken from the cancer patient comprising using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score; (B) deriving a value for % biomarker positivity (PBP) for for all T cells in a field of view of the sample expressing $Ki67^+$, and recording the value for PBP; and (C) comparing the interaction score to a first threshold value and comparing the value for PBP to a second threshold value; wherein if (1) either the interaction score is greater than or equal to the first threshold value or the value for PBP is less than the second threshold value, or (2) the interaction score is greater than or equal to the first threshold value and the value for PBP is less than the second threshold value, then the cancer patient is likely to benefit from adjuvant chemotherapy.

In another aspect, provided herein are methods of treating cancer in a patient in need thereof, the method comprising (A) predicting a likelihood that the patient will respond positively to immunotherapy using the methods disclosed herein; and (B) if the patient is likely to respond positively to immunotherapy, then administering immunotherapy to the patient.

In another aspect, provided herein are methods of treating cancer in a patient in need thereof, the method comprising (A) predicting a likelihood that the patient will respond positively to adjuvant chemotherapy using the methods disclosed herein; and (B) if the patient is likely to respond positively to adjuvant chemotherapy, then administering adjuvant chemotherapy to the patient.

% Biomarker Positivity

The methods disclosed herein may comprise deriving a value for % biomarker positivity (PBP) for all cells or, optionally, one or more subsets thereof, present in a field of view of a tissue sample taken from a cancer patient.

In some embodiments, the sample may be stained using a plurality of fluorescence tags with affinity for specific biomarkers. A digital image of the stained sample may be obtained, and the image further analyzed based on the location of the fluorescence tags. Rather than whole-image analysis, fields of view may be prioritized based on the number of cells that express a first biomarker of interest. A predetermined number of fields of view may then be further analyzed for fluorescence signals. In some embodiments, the use of four different types of fluorescence tags generates an image of fluorescence signals corresponding to a first biomarker of interest and an image of fluorescence signals corresponding a second biomarker of interest as well as to an image of fluorescence signals corresponding a biomarker expressed by all cells and an image of fluorescence signals corresponding a subset biomarker (*e.g.,* a biomarker expressed by tumor cells). In further embodiments, the images of fluorescence signals are manipulated to generate one or more masks of fluorescence signals corresponding to cells within the image. In some embodiments, the one or more masks of fluorescence signals comprise one or more selected from the group consisting of a mask of all cells within the image, a mask of all cells that express the subset biomarker (*e.g.,* all tumor cells) within the image, a mask of all cells that do not express the subset biomarker (*e.g.,* all non-tumor cells) within the image, a mask of all cells expressing a first biomarker of interest within the image, and a mask of all cells expressing a second biomarker of interest within the image. The areas of these masks may be used to derive a value for PBP as desired. In some embodiments, a value for PBP for all cells expressing the subset biomarker is derived. In some embodiments, a value for PBP for a first subset of all cells expressing the subset biomarker and the first biomarker of interest is derived. In some embodiments, a value for PBP for a second subset of all cells expressing the subset biomarker and the second biomarker of interest is derived. In some embodiments, a value for PBP for a second subset of all cells that express the second biomarker of interest but do not express the subset biomarker is derived.

Accordingly, in some embodiments, deriving a value for % biomarker positivity (PBP) for all cells or, optionally, one or more subsets thereof present in a field of view, comprises:

(i) generating an image of first fluorescence signals representative of nuclei of all cells present in a field of view, and dilating the first fluorescence signals to a diameter of that of an entire cell to construct a first mask of all cells present in the field of view;

(ii) constructing a second mask of second fluorescence signals representative of all areas present in the field of view, which express a subset biomarker;
(iii) optionally, constructing a third mask of third fluorescence signals representative of all areas present in the field of view, which express a first biomarker of interest;
(iv) combining said first and second masks in a manner that provides a fourth mask comprising fluorescence signals representative of all cells in the field of view, which also express the subset biomarker;
(v) optionally, combining said first and third masks in a manner that provides a fifth mask comprising fluorescence signals representative of all cells in the field of view, which also express the first biomarker of interest;
(vi) deriving a value for PBP for all cells expressing the subset biomarker by dividing the total area of the fourth mask by the total area of the first mask;
(vii) optionally, combining said fourth and fifth masks in a manner that provides a sixth mask comprising fluorescence signals representative of all cells in the field of view, which

(a) express the subset biomarker and the first biomarker of interest; or
(b) express the subset biomarker in the absence of the first biomarker of interest;
and

(viii) optionally, deriving a value for PBP for the first subset of all cells which either (a) express the subset biomarker and the first biomarker of interest or (b) express the subset biomarker in the absence of the first biomarker of interest, by dividing the total area of the sixth mask by the total area of the fourth mask.

In some embodiments, the optional steps are not performed. In some embodiments, the total area is measured in pixels. In some embodiments, the total area of the fourth mask and the total area of the first mask are each measured in pixels. In some embodiments, the total area of the sixth mask and the total area of the fourth mask are each measured in pixels. In some embodiments, the total area of the first mask, the total area of the fourth mask, and the total area of the sixth mask are each measured in pixels. In some embodiments, a pixel is 0.5 $\mu$m wide.

In some embodiments, deriving a value for % biomarker positivity (PBP) for all cells or, optionally, one or more subsets thereof present in a field of view, comprises:

(i) generating an image of first fluorescence signals representative of nuclei of all cells present in a field of view, and dilating the first fluorescence signals to a diameter of that of an entire cell to construct a first mask of all cells present in the field of view;
(ii) constructing a second mask of second fluorescence signals representative of all areas present in the field of view, which express a subset biomarker;
(iii) optionally, constructing a third mask of third fluorescence signals representative of all areas present in the field of view, which express a first biomarker of interest;
(iv) combining said first and second masks in a manner that provides a fourth mask comprising fluorescence signals representative of all cells in the field of view, which also express the subset biomarker;
(v) optionally, combining said first and third masks in a manner that provides a fifth mask comprising fluorescence signals representative of all cells in the field of view, which also express the first biomarker of interest;
(vi) deriving a value for PBP for all cells expressing the subset biomarker by dividing the total area of the fourth mask by the total area of the first mask;
(vii) optionally, combining said fourth and fifth masks in a manner that provides a sixth mask comprising fluorescence signals representative of all cells in the field of view, which express the subset biomarker and the first biomarker of interest; and
(viii) optionally, deriving a value for PBP for the first subset of all cells expressing the subset biomarker and the first biomarker of interest by dividing the total area of the sixth mask by the total area of the fourth mask.

In some embodiments, the optional steps are not performed. In some embodiments, the total area is measured in pixels. In some embodiments, the total area of the fourth mask and the total area of the first mask are each measured in pixels. In some embodiments, the total area of the sixth mask and the total area of the fourth mask are each measured in pixels. In some embodiments, the total area of the first mask, the total area of the fourth mask, and the total area of the sixth mask are each measured in pixels. In some embodiments, a pixel is 0.5 $\mu$m wide.

In some embodiments, deriving a value for % biomarker positivity (PBP) for all cells or, optionally, one or more subsets thereof present in a field of view, comprises:

(i) generating an image of first fluorescence signals representative of nuclei of all cells present in a field of view, and dilating the first fluorescence signals to a diameter of that of an entire cell to construct a first mask of all cells present in the field of view;
(ii) constructing a second mask of second fluorescence signals representative of all areas present in the field of view, which express a subset biomarker;
(iii) constructing a third mask of third fluorescence signals representative of all areas present in the field of view, which express a first biomarker of interest;
(iv) combining said first and second masks in a manner that provides a fourth mask comprising fluorescence signals representative of all cells in the field of view, which also express the subset biomarker;

(v) combining said first and third masks in a manner that provides a fifth mask comprising fluorescence signals representative of a first subset of all cells in the field of view, which also express the first biomarker of interest;

(vi) deriving a value for PBP for all cells expressing the subset biomarker by dividing the total area of the fourth mask by the total area of the first mask;

(vii) combining said fourth and fifth masks in a manner that provides a sixth mask comprising fluorescence signals representative of the first subset of all cells in the field of view, which express the subset biomarker and the first biomarker of interest; and

(viii) deriving a value for PBP for the first subset of all cells expressing the subset biomarker and the first biomarker of interest by dividing the total area of the sixth mask by the total area of the fourth mask.

In some embodiments, the total area is measured in pixels. In some embodiments, the total area of the fourth mask and the total area of the first mask are each measured in pixels. In some embodiments, the total area of the sixth mask and the total area of the fourth mask are each measured in pixels. In some embodiments, the total area of the first mask, the total area of the fourth mask, and the total area of the sixth mask are each measured in pixels. In some embodiments, a pixel is 0.5 $\mu$m wide.

In some embodiments, deriving a value for % biomarker positivity (PBP) for all cells or, optionally, one or more subsets thereof present in a field of view, further comprises:

(ix) constructing a seventh mask of fourth fluorescence signals representative of all areas present in the field of view, which express a second biomarker of interest;

(x) combining said first and seventh masks in a manner that provides an eighth mask comprising fluorescence signals representative of a second subset of all cells in the field of view, which also express the second biomarker of interest;

(xi) combining said fourth and eighth masks in a manner that provides a ninth mask comprising fluorescence signals representative of the second subset of all cells in the field of view, which express the subset biomarker and the second biomarker of interest; and

(xii) deriving a value for PBP for the second subset of all cells expressing the subset biomarker and the second biomarker of interest by dividing the total area of the ninth mask by the total area of the fourth mask.

In some embodiments, the total area is measured in pixels. In some embodiments, the total area of the ninth mask and the total area of the fourth mask are each measured in pixels. In some embodiments, a pixel is 0.5 $\mu$m wide.

In some embodiments, deriving a value for % biomarker positivity (PBP) for all cells or, optionally, one or more subsets thereof present in a field of view, further comprises:

(ix) constructing a seventh mask of fourth fluorescence signals representative of all areas present in the field of view, which express a second biomarker of interest;

(x) subtracting said second mask from said first mask in a manner that provides an eighth mask comprising fluorescence signals representative of all cells that do not express the subset biomarker in the field of view;

(xi) combining said seventh and eighth masks in a manner that provides a ninth mask comprising fluorescence signals representative of all cells that express the second biomarker of interest but do not express the subset biomarker in the field of view; and

(xii) deriving a value for PBP for all cells that express the second biomarker of interest but do not express the subset biomarker by dividing the total area of the ninth mask by the total area of the eighth mask.

In some embodiments, deriving a value for % biomarker positivity (PBP) for all cells or, optionally, one or more subsets thereof present in a field of view, further comprises:

(ix) constructing a seventh mask of fourth fluorescence signals representative of all areas present in the field of view, which express a second biomarker of interest;

(x) combining said sixth and seventh masks in a manner that provides an eighth mask comprising fluorescence signals representative of all cells that

(a) express the subset biomarker, the first biomarker of interest, and the second biomarker of interest in the field of view;

(b) express the subset biomarker and the first biomarker of interest in the absence of the second biomarker of interest in the field of view; or

(c) express the subset biomarker and the second biomarker of interest in the absence of the first biomarker of interest in the field of view; and

(xii) deriving a value for PBP for all cells that express the first biomarker of interest or the second biomarker of interest, or a combination thereof, as well as the subset biomarker, by dividing the total area of the eighth mask by the total area of the fourth mask.

In some embodiments, deriving a value for % biomarker positivity (PBP) for all cells or, optionally, one or more subsets thereof present in a field of view, further comprises additional cycles of steps analogous to steps (ix), (x), and (xii) with respect to one or more additional biomarkers of interest (*e.g.,* a third biomarker of interest).

In some embodiments, the total area is measured in pixels. In some embodiments, the total area of the ninth mask and the total area of the eighth mask are each measured in pixels. In some embodiments, a pixel is 0.5 μm wide.

In some embodiments, a subset of cells identified by a subset biomarker and a non-subset of cells corresponds to tumor cells and non-tumor cells, respectively or vice versa. In some embodiments, a subset of cells identified by a subset biomarker and a non-subset of cells corresponds to viable cells and non-viable cells, respectively or vice versa. In some embodiments, a subset of cells identified by a subset biomarker is a subset of viable cells and a non-subset of cells consists of the viable cells not included in the subset of viable cells. In some embodiments, a subset of cells identified by a subset biomarker and a non-subset of cells corresponds to T cells and non-T cells, respectively or vice versa. In some embodiments, a subset of cells identified by a subset biomarker and a non-subset of cells corresponds to myeloid cells and non-myeloid cells, respectively or vice versa.

In some embodiments, the first subset of all the cells in the field of view comprises tumor cells. In some embodiments, the first subset of all the cells in the field of view comprises non-tumor cells. In some embodiments, the first subset of all the cells in the field of view comprises non-tumor and tumor cells. In some embodiments, the first subset of all the cells in the field of view comprises HLA-DR$^+$ cells.

In some embodiments, the first subset of all the cells in the field of view comprises T-cells. In some embodiments, the T-cells express CD3. In some embodiments, the T-cells express CD8. In some embodiments, the T-cells express CD4.

In some embodiments, the first biomarker of interest comprises a biomarker selected from the group consisting of CD11b, CD33, HLA-DR, IDO-1, ARG1, granzyme B, B2M, PD-L1, PD-L2, B7-H3, B7-H4, HLA-DR, Galectin 9, CD80, CD86, 4.1BBL, ICOSL, CD40, OX40L, IDO-1, GITRL, PD-1, TIM3, LAG3, 41BB, OX40, CTLA-4, CD40L, CD28, GITR, ICOS, CD28, CD3, CD4, CD8, FoxP3, CD25, CD16, CD56, CD68, CD163, CD80, and CD86. In some embodiments, the first biomarker of interest comprises a biomarker selected from the group consisting of PD-L1, PD-L2, B7-H3, B7-H4, HLA-DR, Galectin 9, CD80, CD86, 4.1BBL, ICOSL, CD40, OX40L, IDO-1, GITRL, ICOS, CD28, CD4, CD8, FoxP3, CD25, CD16, CD56, CD68, CD163, CD80, and CD86. In some embodiments, the first biomarker of interest comprises a biomarker selected from the group consisting of PD-L1, PD-L2, B7-H3, B7-H4, HLA-DR, Galectin 9, CD80, CD86, 4.1BBL, ICOSL, CD40, OX40L, IDO-1, and GITRL. In some embodiments, the first biomarker of interest comprises a biomarker selected from the group consisting of PD-L1, Galectin 9, and MHC. In some embodiments, the first biomarker of interest comprises PD-L1. In some embodiments, the first biomarker of interest comprises IDO-1.

In some embodiments, the second biomarker of interest comprises a biomarker selected from PD-1, TIM-3, and TCR. In some embodiments, the second biomarker of interest comprises PD-1.

In some embodiments, the first biomarker of interest and the second biomarker of interest are different from each other and comprise a biomarker selected from the group consisting of PD-L1, PD-L2, B7-H3, B7-H4, HLA-DR, Galectin 9, CD80, CD86, 4.1BBL, ICOSL, CD40, OX40L, IDO-1, GITRL, ICOS, CD28, CD3, CD4, CD8, FoxP3, CD25, CD16, CD56, CD68, CD163, CD80, and CD86. In some embodiments, the first biomarker of interest and the second biomarker of interest are different from each other and comprise a biomarker selected from the group consisting of CD11b, CD33, HLA-DR, IDO-1, ARG1, granzyme B, B2M, PD-L1, PD-L2, B7-H3, B7-H4, HLA-DR, Galectin 9, CD80, CD86, 4.1BBL, ICOSL, CD40, OX40L, IDO-1, GITRL, ICOS, CD28, CD3, CD4, CD8, FoxP3, CD25, CD16, CD56, CD68, CD163, CD80, and CD86.

In some embodiments, the first biomarker of interest comprises a biomarker selected from the group consisting of PD-L1, PD-L2, B7-H3, B7-H4, HLA-DR, Galectin 9, CD80, CD86, 4.1BBL, ICOSL, CD40, OX40L, IDO-1, GITRL, ICOS, CD28, CD4, CD8, FoxP3, CD25, CD16, CD56, CD68, CD163, CD80, and CD86; and the second biomarker of interest comprises a biomarker selected from PD-1, TIM-3, and TCR. In some embodiments, the first biomarker of interest comprises PD-L1 and the second biomarker of interest comprises PD-1. In some embodiments, the first biomarker of interest comprises PD-L1 and the second biomarker of interest comprises CD80. In some embodiments, the first biomarker of interest comprises CTLA-4 and the second biomarker of interest comprises CD80. In some embodiments, the first biomarker of interest comprises PD-L2 and the second biomarker of interest comprises PD-1. In some embodiments, the first biomarker of interest comprises CTLA-4 and the second biomarker of interest comprises CD86. In some embodiments, the first biomarker of interest comprises LAG-3 and the second biomarker of interest comprises HLA-DR. In some embodiments, the first biomarker of interest comprises TIM-3 and the second biomarker of interest comprises Galectin 9. In some embodiments, the first biomarker of interest comprises 41BB and the second biomarker of interest comprises 4.1BBL. In some embodiments, the first biomarker of interest comprises OX40 and the second biomarker of interest comprises OX40L. In some embodiments, the first biomarker of interest comprises CD40 and the second biomarker of interest comprises CD40L. In some embodiments, the first biomarker of interest comprises ICOS and the second biomarker of interest comprises ICOSL.

In some embodiments, the first biomarker of interest comprises GITR and the second biomarker of interest comprises GITRL. In some embodiments, the first biomarker of interest comprises HLA-DR and the second biomarker of interest comprises TCR. In some embodiments, the first biomarker of interest comprises CD25 and the second biomarker of interest comprises FoxP3. In some embodiments, the first biomarker of interest comprises CD4 and the second biomarker of interest comprises CD8. In some embodiments, the first biomarker of interest comprises CD3 and the second biomarker of interest comprises PD-1. In some embodiments, the first biomarker of interest comprises CD56 and the second biomarker of interest comprises CD16. In some embodiments, the first biomarker of interest comprises HLA-DR and the second biomarker of interest comprises IDO-1. In some embodiments, the first biomarker of interest comprises CD33 and the second biomarker of interest comprises ARG1.

In some embodiments, the subset biomarker is only expressed in tumor cells. In some embodiments, the subset biomarker is expressed only in non-tumor cells. In some embodiments, the subset biomarker is expressed in T-cells. In some embodiments, the subset biomarker comprises CD3. In some embodiments, the subset biomarker comprises CD19. In some embodiments, the subset biomarker comprises CD45. In some embodiments, the subset biomarker is expressed in myeloid cells. In some embodiments, the subset biomarker comprises CD11b. In some embodiments, the subset biomarker comprises HLA-DR.

In some embodiments, the first biomarker of interest comprises Ki67 and the first subset of all the cells in the field of view comprises CD8 positive cells.

In some embodiments, the subset biomarker comprises HLA-DR and the first biomarker of interest comprises IDO-1.

In some embodiments, the methods disclosed herein comprise deriving a value for % biomarker positivity (PBP) for all HLA-$DR^+$ cells present in a field of view expressing HLA-$DR^+$IDO-$1^+$, comprising:

(i) generating an image of first fluorescence signals representative of nuclei of all cells present in a field of view, and dilating the first fluorescence signals to a diameter of that of an entire cell to construct a first mask of all cells present in the field of view;
(ii) constructing a second mask of second fluorescence signals representative of all areas present in the field of view, which express HLA-$DR^+$;
(iii) constructing a third mask of third fluorescence signals representative of all areas present in the field of view, which express IDO-$1^+$;
(iv) combining said first and second masks in a manner that provides a fourth mask comprising fluorescence signals representative of all cells in the field of view, which also express HLA-$DR^+$;
(v) combining said first and third masks in a manner that provides a fifth mask comprising fluorescence signals representative of all cells in the field of view, which also express IDO-$1^+$;
(vi) combining said fourth and fifth masks in a manner that provides a sixth mask comprising fluorescence signals representative of all cells in the field of view, which express HLA-$DR^+$ and IDO-$1^+$; and
(vii) deriving the value for PBP for all cells present in the field of view expressing HLA-$DR^+$IDO-$1^+$ by dividing the total area of the sixth mask by the total area of the fourth mask.

In some embodiments, the methods disclosed herein comprise deriving a value for % biomarker positivity (PBP) for all tumor cells present in a field of view, comprising:

(i) generating an image of first fluorescence signals representative of nuclei of all cells present in a field of view, and dilating the first fluorescence signals to a diameter of that of an entire cell to construct a first mask of all cells present in the field of view;
(ii) constructing a second mask of second fluorescence signals representative of all areas present in the field of view, which express a tumor biomarker;
(iii) combining said first and second masks in a manner that provides a third mask comprising fluorescence signals representative of all tumor cells in the field of view;
(iv) constructing a fourth mask of third fluorescence signals representative of all areas present in the field of view, which express a biomarker of interest;
(v) combining said third and fourth masks in a manner that provides a fifth mask comprising fluorescence signals representative of all tumor cells in the field of view, which also express the biomarker of interest; and
(vi) deriving a value for PBP for all tumor cells expressing the biomarker of interest by dividing the total area of the fifth mask by the total area of the third mask.

In some embodiments, the total area is measured in pixels. In some embodiments, the total area of the fifth mask and the total area of the third mask are each measured in pixels. In some embodiments, a pixel is 0.5 $\mu$m wide. In some embodiments, the biomarker of interest comprises a biomarker selected from the group consisting of PD-L1, Galectin 9, and MHC. In some embodiments, the biomarker of interest comprises PD-L1. In some embodiments, the biomarker of interest comprises Galectin 9. In some embodiments, the biomarker of interest comprises MHC. In some embodiments, the field of view further comprises non-tumor cells. In some embodiments, the non-tumor cells comprise immune cells and stromal cells.

In some embodiments, the methods disclosed herein comprise deriving a value for % biomarker positivity (PBP) for all non-tumor cells present in a field of view, comprising:

(i) generating an image of first fluorescence signals representative of nuclei of all cells present in a field of view, and dilating the first fluorescence signals to a diameter of that of an entire cell to construct a first mask of all cells present in the field of view;
(ii) constructing a second mask of second fluorescence signals representative of all areas present in the field of view, which express a tumor biomarker;
(iii) subtracting said second mask from said first mask in a manner that provides a third mask comprising fluorescence signals representative of all non-tumor cells in the field of view;
(iv) constructing a fourth mask of fluorescence signals representative of all areas present in the field of view, which express a biomarker of interest;
(v) combining said third and fourth masks in a manner that provides a fifth mask comprising fluorescence signals representative of all non-tumor cells in the field of view, which also express the biomarker of interest; and
(vi) deriving a value for PBP for all non-tumor cells expressing the biomarker of interest by dividing the total area of the fifth mask by the total area of the third mask.

In some embodiments, the total area is measured in pixels. In some embodiments, the total area of the fifth mask and the total area of the third mask are each measured in pixels. In some embodiments, a pixel is 0.5 $\mu$m wide. In some embodiments, the biomarker of interest comprises a biomarker selected from the group consisting of PD-L1, PD-L2, B7-H3, B7-H4, HLA-DR, CD80, CD86, 4.1BBL, ICOSL, CD40, OX40L, IDO-1, GITRL, PD-1, TIM3, LAG3, 41BB, OX40, CTLA-4, CD40L, CD28, GITR, ICOS, CD28, CD3, CD4, CD8, FoxP3, CD25, CD16, CD56, CD68, CD163, CD80, and CD86, or a combination of two or more thereof. In some embodiments, the biomarker of interest comprises a biomarker selected from the group consisting of PD-L1, PD-L2, B7-H3, B7-H4, HLA-DR, CD80, CD86, 4.1BBL, ICOSL, CD40, OX40L, IDO-1, GITRL, PD-1, TIM3, LAG3, 41BB, OX40, CTLA-4, CD40L, CD28, GITR, ICOS, CD28, CD3, CD4, CD8, FoxP3, CD25, CD16, CD56, CD68, CD163, CD80, and CD86. In some embodiments, the biomarker of interest comprises a biomarker selected from the group consisting of PD-L1, PD-L2, B7-H3, B7-H4, HLA-DR, CD80, CD86, 4.1BBL, ICOSL, CD40, OX40L, IDO-1, GITRL, PD-1, TIM3, LAG3, 41BB, OX40, CTLA-4, CD40L, CD28, GITR, ICOS, CD28, CD4, CD8, FoxP3, CD25, CD16, CD56, CD68, CD163, CD80, and CD86. In some embodiments, the biomarker of interest comprises a biomarker selected from the group consisting of PD-L1, PD-L2, B7-H3, B7-H4, HLA-DR, CD80, CD86, 4.1BBL, ICOSL, CD40, OX40L, IDO-1, GITRL, PD-1, TIM3, LAG3, 41BB, OX40, CTLA-4, CD40L, CD28, GITR, ICOS, and CD28. In some embodiments, the biomarker of interest comprises PD-L1. In some embodiments, the biomarker of interest comprises PD-1. In some embodiments, the non-tumor cells comprise immune cells and stromal cells.

In some embodiments, the value for PBP is compared to a threshold PBP. In some embodiments, the threshold PBP ranges from about 2% to about 10%. In some embodiments, the threshold PBP ranges from about 2% to about 9%. In some embodiments, the threshold PBP ranges from about 2% to about 8%. In some embodiments, the threshold PBP ranges from about 2% to about 7%. In some embodiments, the threshold PBP ranges from about 2% to about 6%. In some embodiments, the threshold PBP ranges from about 3% to about 10%. In some embodiments, the threshold PBP ranges from about 3% to about 9%. In some embodiments, the threshold PBP ranges from about 3% to about 8%. In some embodiments, the threshold PBP ranges from about 3% to about 7%. In some embodiments, the threshold PBP ranges from about 3% to about 6%. In some embodiments, the threshold PBP ranges from about 4% to about 10%. In some embodiments, the threshold PBP ranges from about 4% to about 9%. In some embodiments, the threshold PBP ranges from about 4% to about 8%. In some embodiments, the threshold PBP ranges from about 4% to about 7%. In some embodiments, the threshold PBP ranges from about 4% to about 6%. In some embodiments, the threshold PBP ranges from about 5% to about 10%, or from about 10% to about 15%, or from about 15% to about 20%, or from about 10% to about 20%, or from about 20% to about 25%, or from about 20% to about 30%, or from about 25% to about 30%, or from about 30% to about 35%, or from about 30% to about 40%, or from about 35% to about 40%, or from about 40% to about 45%, or from about 40% to about 50%. In some embodiments, the threshold PBP is about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50%, including increments therein. In some embodiments, the threshold PBP is about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50% including increments therein, plus or minus 1%.

**FIG. 11** is a flowchart depicting the steps of one embodiment of deriving a value for % biomarker positivity (PBP) or a PBP score. In step 1101, image data is obtained and in step 1102, the image data is unmixed such that data specific to various types of fluorescence signals are separated into different channels. In step 1103, data from a first channel is used to generate a mask of all cells. In step 1104, data from a second channel is used to generate a mask of the area in a field of view that expresses a subset biomarker, for example, this subset mask may be a mask of a tumor area present in a field of view. In step 1105, the all cell mask and the subset mask (*e.g.,* a tumor area mask) are combined to generate a mask of all subset cells.

In some embodiments, a subset of cells identified by a subset biomarker and a non-subset of cells corresponds to tumor cells and non-tumor cells, respectively or vice versa. In some embodiments, a subset of cells identified by a subset biomarker and a non-subset of cells corresponds to viable cells and non-viable cells, respectively or vice versa. In some embodiments, a subset of cells identified by a subset biomarker is a subset of viable cells and a non-subset of cells consists of the viable cells not included in the subset of viable cells. In some embodiments, a subset of cells identified by a subset biomarker and a non-subset of cells corresponds to T cells and non-T cells, respectively or vice versa. In some embodiments, a subset of cells identified by a subset biomarker and a non-subset of cells corresponds to myeloid cells and non-myeloid cells, respectively or vice versa. In some embodiments, a subset of cells identified by a subset biomarker and a non-subset of cells corresponds to HLA-DR$^+$ cells and HLA-DR$^-$ cells, respectively or vice versa.

In certain embodiments, combining the all cell mask and the subset mask may identify all tumor cells and/or all non-tumor cells. The process may be carried out on only a selected type of cell of interest, for example, only tumor cells or only non-tumor cells. The process may also be directed to an analysis of both. In step 1106, data from a third channel is used to generate a mask of all cells that are positive for a biomarker (based on fluorescence signals representing the presence of a fluorescent tag with an affinity for binding to the particular biomarker of interest). In steps 1107 and 1108, the biomarker mask generated in step 1106 is combined with the subset cell mask generated in step 1105. Step 1107 combines the biomarker mask with the subset cell mask in a first manner, to generate a mask of all subset cells that are positive for the biomarker. Step 1108 combines the biomarker mask with the subset cell mask in a second manner, to generate a mask of subset cells that are not positive for the biomarker. One or both of steps 1107 and 1108 may be performed according the various embodiments of the method. In step 1109/1110, a PBP score is calculated by dividing the area of the subset cells of interest (*e.g.,* the subset cells that are positive for the biomarker identified by the mask in step 1107 or the subset cells that are not positive for the biomarker identified by the mask in step 1108) by the total area of all subset cells. One or both of steps 1109 and 1110 may be performed according the various embodiments of the method.

**FIG. 12** is a flowchart depicting the steps of a second embodiment of a method for deriving a value for % biomarker positivity (PBP). In step 1201, image data is obtained and in step 1202, the image data is unmixed such that data specific to various types of fluorescence signals are separated into different channels. In step 1203, data from a first channel is used to generate a mask of all cells. In step 1204, data from a second channel is used to generate a mask of all cells that are positive for a biomarker (based on fluorescence signals representing the presence of a fluorescent tag with an affinity for binding to the particular biomarker of interest). In step 1205, a PBP score is calculated by dividing the area of the cells that are positive for the biomarker (which is identified by the mask created in step 1204) by the total area of all cells of interest (from step 1203). The process of **FIG. 12** may be carried out separately or concurrently with the method depicted in **FIG. 11.** In other words, a PBP score may be calculated for all cells, all tumor cells, and all non-tumor cells, or any combination thereof, may combining the methods of **FIGS. 11** and **12.**

In the methods disclosed herein, the manipulation of the digital images may be carried out by a computing system comprising a controller, such as the controller illustrated in the block diagram of **FIG. 13,** according to an exemplary embodiment. Controller 200 is shown to include a communications interface 202 and a processing circuit 204. Communications interface 202 may include wired or wireless interfaces (*e.g.,* jacks, antennas, transmitters, receivers, transceivers, wire terminals, etc.) for conducting data communications with various systems, devices, or networks. For example, communications interface 202 may include an Ethernet card and port for sending and receiving data via an Ethernet-based communications network and/or a WiFi transceiver for communicating via a wireless communications network. Communications interface 202 may be configured to communicate via local area networks or wide area networks (*e.g.,* the Internet, a building WAN, etc.) and may use a variety of communications protocols (*e.g.,* BACnet, IP, LON, etc.).

Communications interface 202 may be a network interface configured to facilitate electronic data communications between controller 200 and various external systems or devices (*e.g.,* imaging device 102). For example, controller 200 may receive imaging data for the selected fields of view from the imaging device 102, to analyze the data and calculate the spatial proximity score (SPS).

Still referring to **FIG. 13,** processing circuit 204 is shown to include a processor 206 and memory 208. Processor 206 may be a general purpose or specific purpose processor, an application specific integrated circuit (ASIC), one or more field programmable gate arrays (FPGAs), a group of processing components, or other suitable processing components. Processor 506 may be configured to execute computer code or instructions stored in memory 508 or received from other computer readable media (*e.g.,* CDROM, network storage, a remote server, etc.).

Memory 208 may include one or more devices (*e.g.*, memory units, memory devices, storage devices, etc.) for storing data and/or computer code for completing and/or facilitating the various processes described in the present disclosure. Memory 208 may include random access memory (RAM), read-only memory (ROM), hard drive storage, temporary storage, non-volatile memory, flash memory, optical memory, or any other suitable memory for storing software objects and/or computer instructions. Memory 208 may include database components, object code components, script components, or any other type of information structure for supporting the various activities and information structures described in the present disclosure. Memory 508 may be communicably connected to processor 206 via processing circuit 204 and may include computer code for executing (*e.g.*, by processor 206) one or more processes described herein.

Still referring to **FIG. 13,** controller 200 is shown to receive input from an imaging device 102. The imaging device acquires all of the imaging data and records it, along with all of the meta-data which describes it. The imaging device will then serialize the data into a stream which can be read by controller 200. The data stream may accommodate any binary data stream type such as the file system, a RDBM or direct TCP/IP communications. For use of the data stream, controller 200 is shown to include a spectral unmixer 210. The spectral unmixer 210 may receive image data from an imaging device 102 on which it performs spectral unmixing to unmix an image presenting various wavelengths into individual, discrete channels for each band of wavelengths. For example, the image data may be "unmixed" into separate channels for each of the various fluorophores used to identify cells or proteins of interest in the tissue sample. The fluorophore, by way of example only, may be one or more of the group consisting of DAPI, Cy® 2, Cy® 3, Cy® 3,5, Cy® 5, FITC, TRITC, Alexa Fluor® 488, Alexa Fluor® 555, Alexa Fluor® 594, and Texas Red. In one example, one of the channels may include image data that falls within a predetermined band surrounding a wavelength of 461 nm (the maximum emission wavelength for DAPI), to identify nuclei in the image. Other channels may include image data for different wavelengths to identify different portions of the tissue sample using different fluorophores.

Controller 200 is also shown to include various maskers, such as cell masker 212, subset area masker 216, and biomarker masker 222. These, or other maskers that may be included in the controller 200 in other embodiments, are used to receive an unmixed signal from the spectral unmixer 210 and create a mask for the particular cell or area of interest, dependent on the fluorophore used to identify certain features of interest in the tissue sample. To create a mask, the maskers (such as cell masker 212, subset area masker 216, and biomarker masker 222) receive image data related to an intensity of each pixel in the field of view. Pixel intensity is directly proportional to the amount of fluorescence emitted by the sample, which in turn, is directly proportional to the amount of protein biomarker in the sample (when using a fluorophore to identify a particular biomarker). An absolute threshold may be set based on the values which exist in the image pixels. All the pixels which are greater than or equal to the threshold value will be mapped to 1.0, or "on", and all other pixels will be mapped to 0.0, or "off." In this way, a binary mask is created to identify the cell or tissue portion of interest in the field of view. In other embodiments, a mask is created using a lower bound wherein all pixels with an intensity at or above a lower bound are accepted and used as the pixel value for the mask. If the intensity is below the lower bound, the pixel value is set to 0.0, or "off".

In the example flow diagram for masking shown in **FIG. 14,** it is shown that the DAPI and 488 dye channels (or other fluorophore for identifying nuclei and tumor areas, respectively) use the lower bound protocol (steps 1410, 1412, 1420, 1422), while the Cy5 channel (or other fluorophore for identifying a biomarker of interest) uses a threshold value protocol (step 1430), for providing the mask output. In association with the lower bound protocol, there is also a histogram step to determine the lower bound. In particular, *histogram threshold* (step 1412, 1422) produces a threshold of an input image but uses a sliding scale to determine the point at which the thresholding occurs. The inputs are the current image and a user defined *threshold percentage.* The latter is used to determine at what percent of the total intensity the threshold level should be set. Firstly, the intensity of every pixel is summed into a total intensity. The *threshold percentage* is multiplied by this total intensity to obtain a cut-off sum. Finally, all pixels are grouped by intensity (in a histogram) and their intensities summed from lowest to highest (bin by bin) until the cut-off sum is achieved. The last highest pixel intensity visited in the process is the threshold for the current image. All pixels with intensities greater than that value have their intensities set to maximum while all others are set to the minimum.

The steps identified as steps 1414, 1416, 1424, 1426, 1428, 1432, 1434, 1436 in **FIG. 14** represent intermediary steps that occur in the initial maskers, such as cell masker 212 (steps 1414, 1416), subset area masker 216 (steps 1424, 1426, 1428), and biomarker masker 222(steps 1432, 1434, 1436). These steps are defined as follows:

*Dilate* increases the area of brightest regions in an image. Two inputs are need for *dilate.* The first is the implicit current image and the second is the number of *iterations* to dilate. It is assumed that only binary images are used for the first input. The procedure will operate on continuous images, but the output will not be a valid *dilate.* The *dilate* process begins by first finding the maximum pixel intensity in the image. Subsequently, each pixel in the image is examined once. If the pixel under investigation has intensity equal to the maximum intensity, that pixel will be drawn in the output image as a circle with *iterations* radius and centered on the original pixel. All pixels in that circle will have intensity equal to the maximum intensity. All other pixels are copied into the output image without modification.

The *fill holes* procedure will fill "empty" regions of an image with pixels at maximum intensity. These empty regions are those that have a minimum intensity and whose pixel area (*size*) is that specified by the user. The current image and *size* are the two inputs required. Like *dilate* this procedure should only be applied to binary images.

*Erode* processes images in the same fashion as *dilate.* All functionality is the same as *dilate* except that the first step determines the minimum intensity in the image, only pixels matching that lowest intensity are altered, and the circles used to bloom the found minimum intensity pixels are filled with the lowest intensity value. Like *dilate* this procedure should only be applied to binary images.

*Remove Objects.* Two inputs are expected: the current image and *object size. Remove objects* is the opposite of the *fill holes* procedure. Any regions containing only pixels with maximum intensity filling an area less than the input *object size* will be set to minimum intensity and thusly "removed." This procedure should only be applied to binary images; application to continuous images may produce unexpected results.

The output at steps 1418, 1429, and 1438 are the resultant cell mask, subset mask (or, in this particular example, tumor area mask), and biomarker cell mask, respectively. FIG. 14 further depicts the combinations of these resultant masks to obtain the relevant area information for the PBP score. These combinations are described below with reference to the combination maskers of the controller 200, depicted in **FIG. 13.**

Controller 200 is shown to include combination maskers, such as subset cell masker 218, non-subset cell masker 220, and combination masker 230. In some embodiments, the subset cells identified by masker 218 and the non-subset cells identified by masker 220 are tumor cells and non-tumor cells, respectively. Subset cell masker performs an *And* operation, as shown at step 1452 in **FIG. 14,** to combine the output of the cell masker 212 (representative of all cells in the image) with the output of the subset area masker 216. Accordingly, subset cell masker generates a mask of all subset cells in the image. This same combination, using an *Out* operation performed by non-subset cell masker 220 as shown at step 1454 in **FIG. 14,** generates a mask of all non-subset cells in the sample image.

Combination masker 230 is configured to combine two input masks. As depicted in **FIG. 14,** combination masker 230 combines the biomarker mask with one of the subset cell mask (from subset cell masker 218) or non-subset cell mask (from non-subset cell masker 220), or both biomarker mask + subset mask and biomarker mask + non-subset mask. The dotted lines represent that either one or both of the cell masks may be combined with the biomarker mask at combination masker 230. The result of the combination masker 230 is a mask representative of all subset cells that are positive for the biomarker and/or all non-subset cells that are positive for the biomarker. The combination masker 230 may combine the masks in an alternate manner such that the result of the combination masker 230 is a mask representative of subset cells that are not positive for the biomarker (biomarker negative). If the cells of interest are not specifically related to the subset, for example tumor or non-tumor, but rather, all cells, then the biomarker positive mask is not combined with any additional mask and passes through the combination masker 230 without modification.

To calculate the % biomarker positivity (PBP) score, the area of the selected subset cell *(e.g.,* all, tumor, or non-tumor) biomarker positive mask or biomarker negative mask (in which case the score represents biomarker negativity) is determined in pixels at the area evaluator 232. The total area of all the selected cells (positive and negative for the biomarker), is determined in pixels at the area evaluator 232. The dotted lines terminating at area evaluator 232 indicate that the total area inputs may be one or more of the all cell mask, the subset cell mask, and the non-subset cell mask, to be calculated separately. A percent biomarker positivity score is determined at the positivity calculator 236. In one embodiment, the BPB score is calculated by dividing the area of the selected cell biomarker positive mask from area evaluator 232 by the area of the all selected cell mask from area evaluator 232, and multiplying 100. In one embodiment, the equation executed by the interaction calculator 236 is:

$$BPB = \frac{A_P}{A_A} \times 100$$

wherein $A_P$ is a biomarker positive area for the selected type of subset cell (*e.g.,* all, tumor, or non-tumor) and $A_A$ is the total area of all cells of the selected cell type (all, tumor, non-tumor). Similarly, $A_N$ could replace $A_P$ in the above equation, wherein $A_N$ is a biomarker negative area for the selected type of cell (*e.g.,* all, tumor, or non-tumor), to determine a score representative of percent biomarker negativity for the type of subset cell.

The *And* procedure is modeled after a binary AND operation, but differs in significant ways. *And* accepts the current image and a user selected resultant. The output is an image created by performing a multiplication of the normalized intensities of matching pixels from the two input images. In some cases, image intensity data is already normalized. Therefore, the *And* procedure is simply a pixel-wise multiplication of the two images. The two inputs required for *Out* are the current image and a user selected resultant. *Out* removes the second image form the first according to the formula A * (1 - $B/B_{max}$) where A is the current image, B the user selected image to remove, and $B_{max}$ is the maximum intensity of B. Note that the division of B by $B_{max}$ normalizes B.

Deriving a value of PBP is also disclosed in International Patent Application No. PCT/US2016/058277

Interaction Score or Spatial Proximity Score

The methods disclosed herein may comprise scoring a sample comprising tumor tissue taken from a cancer patient.

In some embodiments, the sample may be stained using a plurality of fluorescence tags with affinity for specific biomarkers. A digital image of the stained sample may be obtained, and the image further analyzed based on the location of the fluorescence tags. Rather than whole-image analysis, fields of view may be prioritized based on the number of cells that express a first biomarker of interest. A predetermined number of fields of view may then be further analyzed for fluorescence signals. In some embodiments, the use of four different types of fluorescence tags generates an image of fluorescence signals corresponding to a first biomarker of interest and an image of fluorescence signals corresponding a second biomarker of interest as well as to an image of fluorescence signals corresponding a biomarker expressed by all cells and an image of fluorescence signals corresponding a biomarker expressed by tumor cells. In further embodiments, the images of fluorescence signals are manipulated to generate one or more masks of fluorescence signals corresponding to cells within the image. In some embodiments, the one or more masks of fluorescence signals comprise one or more selected from the group consisting of a mask of all cells within the image, a mask of all tumor cells within the image, a mask of all non-tumor cells within the image, a mask of all cells expressing a first biomarker of interest within the image, a mask of all cells expressing a second biomarker of interest within the image, and an interaction mask representing all cells expressing a first biomarker of interest within the image as well as proximally located cells expressing a second biomarker of interest. In still further embodiments, the interaction mask is used to generate an interaction compartment of the cells from all selected fields of view expressing the second biomarker of interest that were proximally located to the cells expressing the first biomarker of interest. The total area of the interaction compartment may be used to generate a score representative of a spatial proximity between at least one pair of cells, a first member of the at least one pair of cells expressing the first biomarker and a second member of the at least one pair of cells expressing the second biomarker that is different from the first biomarker. In some embodiments, the score indicates the likelihood that the cancer patient will respond positively to immunotherapy.

Accordingly, in some embodiments, the methods disclosed herein comprise scoring a sample comprising tumor tissue taken from a cancer patient, the scoring step comprising: (i) using the sample comprising tumor tissue taken from the cancer patient, determining a score representative of a spatial proximity between at least one pair of cells, a first member of the at least one pair of cells expressing a first biomarker and a second member of the at least one pair of cells expressing a second biomarker that is different from the first biomarker; and (ii) recording the score, which score when compared to a threshold value is indicative of a likelihood that the cancer patient will respond positively to immunotherapy. In some embodiments, the first biomarker is PD-L1 and the second biomarker is PD-1. In some embodiments, the first biomarker is PD-1 and the second biomarker is PD-L1.

In some embodiments, the first member of the at least one pair of cells comprises a tumor cell, a myeloid cell, or a stromal cell and the second member of the at least one pair of cells comprises an immune cell. In some embodiments, the tumor cell, myeloid cell, or stromal cell expresses PD-L1 and the immune cell expresses PD-1.

In some embodiments, the first member of the at least one pair of cells comprises a tumor cell and the second member of the at least one pair of cells comprises an immune cell. In some embodiments, the first member of the at least one pair of cells comprises a myeloid cell and the second member of the at least one pair of cells comprises an immune cell. In some embodiments, the first member of the at least one pair of cells comprises a stromal cell and the second member of the at least one pair of cells comprises an immune cell. In some embodiments, the first member of the at least one pair of cells expresses PD-L1 and the immune cell expresses PD-1.

In some embodiments, the first member of the at least one pair of cells expresses PD-L1. In some embodiments, the second member of the at least one pair of cells expresses PD-1. In some embodiments, the first member of the at least one pair of cells expresses PD-L1, and the second member of the at least one pair of cells expresses PD-1.

In some embodiments, the first member of the at least one pair of cells expresses PD-1. In some embodiments, the second member of the at least one pair of cells expresses PD-L1. In some embodiments, the first member of the at least one pair of cells expresses PD-1, and the second member of the at least one pair of cells expresses PD-L1.

In some embodiments, the spatial proximity between the at least one pair of cells ranges from about 0.5 μm to about 50 μm. In some embodiments, the spatial proximity ranges from 2.5 μm to about 50 μm. In some embodiments, the spatial proximity ranges from 2.5 μm to about 45 μm. In some embodiments, the spatial proximity ranges from 2.5 μm to about 40 μm. In some embodiments, the spatial proximity ranges from 2.5 μm to about 35 μm. In some embodiments, the spatial proximity ranges from 2.5 μm to about 30 μm. In some embodiments, the spatial proximity ranges from 2.5 μm to about 25 μm. In some embodiments, the spatial proximity ranges from 2.5 μm to about 20 μm. In some embodiments, the spatial proximity ranges from 2.5 μm to about 15 μm. In some embodiments, the spatial proximity ranges from 5 μm to about 50 μm. In some embodiments, the spatial proximity ranges from 5 μm to about 45 μm. In some embodiments, the spatial proximity ranges from 5 μm to

about 40 μm. In some embodiments, the spatial proximity ranges from 5 μm to about 35 μm. In some embodiments, the spatial proximity ranges from 5 μm to about 30 μm. In some embodiments, the spatial proximity ranges from 5 μm to about 25 μm. In some embodiments, the spatial proximity ranges from 5 μm to about 20 μm. In some embodiments, the spatial proximity ranges from 5 μm to about 15 μm. In some embodiments, the spatial proximity is about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 μm.

In some embodiments, the spatial proximity between the at least one pair of cells ranges from about 1 pixel to about 100 pixels. In some embodiments, the spatial proximity ranges from about 5 to about 100 pixels. In some embodiments, the spatial proximity ranges from about 5 to about 90 pixels. In some embodiments, the spatial proximity ranges from about 5 to about 80 pixels. In some embodiments, the spatial proximity ranges from about 5 to about 70 pixels. In some embodiments, the spatial proximity ranges from about 5 to about 60 pixels. In some embodiments, the spatial proximity ranges from about 5 to about 50 pixels. In some embodiments, the spatial proximity ranges from about 5 to about 40 pixels. In some embodiments, the spatial proximity ranges from about 5 to about 30 pixels. In some embodiments, the spatial proximity ranges from about 10 to about 100 pixels. In some embodiments, the spatial proximity ranges from about 10 to about 90 pixels. In some embodiments, the spatial proximity ranges from about 10 to about 80 pixels. In some embodiments, the spatial proximity ranges from about 10 to about 70 pixels. In some embodiments, the spatial proximity ranges from about 10 to about 60 pixels. In some embodiments, the spatial proximity ranges from about 10 to about 50 pixels. In some embodiments, the spatial proximity ranges from about 10 to about 40 pixels. In some embodiments, the spatial proximity ranges from about 10 to about 30 pixels. In some embodiments, the spatial proximity is about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 pixels. In some embodiments, a pixel is 0.5 μm wide.

In some embodiments, the determining step comprises: (i) selecting a predetermined number of fields of view available from the sample comprising tumor tissue taken from the cancer patient, which is stained with a plurality of fluorescence tags, which selection is biased toward selecting fields of view that contain a greater number of cells that express the first biomarker relative to other fields of view; (ii) for each of the selected fields of view, dilating fluorescence signals attributable to the first biomarker by a margin sufficient to encompass proximally located cells expressing the second biomarker; and (iii) dividing a first total area for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the determining step comprises: (i) selecting a predetermined number of fields of view available from the sample comprising tumor tissue taken from the cancer patient, which is stained with a plurality of fluorescence tags, which selection is biased toward selecting fields of view that contain a greater number of cells that express the first biomarker relative to other fields of view; (ii) for each of the selected fields of view, dilating fluorescence signals attributable to the first biomarker to encompass proximally located cells expressing the second biomarker within about 0.5 μm to about 50 μm of a plasma membrane of the cells that express the first biomarker; and (iii) dividing a first total area for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the determining step comprises: (i) selecting a predetermined number of fields of view available from the sample comprising tumor tissue taken from the cancer patient, which is stained with a plurality of fluorescence tags, which selection is biased toward selecting fields of view that contain a greater number of cells that express the first biomarker relative to other fields of view; (ii) for each of the selected fields of view, dilating fluorescence signals attributable to the first biomarker by a margin ranging from about 1 to about 100 pixels to encompass proximally located cells expressing the second biomarker; and (iii) dividing a first total area, as measured in pixels, for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the determining step comprises: (i) selecting a predetermined number of fields of view available from the sample comprising tumor tissue taken from the cancer patient, which is stained with a plurality of fluorescence tags, which selection is biased toward selecting fields of view that contain a greater number of cells that express the first biomarker relative to other fields of view; (ii) for each of the selected fields of view, dilating fluorescence signals attributable to the first biomarker by a margin ranging from about 1 to about 100 pixels to encompass cells expressing the second biomarker within about 0.5 μm to about 50 μm of a plasma membrane of the cells that express the first biomarker; and (iii) dividing a first total area, as measured in pixels, for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, four fluorescence tags, each specific to a different biomarker, are used in the determining step. In further embodiments, a first fluorescence tag is associated with the first biomarker, a second fluorescence tag is associated with the second biomarker, a third fluorescence tag is associated with a third biomarker, and a fourth fluorescence tag is associated with a fourth biomarker. In some embodiments, the first biomarker comprises a tumor and non-tumor marker. In some embodiments, the second biomarker comprises a non-tumor marker. In some embodiments, the first biomarker comprises a tumor and non-tumor marker, and the second biomarker comprises a non-tumor marker. In some embodiments, the third biomarker is expressed by all cells. In some embodiments, the fourth biomarker is expressed only in tumor cells. In some embodiments, the third biomarker is expressed by all cells and the fourth biomarker is expressed only in tumor cells. In some embodiments, one or more fluorescence tags comprise a fluorophore conjugated to an antibody having a binding affinity for a specific biomarker or another antibody. In some embodiments, one or more fluorescence tags are fluorophores with affinity for a specific biomarker.

In some embodiments, the fluorescence signals attributable to the first biomarker are dilated by a margin ranging from about 1 to about 100 pixels. In some embodiments, the margin is from about 5 to about 100 pixels. In some embodiments, the margin is from about 5 to about 90 pixels. In some embodiments, the margin is from about 5 to about 80 pixels. In some embodiments, the margin is from about 5 to about 70 pixels. In some embodiments, the margin is from about 5 to about 60 pixels. In some embodiments, the margin is from about 5 to about 50 pixels. In some embodiments, the margin is from about 5 to about 40 pixels. In some embodiments, the margin is from about 5 to about 30 pixels. In some embodiments, the margin is from about 10 to about 100 pixels. In some embodiments, the margin is from about 10 to about 90 pixels. In some embodiments, the margin is from about 10 to about 80 pixels. In some embodiments, the margin is from about 10 to about 70 pixels. In some embodiments, the margin is from about 10 to about 60 pixels. In some embodiments, the margin is from about 10 to about 50 pixels. In some embodiments, the margin is from about 10 to about 40 pixels. In some embodiments, the margin is from about 10 to about 30 pixels. In some embodiments, the margin is about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 pixels. In some embodiments, a pixel is 0.5 μm wide.

In some embodiments, dilating fluorescence signals attributable to the first biomarker encompasses proximally located cells expressing the second biomarker within about 0.5 μm to about 50 μm of a plasma membrane of the cells that express the first biomarker. In some embodiments, dilating fluorescence signals attributable to the first biomarker encompasses proximally located cells expressing the second biomarker within about 2.5 μm to about 50 μm of a plasma membrane of the cells that express the first biomarker. In some embodiments, dilating fluorescence signals attributable to the first biomarker encompasses proximally located cells expressing the second biomarker within about 2.5 μm to about 45 μm of a plasma membrane of the cells that express the first biomarker. In some embodiments, dilating fluorescence signals attributable to the first biomarker encompasses proximally located cells expressing the second biomarker within about 2.5 μm to about 40 μm of a plasma membrane of the cells that express the first biomarker. In some embodiments, dilating fluorescence signals attributable to the first biomarker encompasses proximally located cells expressing the second biomarker within about 2.5 μm to about 35 μm of a plasma membrane of the cells that express the first biomarker. In some embodiments, dilating fluorescence signals attributable to the first biomarker encompasses proximally located cells expressing the second biomarker within about 2.5 μm to about 30 μm of a plasma membrane of the cells that express the first biomarker. In some embodiments, dilating fluorescence signals attributable to the first biomarker encompasses proximally located cells expressing the second biomarker within about 2.5 μm to about 25 μm of a plasma membrane of the cells that express the first biomarker. In some embodiments, dilating fluorescence signals attributable to the first biomarker encompasses proximally located cells expressing the second biomarker within about 2.5 μm to about 20 μm of a plasma membrane of the cells that express the first biomarker. In some embodiments, dilating fluorescence signals attributable to the first biomarker encompasses proximally located cells expressing the second biomarker within about 2.5 μm to about 15 μm of a plasma membrane of the cells that express the first biomarker. In some embodiments, dilating fluorescence signals attributable to the first biomarker encompasses proximally located cells expressing the second biomarker within about 5 μm to about 50 μm of a plasma membrane of the cells that express the first biomarker. In some embodiments, dilating fluorescence signals attributable to the first biomarker encompasses proximally located cells expressing the second biomarker within about 5 μm to

about 45 μm of a plasma membrane of the cells that express the first biomarker. In some embodiments, dilating fluorescence signals attributable to the first biomarker encompasses proximally located cells expressing the second biomarker within about 5 μm to about 40 μm of a plasma membrane of the cells that express the first biomarker. In some embodiments, dilating fluorescence signals attributable to the first biomarker encompasses proximally located cells expressing the second biomarker within about 5 μm to about 35 μm of a plasma membrane of the cells that express the first biomarker. In some embodiments, dilating fluorescence signals attributable to the first biomarker encompasses proximally located cells expressing the second biomarker within about 5 μm to about 30 μm of a plasma membrane of the cells that express the first biomarker. In some embodiments, dilating fluorescence signals attributable to the first biomarker encompasses proximally located cells expressing the second biomarker within about 5 μm to about 25 μm of a plasma membrane of the cells that express the first biomarker. In some embodiments, dilating fluorescence signals attributable to the first biomarker encompasses proximally located cells expressing the second biomarker within about 5 μm to about 20 μm of a plasma membrane of the cells that express the first biomarker. In some embodiments, dilating fluorescence signals attributable to the first biomarker encompasses proximally located cells expressing the second biomarker within about 5 μm to about 15 μm of a plasma membrane of the cells that express the first biomarker. In some embodiments, dilating fluorescence signals attributable to the first biomarker encompasses proximally located cells expressing the second biomarker within about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 μm of a plasma membrane of the cells that express the first biomarker. In some embodiments, the second biomarker on the proximally located cells is in direct contact with the first biomarker.

In some embodiments, the first total area for all cells from each of the selected fields of view, which express the second biomarker, is measured in pixels.

In some embodiments, the normalization factor is a second total area for all non-tumor cells from each of the selected fields of view. In some embodiments, the second total area is measured in pixels. In some embodiments, both the first total area and the second total area measured in pixels.

In some embodiments, the normalization factor is a second total area for all cells from each of the selected fields of view which have the capacity to express the second biomarker. In some embodiments, the second total area is measured in pixels. In some embodiments, both the first total area and the second total area measured in pixels.

In some embodiments, the normalization factor is a second total area for all cells from each of the selected fields of view. In some embodiments, the second total area is measured in pixels. In some embodiments, both the first total area and the second total area measured in pixels.

In some embodiments, the threshold score is about 500 to about 5000. In some embodiments, the threshold score is about 500 to about 4500. In some embodiments, the threshold score is about 500 to about 4000. In some embodiments, the threshold score is about 500 to about 3500. In some embodiments, the threshold score is about 500 to about 3000. In some embodiments, the threshold score is about 500 to about 2500. In some embodiments, the threshold score is about 500 to about 2000. In some embodiments, the threshold score is about 500 to about 1500. In some embodiments, the threshold score is about 500 to about 1000. In some embodiments, the threshold score is about 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, or 5000, including increments therein. In some embodiments, the threshold score is about 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, or 5000, including increments therein, plus or minus 100.

In some embodiments, the predetermined factor is from about 10 to about $10^5$. In some embodiments, the predetermined factor is from about $10^2$ to about $10^5$. In some embodiments, the predetermined factor is from about $10^3$ to about $10^5$. In some embodiments, the predetermined factor is from about $10^4$ to about $10^5$. In some embodiments, the predetermined factor is about 10, 20, 30, 40, 50, 60, 70, 80, 90, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10000, 20000, 30000, 40000, 50000, 60000, 70000, 80000, 90000, or $10^5$, including increments therein.

In some embodiments, the methods disclosed herein comprise determining a score representative of a spatial proximity between at least one pair of cells selected from among a plurality of cells present in a predetermined number of fields of view available from a sample comprising tumor tissue, which sample is taken from a cancer patient, the determining step comprising: (i) selecting a predetermined number of fields of view available from the sample comprising tumor tissue taken from the cancer patient, which is stained with a plurality of fluorescence tags, which selection is biased toward selecting fields of view that contain a greater number of cells that express a first specific biomarker relative to other fields of view; (ii) for each of the selected fields of view, dilating fluorescence signals attributable to the first specific biomarker to encompass proximally located cells expressing a second specific biomarker; and (iii) dividing a first total area for all cells from each of the selected fields of view, which express the second specific biomarker and are encompassed within the dilated fluorescence signals attributable to the cells expressing the first specific biomarker, with a normalization score, and multiplying the resulting quotient by a predetermined factor to arrive at a spatial proximity score.

In some embodiments, the methods disclosed herein comprise determining a score representative of a spatial proximity between at least one pair of cells selected from among a plurality of cells present in a predetermined number of fields of view available from a sample comprising tumor tissue, which sample is taken from a cancer patient, the determining step comprising: (i) selecting a predetermined number of fields of view available from the sample comprising tumor tissue taken from the cancer patient, which is stained with a plurality of fluorescence tags, which selection is biased toward selecting fields of view that contain a greater number of cells that express a first biomarker relative to other fields of view; (ii) for each of the selected fields of view, dilating fluorescence signals attributable to the first biomarker to encompass cells expressing a second biomarker within about 0.5 μm to about 50 μm of a plasma membrane of the cells that express the first biomarker; and (iii) dividing a first total area for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the methods disclosed herein comprise determining a score representative of a spatial proximity between at least one pair of cells selected from among a plurality of cells present in a predetermined number of fields of view available from a sample comprising tumor tissue, which sample is taken from a cancer patient, the determining step comprising: (i) selecting a predetermined number of fields of view available from the sample comprising tumor tissue taken from the cancer patient, which is stained with a plurality of fluorescence tags, which selection is biased toward selecting fields of view that contain a greater number of cells that express a first biomarker relative to other fields of view; (ii) for each of the selected fields of view, dilating fluorescence signals attributable to the first biomarker by a margin ranging from about 1 to about 100 pixels to encompass proximally located cells expressing a second biomarker; and (iii) dividing a first total area, as measured in pixels, for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the methods disclosed herein comprise determining a score representative of a spatial proximity between at least one pair of cells selected from among a plurality of cells present in a predetermined number of fields of view available from a sample comprising tumor tissue, which sample is taken from a cancer patient, the determining step comprising: (i) selecting a predetermined number of fields of view available from the sample comprising tumor tissue taken from the cancer patient, which is stained with a plurality of fluorescence tags, which selection is biased toward selecting fields of view that contain a greater number of cells that express a first biomarker relative to other fields of view; (ii) for each of the selected fields of view, dilating fluorescence signals attributable to the first biomarker by a margin ranging from about 1 to about 100 pixels to encompass cells expressing a second biomarker within about 0.5 μm to about 50 μm of a plasma membrane of the cells that express the first biomarker; and (iii) dividing a first total area, as measured in pixels, for all cells from each of the selected fields of view, which express the second biomarker and are encompassed within the dilated fluorescence signals attributable to the cells expressing the first biomarker, with a normalization factor, and multiplying the resulting quotient by a predetermined factor to arrive at a spatial proximity score.

In some embodiments, the spatial proximity score (SPS) is determined by the following equation:

$$SPS = \frac{A_I}{A_{NT}} \times 10^4$$

wherein $A_I$ is a total interaction area (total area of cells expressing the second specific biomarker and encompassed by dilated fluorescence signals attributable to cells expressing the first specific biomarker) and $A_{NT}$ is the total area of non-tumor cells.

In some embodiments, the spatial proximity score is determined by the following equation:

$$SPS = \frac{A_I}{A_C} \times 10^4$$

wherein $A_I$ is a total interaction area (total area of cells expressing the second specific biomarker and encompassed by dilated fluorescence signals attributable to cells expressing the first specific biomarker) and $A_C$ is the total area of cells that have a capacity to express the second specific biomarker.

In some embodiments, scoring a sample comprising tumor tissue from a cancer patient is used in methods of treating cancer in the patient. In some embodiments, scoring a sample comprising tumor tissue from a cancer patient is performed prior to administration of immunotherapy.

**FIG. 15** is a flowchart depicting the steps of one embodiment of scoring a sample comprising tumor tissue taken from a cancer patient. In step 1401, image data is obtained and in step 1402, the image data is unmixed such that data specific to various types of fluorescence signals are separated into different channels. In step 1403, data from a first channel is used to generate a mask of all cells that are positive for a first biomarker (first biomarker mask). The mask of all cells is then dilated (step 1404) to generate a dilated mask representative of a predetermined proximity within which an interacting cell (positive for a second biomarker) may be found. In some embodiments, the first biomarker mask is dilated between 1 and 100 pixels. In step 1405, data from a second channel is used to generate a mask of all cells that are positive for the second biomarker (second biomarker mask). In step 1406, the first biomarker mask and the second biomarker mask are combined to generate an interaction mask identifying cells that are positive for the second biomarker that are within the predetermined proximity of a cell positive for the first biomarker. In step 1407, a spatial proximity score is calculated based on the area of the interaction mask.

**FIG. 16** is a second flowchart depicting the steps of a second embodiment of scoring sample comprising tumor tissue taken from a cancer patient. In step 1501, image data is obtained and in step 1502, the image data is unmixed such that data specific to various types of fluorescence signals are separated into different channels. In step 1503, data from a first channel is used to generate a mask of all cells in the field of view and in step 1504 data from a second channel is used to generate a mask of a subset area, such as tumor area, in the field of view. In step 1505 the mask of all cells is combined with the subset area mask to generate a mask of subset cells and a mask of non-subset cells. In some embodiments, the subset cells are tumor cells and the non-subset cells are non-tumor cells. In step 1506, data from a third channel is used to generate a mask of all cells that are positive for a first biomarker (first biomarker mask). The mask of all positive cells is then dilated (step 1507) to generate a dilated mask representative of a predetermined proximity within which an interacting cell (*i.e.,* a cell that is positive for a second biomarker) may be found. In some embodiments, the first biomarker mask is dilated between 1 and 100 pixels. In step 1508, data from a fourth channel is used to generate a mask of all cells that are positive for the second biomarker (second biomarker mask). In step 1509, the dilated mask and the second biomarker mask are combined to generate an interaction mask identifying cells that are positive for the second biomarker and are within the predetermined proximity of a cell positive for the first biomarker. In step 1510, a spatial proximity score is calculated by dividing the area of the interaction mask by an area of all cells that are capable of being positive for the second biomarker (the subset cells) or by an area of all cells (as indicated by the dotted lines in the flowchart of **FIG. 16** representing use of either input). In some embodiments, the cells that are capable of being positive for the second biomarker are tumor cells or non-tumor cells.

In some embodiments, a subset of cells and a non-subset of cells corresponds to tumor cells and non-tumor cells, respectively or vice versa. In some embodiments, a subset of cells and a non-subset of cells corresponds to viable cells and non-viable cells, respectively or vice versa. In some embodiments, a subset of cells is a subset of viable cells and a non-subset of cells consists of the viable cells not included in the subset of viable cells. In some embodiments, a subset of cells and a non-subset of cells corresponds to T cells and non-T cells, respectively or vice versa. In some embodiments, a subset of cells and a non-subset of cells corresponds to myeloid cells and non-myeloid cells, respectively or vice versa.

In some embodiment, the spatial proximity score is representative of a nearness of a pair of cells. In some embodiments, the nearness of a pair of cells may be determined by a proximity between the boundaries of the pair of cells, a proximity between the centers of mass of the pair of cells, using boundary logic based on a perimeter around a selected first cell of the pair of cells, determining an intersection in the boundaries of the pair of cells, and/or by determining an area of overlap of the pair of cells.

In some embodiment, the spatial proximity score is associated with metadata associated with the images of the sample, included in a generated report, provided to an operator to determine immunotherapy strategy, recorded in a database, associated with a patient's medical record, and/or displayed on a display device.

In the methods disclosed herein, the manipulation of the digital images may be carried out by a computing system comprising a controller, such as the controller illustrated in the block diagram of **FIG. 17,** according to an exemplary embodiment. Controller 200 is shown to include a communications interface 202 and a processing circuit 204. Communications interface 202 may include wired or wireless interfaces (e.g., jacks, antennas, transmitters, receivers, transceivers, wire terminals, etc.) for conducting data communications with various systems, devices, or networks. For example, communications interface 202 may include an Ethernet card and port for sending and receiving data via an Ethernet-based communications network and/or a WiFi transceiver for communicating via a wireless communications network. Communications interface 202 may be configured to communicate via local area networks or wide area networks (e.g., the Internet, a building WAN, etc.) and may use a variety of communications protocols (e.g., BACnet, IP, LON, etc.).

Communications interface 202 may be a network interface configured to facilitate electronic data communications between controller 200 and various external systems or devices (e.g., imaging device 102). For example, controller 200 may receive imaging data for the selected fields of view from the imaging device 102, to analyze the data and calculate the spatial proximity score (SPS).

Still referring to **FIG. 17,** processing circuit 204 is shown to include a processor 206 and memory 208. Processor 206 may be a general purpose or specific purpose processor, an application specific integrated circuit (ASIC), one or more field programmable gate arrays (FPGAs), a group of processing components, or other suitable processing components. Processor 506 may be configured to execute computer code or instructions stored in memory 508 or received from other computer readable media (e.g., CDROM, network storage, a remote server, etc.).

Memory 208 may include one or more devices (e.g., memory units, memory devices, storage devices, etc.) for storing data and/or computer code for completing and/or facilitating the various processes described in the present disclosure. Memory 208 may include random access memory (RAM), read-only memory (ROM), hard drive storage, temporary storage, non-volatile memory, flash memory, optical memory, or any other suitable memory for storing software objects and/or computer instructions. Memory 208 may include database components, object code components, script components, or any other type of information structure for supporting the various activities and information structures described in the present disclosure. Memory 508 may be communicably connected to processor 206 via processing circuit 204 and may include computer code for executing (e.g., by processor 206) one or more processes described herein.

Still referring to **FIG. 17,** controller 200 is shown to receive input from an imaging device 102. The imaging device acquires all of the imaging data and records it, along with all of the meta-data which describes it. The imaging device will then serialize the data into a stream which can be read by controller 200. The data stream may accommodate any binary data stream type such as the file system, a RDBM or direct TCP/IP communications. For use of the data stream, controller 200 is shown to include a spectral unmixer 210. The spectral unmixer 210 may receive image data from an imaging device 102 on which it performs spectral unmixing to unmix an image presenting various wavelengths into individual, discrete channels for each band of wavelengths. For example, the image data may be "unmixed" into separate channels for each of the various fluorophores used to identify cells or proteins of interest in the tissue sample. The fluorophore, by way of example only, may be one or more of the group consisting of DAPI, Cy® 2, Cy® 3, Cy® 3,5, Cy® 5, FITC, TRITC, a 488 dye, a 555 dye, a 594 dye, and Texas Red. In one example, one of the channels may include image data that falls within a predetermined band surrounding a wavelength of 461 nm (the maximum emission wavelength for DAPI), to identify nuclei in the image. Other channels may include image data for different wavelengths to identify different portions of the tissue sample using different fluorophores.

Controller 200 is also shown to include various maskers, such as cell masker 212, subset area masker 216, first biomarker masker 22, and second biomarker masker 224. These, or other maskers that may be included in the controller 200 in other embodiments, are used to receive an unmixed signal from the spectral unmixer 210 and create a mask for the particular cell or area of interest, dependent on the fluorophore used to identify certain features of interest in the tissue sample. To create a mask, the maskers (such as cell masker 212, subset area masker 216, first biomarker masker 22, and second biomarker masker 224) receive image data related to an intensity of each pixel in the field of view. Pixel intensity is directly proportional to the amount of fluorescence emitted by the sample, which in turn, is directly proportional to the amount of protein biomarker in the sample (when using a fluorophore to identify a particular biomarker). An absolute threshold may be set based on the values which exist in the image pixels. All the pixels which are greater than or equal to the threshold value will be mapped to 1.0, or "on", and all other pixels will be mapped to 0.0, or "off." In this way, a binary mask is created to identify the cell or tissue portion of interest in the field of view. In other embodiments, a mask is created using a lower bound wherein all pixels with an intensity at or above a lower bound are accepted and used as the pixel value for the mask. If the intensity is below the lower bound, the pixel value is set to 0.0, or "off'.

In the example flow diagram for masking shown in **FIG. 18,** it is shown that the DAPI and 488 channels (for identifying nuclei and tumor areas, respectively) use the lower bound protocol (steps 1710, 1712, 1720, 1722), while Cy5 and Cy3.5 channels (for identifying biomarkers) use a threshold value protocol (steps 1730, 1740), for providing the mask outputs. In association with the lower bound protocol, there is also a histogram step to determine the lower bound. In particular, *histogram threshold* (step 1712, 1722) produces a threshold of an input image but uses a sliding scale to determine the point at which the thresholding occurs. The inputs are the current image and a user defined *threshold percentage.* The latter is used to determine at what percent of the total intensity the threshold level should be set. Firstly, the intensity of every pixel is summed into a total intensity. The *threshold percentage* is multiplied by this total intensity to obtain a cut-off sum. Finally, all pixels are grouped by intensity (in a histogram) and their intensities summed from lowest to highest (bin by bin) until the cut-off sum is achieved. The last highest pixel intensity visited in the process is the threshold for the current image. All pixels with intensities greater than that value have their intensities set to maximum while all others are set to the minimum.

The steps identified as steps 1714, 1716, 1724, 1726, 1728, 1732, 1734, 1736, 1742, 1744 in **FIG. 18** represent intermediary steps that occur in the initial maskers, such as cell masker 212, subset area masker 216, first biomarker masker 222, and second biomarker masker 224. These steps are defined as follows:

*Dilate* increases the area of brightest regions in an image. Two inputs are need for *dilate.* The first is the implicit current image and the second is the number of *iterations* to dilate. It is assumed that only binary images are used for the first input. The procedure will operate on continuous images, but the output will not be a valid *dilate.* The *dilate* process begins by first finding the maximum pixel intensity in the image. Subsequently, each pixel in the image is examined once. If the pixel under investigation has intensity equal to the maximum intensity, that pixel will be drawn in the output image as a circle with *iterations* radius and centered on the original pixel. All pixels in that circle will have intensity equal to the maximum intensity. All other pixels are copied into the output image without modification.

The *fill holes* procedure will fill "empty" regions of an image with pixels at maximum intensity. These empty regions are those that have a minimum intensity and whose pixel area (*size*) is that specified by the user. The current image and *size* are the two inputs required. Like *dilate* this procedure should only be applied to binary images.

*Erode* processes images in the same fashion as *dilate.* All functionality is the same as *dilate* except that the first step determines the minimum intensity in the image, only pixels matching that lowest intensity are altered, and the circles used to bloom the found minimum intensity pixels are filled with the lowest intensity value. Like *dilate* this procedure should only be applied to binary images.

*Remove Objects.* Two inputs are expected: the current image and *object size. Remove objects* is the opposite of the *fill holes* procedure. Any regions containing only pixels with maximum intensity filling an area less than the input *object size* will be set to minimum intensity and thusly "removed." This procedure should only be applied to binary images; application to continuous images may produce unexpected results.

The output at final steps 1718, 1729, 1738, and 1746 are the resultant cell mask, subset area mask (or, in this particular example, the tumor area mask), biomarker 1 cell mask, and biomarker 2 cell mask, respectively. **FIG. 18** further depicts the combinations of these resultant masks to calculate the spatial proximity score. These combinations are described below with reference to the combination maskers of the controller 200, depicted in **FIG. 17.**

Controller 200 is shown to include combination maskers, such as subset cell masker 218, non-subset cell masker 220, and interaction masker 230. Subset cell masker performs an *And* operation, as shown at step 1752 in **FIG. 18,** to combine the output of the cell masker 212 (representative of all cells in the image) with the output of the subset area masker 216. Accordingly, subset cell masker generates a mask of all subset cells in the image. In some embodiments, the subset cells are tumor cells. This same combination, using an *Out* operation performed by non-subset cell masker 220 as shown at step 1754 in **FIG. 18,** generates a mask of all non-subset cells in the sample image. In some embodiments, the non-subset cells are non-tumor cells.

Before being combined with another mask, the first biomarker mask (from first biomarker masker 222) is dilated by dilator 226. The dilated mask represents an area surrounding those cells expressing a first biomarker, so as to identify a space in which cells expressing the second biomarker would be within a proper proximity to interact with the cell expressing the first biomarker. This is represented by steps 1756 and 1758 of **FIG. 18.** The flow chart of **FIG. 18** shows the dilation taking place in two steps, 1756 and 1758. This may be required when there is a limit to the maximum iterations in each step. For example, there may be a maximum of 10 iterations (corresponding to a 10 pixel increase), so when a 20 pixel increase is needed, the dilation must be split into two subsequent steps.

Within second biomarker masker 224, the biomarker mask may be combined with the non-subset cell mask described above, using an *And* operation, as shown in step 1760 of **FIG. 18,** to generate a mask of all non-subset cells that are positive for the first biomarker. This mask is then combined (step 1762) at interaction masker 230 with the dilated mask from dilator 226 to generate an interaction mask. The interaction mask identified the non-subset cells that are positive for the second biomarker and that are also within the interaction area, or that overlap the dilated mask. These identified cells, then, represent the cells that could interact with the cells positive for the first biomarker, thus resulting in greater therapy response.

To calculate the spatial proximity score (SPS), the area of the interaction mask is determined in pixels at the area evaluator 232. In some embodiments, the area of all the cells that are capable of expressing the second biomarker is determined in pixels at the area evaluator 234. The cells that are capable of expressing the second biomarker may be tumor cells or non-tumor cells. In some embodiments, In some embodiments, the area of all cells is determined in pixels at the area evaluator 234. An interaction, or spatial proximity, score is determined at the interaction calculator 236 by dividing the area from area evaluator 232 by the area from area evaluator 234 and multiplying by a predetermined factor. As described above, in one embodiment, the equation executed by the interaction calculator 236 is:

$$SPS = \frac{A_I}{A_C} \times 10^4$$

wherein $A_I$ is a total interaction area (total area of cells expressing the second specific biomarker and encompassed by dilated fluorescence signals attributable to cells expressing the first specific biomarker) and $A_C$ is the total area of cells that have a capacity to express the second specific biomarker or the total area of all cells in the field of view.

The *And* procedure is modeled after a binary AND operation, but differs in significant ways. *And* accepts the current image and a user selected resultant. The output is an image created by performing a multiplication of the normalized intensities of matching pixels from the two input images. In some cases, image intensity data is already normalized. Therefore, the *And* procedure is simply a pixel-wise multiplication of the two images. The two inputs required for *Out* are the current image and a user selected resultant. *Out* removes the second image form the first according to the formula A * (1 - $B/B_{max}$) where A is the current image, B the user selected image to remove, and $B_{max}$ is the maximum intensity of B. Note that the division of B by $B_{max}$ normalizes B.

Determining a score representative of a spatial proximity between at least one pair of cells selected among a plurality of cells present in a predetermined number of fields of view available from a sample comprising tumor tissue is disclosed in International Patent Application No. PCT/US2016/058281.

Fluorescence tags

In some embodiments, the fluorescence signals are from four fluorescence tags, each specific to a different biomarker. In further embodiments, a first fluorescence tag is associated with the first biomarker of interest, a second fluorescence tag is associated with the second biomarker of interest, a third fluorescence tag is associated with a third biomarker of interest, and a fourth fluorescence tag is associated with a fourth biomarker of interest. In some embodiments, the first biomarker of interest comprises a tumor and non-tumor marker. In some embodiments, the second biomarker of interest comprises a non-tumor marker. In some embodiments, the first biomarker of interest comprises a tumor and non-tumor marker, and the second biomarker of interest comprises a non-tumor marker. In some embodiments, the third biomarker of interest is expressed by all cells. In some embodiments, the fourth biomarker of interest is expressed only in tumor cells. In some embodiments, the third biomarker of interest is expressed by all cells and the fourth biomarker of interest is expressed only in tumor cells. In some embodiments, the fourth biomarker of interest is the subset biomarker. In some embodiments, the third biomarker of interest is expressed by all cells and the fourth biomarker of interest is the subset biomarker. In some embodiments, one or more fluorescence tags comprise a fluorophore conjugated to an antibody having a binding affinity for a specific biomarker or another antibody. In some embodiments, one or more fluorescence tags are fluorophores with affinity for a specific biomarker.

Examples of fluorophores include, but are not limited to, fluorescein, 6-FAM, rhodamine, Texas Red, California Red, iFluor594, tetramethylrhodamine, a carboxyrhodamine, carboxyrhodamine 6F, carboxyrhodol, carboxyrhodamine 110, Cascade Blue, Cascade Yellow, coumarin, Cy2®, Cy3®, Cy3.5®, Cy5®, Cy5.5®, Cy7®, Cy-Chrome, DyLight® 350, DyLight® 405, DyLight® 488, DyLight® 549, DyLight® 594, DyLight® 633, DyLight® 649, DyLight® 680, DyLight® 750, DyLight® 800, phycoerythrin, PerCP (peridinin chlorophyll-a Protein), PerCP-Cy5.5, JOE (6-carboxy-4',5'-dichloro-2',7'-dimethoxyfluorescein), NED, ROX (5-(and -6-)-carboxy-X-rhodamine), HEX, Lucifer Yellow, Marina Blue, Oregon Green 488, Oregon Green 500, Oregon Green 514, Alexa Fluor® 350, Alex Fluor® 430, Alexa Fluor® 488, Alexa Fluor® 532, Alexa Fluor® 546, Alexa Fluor® 568, Alexa Fluor® 594, Alexa Fluor® 633, Alexa Fluor® 647, Alexa Fluor® 660, Alexa Fluor® 680, 7-amino-4-methylcoumarin-3-acetic acid, BODIPY® FL, BODIPY® FL-Br2, BODIPY® 530/550, BODIPY® 558/568, BODIPY® 630/650, BODIPY® 650/665, BODIPY® R6G, BODIPY® TMR, BODIPY® TR, OPAL™ 520, OPAL™ 540, OPAL™ 570, OPAL™ 620, OPAL™ 650, OPAL™ 690, and combinations thereof. In some embodiments, the fluorophore is selected from the group consisting of DAPI, Cy® 2, Cy® 3, Cy® 3,5, Cy® 5, Cy® 7, FITC, TRITC, a 488 dye, a 555 dye, a 594 dye, Texas Red, and Coumarin. Examples of a 488 dye include, but are not limited to, Alexa Fluor® 488, OPAL™ 520, DyLight® 488, and CF™ 488A. Examples of a 555 dye include, but are not limited to, Alexa Fluor® 555. Examples of a 594 dye include, but are not limited to, Alexa Fluor® 594.

As used herein, a "field of view" refers to a section of a whole-slide digital image of a tissue sample. In some embodiments, the whole-slide image has 2-200 predetermined fields of view. In some embodiments, the whole-slide image has 10-200 predetermined fields of view. In some embodiments, the whole-slide image has 30-200 predetermined fields of view. In some embodiments, the whole-slide image has 10-150 predetermined fields of view. In some embodiments, the whole-slide image has 10-100 predetermined fields of view. In some embodiments, the whole-slide image has 10-50 predetermined fields of view. In some embodiments, the whole-slide image has 10-40 predetermined fields of view. In some embodiments, the whole-slide image has 10,15, 20, 25, 30, 35, 40, 45, 50, 55, 60, 65, 70, 75, 80, 85, 90, 95, or 100, including increments therein, predetermined fields of view.

In methods disclosed herein, the cancer patient is a mammal. In some embodiments, the mammal is human. In some embodiments, the mammal is not human. In further embodiments, the mammal is mouse, rat, guinea pig, dog, cat, or horse.

In methods disclosed herein, tumor tissue is taken from a cancer patient. The type of cancer includes, but is not limited to, cancers of the: circulatory system, for example, heart (sarcoma [angiosarcoma, fibrosarcoma, rhabdomyosarcoma, liposarcoma], myxoma, rhabdomyoma, fibroma, lipoma and teratoma), mediastinum and pleura, and other intrathoracic organs, vascular tumors and tumor-associated vascular tissue; respiratory tract, for example, nasal cavity and middle ear, accessory sinuses, larynx, trachea, bronchus and lung such as small cell lung cancer (SCLC), non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; gastrointestinal system, for example, esophagus (squamous cell carcinoma, adenocarcinoma, leiomyosarcoma, lymphoma), stomach (carcinoma, lymphoma, leiomyosarcoma), gastric, pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); genitourinary tract, for example, kidney (adenocarcinoma, Wilm's tumor [nephroblastoma], lymphoma, leukemia), bladder and/or urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); liver, for example, hepatoma (hepatocellular carcinoma), cholangiocarcinoma, hepatoblastoma, angiosarcoma, hepatocellular adenoma, hemangioma, pancreatic endocrine tumors (such as pheochromocytoma, insulinoma, vasoactive intestinal peptide tumor, islet cell tumor and glucagonoma); bone, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; nervous system, for example, neoplasms of the central nervous system (CNS), primary CNS lymphoma, skull cancer (osteoma, hemangioma, granuloma, xanthoma, osteitis deformans), meninges (meningioma, meningiosarcoma, gliomatosis), brain cancer (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); reproductive system, for example, gynecological, uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma) and other sites associated with female genital organs; placenta, penis, prostate, testis, and other sites associated with male genital organs; hematologic system, for example, blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; oral cavity, for example, lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx; skin, for example, malignant melanoma, cutaneous melanoma, basal cell carcinoma, squamous cell carcinoma, Karposi's sarcoma, moles dysplastic nevi, lipoma, angioma, dermatofibroma, and keloids; adrenal glands: neuroblastoma; and other tissues including connective and soft tissue, retroperitoneum and peritoneum, eye, intraocular melanoma, and adnexa, breast, head or/and neck, anal region, thyroid, parathyroid, adrenal gland and other endocrine glands and related structures, secondary and unspecified malignant neoplasm of lymph nodes, secondary malignant neoplasm of respiratory and digestive systems and secondary malignant neoplasm of other sites, or a combination of one or more thereof. In some embodiments, the tumor tissue is taken from a melanoma cancer patient. In some embodiments, the tumor tissue is taken from a lung cancer patient. In some embodiments, the tumor tissue is taken from a non-small cell lung cancer patient.

Examples of immunotherapy include, but are not limited to, monoclonal antibodies (*e.g.,* alemtuzumab or trastuzumab), conjugated monoclonal antibodies (*e.g.,* ibritumomab tiuxetan, brentuximab vendotin, or ado-trastuzumab emtansine), bispecific monoclonal antibodies (blinatumomab), immune checkpoint inhibitors (*e.g.,* ipilimumab, pembrolizumab, nivolumab, atezolizumab, or durvalumab), thalidomide, lenalidomide, pomalidomide, and imiquimod, and combinations thereof. In some embodiments, immunotherapy comprises, consists essentially of, or consists of anti-PD-1 treatment. Non-limiting examples of anti-PD-1 treatment include pembrolizumab, nivolumab, and combinations thereof. In some embodiments, immunotherapy comprises, consists essentially of, or consists of anti-PD-LI treatment. Non-limiting examples of anti-PD-LI treatment include atezolizumab, durvalumab, and combinations thereof. In some embodiments, immunotherapy comprises, consists essentially of, or consists of IDO-1 inhibiting treatment. Non-limiting examples of IDO-1 inhibiting treatment include Indoximod, INCB024360, NLG919, and combinations thereof. In some embodiments, the immunotherapy comprises immune checkpoint therapy plus indoleamine 2,3-dioxigenase (IDO-1) inhibitors (*e.g.,* Indoximod, INCB024360, NLG919), or Arginase-1 inhibitors (*e.g.,* cb-1158). In some embodiments, the immunotherapy is administered in conjunction with chemotherapy. In some embodiments, chemotherapy is given in an adjuvant setting. Examples of chemotherapy or adjuvant chemotherapy include, but are not limited to, cisplatin, etoposide, alimta, carboplatin, paclitaxel, pemetrexed, taxotere, docetaxel, gemcitabine, navelbine, taxol, avastin, bevacizumab, vinorelbine, vinblastine, and combinations thereof.

In some embodiments, the adjuvant chemotherapy comprises an agent selected from an anti-angiogenesis agent (*e.g.,* an agent that stops tumors from developing new blood vessels). Examples of anti-angiogenesis agents include for example VEGF inhibitors, VEGFR inhibitors, TIE-2 inhibitors, PDGFR inhibitors, angiopoetin inhibitors, PKC.beta. inhibitors, COX-2 (cyclooxygenase II) inhibitors, integrins (alpha-v/beta-3), MMP-2 (matrix-metalloprotienase 2) inhibitors, and MMP-9 (matrix-metalloprotienase 9) inhibitors. Preferred anti-angiogenesis agents include sunitinib (Sutent®), bevacizumab (Avastin®), axitinib (AG 13736), SU 14813 (Pfizer), and AG 13958 (Pfizer).

Additional anti-angiogenesis agents include vatalanib (CGP 79787), Sorafenib (Nexavar®), pegaptanib octasodium (Macugen®), vandetanib (Zactima®), PF-0337210 (Pfizer), SU 14843 (Pfizer), AZD 2171 (AstraZeneca), ranibizumab (Lucentis®), Neovastat® (AE 941), tetrathiomolybdata (Coprexa®), AMG 706 (Amgen), VEGF Trap (AVE 0005), CEP 7055 (Sanofi-Aventis), XL 880 (Exelixis), telatinib (BAY 57-9352), and CP-868,596 (Pfizer).

Other anti-angiogenesis agents include enzastaurin (LY 317615), midostaurin (CGP 41251), perifosine (KRX 0401), teprenone (Selbex®) and UCN 01 (Kyowa Hakko).

Other examples of anti-angiogenesis agents which can be used as described herein include celecoxib (Celebrex®), parecoxib (Dynastat®), deracoxib (SC 59046), lumiracoxib (Preige®), valdecoxib (Bextra®), rofecoxib (Vioxx®), iguratimod (Careram®), IP 751 (Invedus), SC-58125 (Pharmacia) and etoricoxib (Arcoxia®).

Other anti-angiogenesis agents include exisulind (Aptosyn®), salsalate (Amigesic®), diflunisal (Dolobid®), ibuprofen (Motrin®), ketoprofen (Orudis®) nabumetone (Relafen®), piroxicam (Feldene®), naproxen (Aleve®, Naprosyn®) diclofenac (Voltaren®), indomethacin (Indocin®), sulindac (Clinoril®), tolmetin (Tolectin®), etodolac (Lodine®), ketorolac (Toradol®), and oxaprozin (Daypro®).

Other anti-angiogenesis agents include ABT 510 (Abbott), apratastat (TMI 005), AZD 8955 (AstraZeneca), incyclinide (Metastat®), and PCK 3145 (Procyon).

Other anti-angiogenesis agents include acitretin (Neotigason®), plitidepsin (Aplidine®), cilengtide (EMD 121974), combretastatin A4 (CA4P), fenretinide (4 HPR), halofuginone (Tempostatin®), Panzem® (2-methoxyestradiol), PF-03446962 (Pfizer), rebimastat (BMS 275291), catumaxomab (Removab®), lenalidomide (Revlimid®) squalamine (EVIZON®), thalidomide (Thalomid®), Ukrain® (NSC 631570), Vitaxin® (MEDI 522), and zoledronic acid (Zometa®).

In some embodiments, the adjuvant chemotherapy comprises a so-called signal transduction inhibitor (e.g., inhibiting the means by which regulatory molecules that govern the fundamental processes of cell growth, differentiation, and survival communicated within the cell). Signal transduction inhibitors include small molecules, antibodies, and antisense molecules. Signal transduction inhibitors include for example kinase inhibitors (*e.g.*, tyrosine kinase inhibitors or serine/threonine kinase inhibitors) and cell cycle inhibitors. More specifically signal transduction inhibitors include, for example, ALK inhibitors, ROS1 inhibitors, TrkA inhibitors, TrkB inhibitors, TrkC inhibitors, farnesyl protein transferase inhibitors, EGF inhibitor, ErbB-1 (EGFR), ErbB-2, pan erb, IGF1R inhibitors, MEK, c-Kit inhibitors, FLT-3 inhibitors, K-Ras inhibitors, PI3 kinase inhibitors, JAK inhibitors, STAT inhibitors, Raf kinase inhibitors, Akt inhibitors, mTOR inhibitor, P70S6 kinase inhibitors, inhibitors of the WNT pathway and so called multi-targeted kinase inhibitors.

Preferred signal transduction inhibitors include gefitinib (Iressa®), cetuximab (Erbitux®), erlotinib (Tarceva®), trastuzumab (Herceptin®), sunitinib (Sutent®) imatinib (Gleevec®), and PD325901 (Pfizer).

Additional examples of signal transduction inhibitors which may be used according to the methods described herein include BMS 214662 (Bristol-Myers Squibb), lonafarnib (Sarasar®), pelitrexol (AG 2037), matuzumab (EMD 7200), nimotuzumab (TheraCIM h-R3®), panitumumab (Vectibix®), Vandetanib (Zactima®), pazopanib (SB 786034), ALT 110 (Alteris Therapeutics), BIBW 2992 (Boehringer Ingelheim), and Cervene® (TP 38).

Other examples of signal transduction inhibitor include PF-2341066 (Pfizer), PF-299804 (Pfizer), canertinib (CI 1033), pertuzumab (Omnitarg®), Lapatinib (Tycerb®), pelitinib (EKB 569), miltefosine (Miltefosin®), BMS 599626 (Bristol-Myers Squibb), Lapuleucel-T (Neuvenge®), NeuVax® (E75 cancer vaccine), Osidem® (IDM 1), mubritinib (TAK-165), CP-724,714 (Pfizer), panitumumab (Vectibix®), lapatinib (Tycerb®), PF-299804 (Pfizer), pelitinib (EKB 569), and pertuzumab (Omnitarg®).

Other examples of signal transduction inhibitors include ARRY 142886 (Array Biopharm), everolimus (Certican®), zotarolimus (Endeavor®), temsirolimus (Torisel®), AP 23573 (ARIAD), and VX 680 (Vertex).

Additionally, other signal transduction inhibitors include XL 647 (Exelixis), sorafenib (Nexavar®), LE-AON (Georgetown University), and GI-4000 (GlobeImmune).

Other signal transduction inhibitors include ABT 751 (Abbott), alvocidib (flavopiridol), BMS 387032 (Bristol Myers), EM 1421 (Erimos), indisulam (E 7070), seliciclib (CYC 200), BIO 112 (One Bio), BMS 387032 (Bristol-Myers Squibb), PD 0332991 (Pfizer), AG 024322 (Pfizer), LOXO-101 (Loxo Oncology), crizotinib, and ceritinib.

In some embodiments, the adjuvant chemotherapy comprises a classical antineoplastic agent. Classical antineoplastic agents include but are not limited to hormonal modulators such as hormonal, anti-hormonal, androgen agonist, androgen antagonist and anti-estrogen therapeutic agents, histone deacetylase (HDAC) inhibitors, gene silencing agents or gene activating agents, ribonucleases, proteosomics, Topoisomerase I inhibitors, Camptothecin derivatives, Topoisomerase II inhibitors, alkylating agents, antimetabolites, poly(ADP-ribose) polymerase-1 (PARP-1) inhibitor, microtubulin inhibitors, antibiotics, plant derived spindle inhibitors, platinum-coordinated compounds, gene therapeutic agents, antisense oligonucleotides, vascular targeting agents (VTAs), and statins.

Examples of classical antineoplastic agents that may be used according to the methods disclosed herein include, but are not limited to, glucocorticoids, such as dexamethasone, prednisone, prednisolone, methylprednisolone, hydrocortisone, and progestins such as medroxyprogesterone, megestrol acetate (Megace), mifepristone (RU-486), Selective Estrogen Receptor Modulators (SERMs; such as tamoxifen, raloxifene, lasofoxifene, afimoxifene, arzoxifene, bazedoxifene, fispemifene, ormeloxifene, ospemifene, tesmilifene, toremifene, trilostane and CHF 4227 (Cheisi)), Selective Estrogen-Receptor Downregulators (SERD's; such as fulvestrant), exemestane (Aromasin), anastrozole (Arimidex), atamestane, fadrozole, letrozole (Femara), gonadotropin-releasing hormone (GnRH; also commonly referred to as luteinizing hormone-releasing hormone [LHRH]) agonists such as buserelin (Suprefact), goserelin (Zoladex), leuprorelin (Lupron), and triptorelin (Trelstar), abarelix (Plenaxis), bicalutamide (Casodex), cyproterone, flutamide (Eulexin), megestrol, nilutamide (Nilandron), and osaterone, dutasteride, epristeride, finasteride, Serenoa repens, PHL 00801, abarelix, goserelin, leuprorelin, triptorelin, bicalutamide, tamoxifen, exemestane, anastrozole, fadrozole, formestane, letrozole, and combinations thereof.

Other examples of classical antineoplastic agents that may be used according to the methods disclosed herein include, but are not limited to, suberolanilide hydroxamic acid (SAHA, Merck Inc./Aton Pharmaceuticals), depsipeptide (FR901228 or FK228), G2M-777, MS-275, pivaloyloxymethyl butyrate and PXD-101; Onconase (ranpirnase), PS-341 (MLN-341), Velcade (bortezomib), 9-aminocamptothecin, belotecan, BN-80915 (Roche), camptothecin, diflomotecan, edotecarin, exatecan (Daiichi), gimatecan, 10-hydroxycamptothecin, irinotecan HCl (Camptosar), lurtotecan, Orathecin (rubitecan, Supergen), SN-38, topotecan, camptothecin, 10-hydroxycamptothecin, 9-aminocamptothecin, irinotecan, SN-38, edotecarin, topotecan, aclarubicin, adriamycin, amonafide, amrubicin, annamycin, daunorubicin, doxorubicin, elsamitrucin, epirubicin, etoposide, idarubicin, galarubicin, hydroxycarbamide, nemorubicin, novantrone (mitoxantrone), pirarubicin, pixantrone, procarbazine, rebeccamycin, sobuzoxane, tafluposide, valrubicin, Zinecard (dexrazoxane), nitrogen mustard N-oxide, cyclophosphamide, AMD-473, altretamine, AP-5280, apaziquone, brostallicin, bendamustine, busulfan, carboquone, carmustine, chlorambucil, dacarbazine, estramustine, fotemustine, glufosfamide, ifosfamide, KW-2170, lomustine, mafosfamide, mechlorethamine, melphalan, mitobronitol, mitolactol, mitomycin C, mitoxatrone, nimustine, ranimustine, temozolomide, thiotepa, and platinum-coordinated alkylating compounds such as cisplatin, Paraplatin (carboplatin), eptaplatin, lobaplatin, nedaplatin, Eloxatin (oxaliplatin, Sanofi), streptozocin, satrplatin, and combinations thereof.

In some embodiments, the adjuvant chemotherapy includes dihydrofolate reductase inhibitors (such as methotrexate and NeuTrexin (trimetresate glucuronate)), purine antagonists (such as 6-mercaptopurine riboside, mercaptopurine, 6-thioguanine, cladribine, clofarabine (Clolar), fludarabine, nelarabine, and raltitrexed), pyrimidine antagonists (such as 5-fluorouracil (5-FU), Alimta (premetrexed disodium, LY231514, MTA), capecitabine (Xeloda®), cytosine arabinoside, Gemzar® (gemcitabine, Eli Lilly), Tegafur (UFT Orzel or Uforal and including TS-1 combination of tegafur, gimestat and otostat), doxifluridine, carmofur, cytarabine (including ocfosfate, phosphate stearate, sustained release and liposomal forms), enocitabine, 5-azacitidine (Vidaza), decitabine, and ethynylcytidine) and other antimetabolites such as eflornithine, hydroxyurea, leucovorin, nolatrexed (Thymitaq), triapine, trimetrexate, N-(5-[N-(3,4-dihydro-2-methyl-4-oxoquinazolin-6-ylmethyl)-N-methylamino]-2-thenoyl)-L-glutamic acid, AG-014699 (Pfizer Inc.), ABT-472 (Abbott Laboratories), INO-1001 (Inotek Pharmaceuticals), KU-0687 (KuDOS Pharmaceuticals) and GPI 18180 (Guilford Pharm Inc) and combinations thereof.

Other examples of classical antineoplastic cytotoxic agents used according to the methods disclosed herein include, but are not limited to, Abraxane (Abraxis BioScience, Inc.), Batabulin (Amgen), EPO 906 (Novartis), Vinflunine (Bristol-Myers Squibb Company), actinomycin D, bleomycin, mitomycin C, neocarzinostatin (Zinostatin), vinblastine, vincristine, vindesine, vinorelbine (Navelbine), docetaxel (Taxotere), Ortataxel, paclitaxel (including Taxoprexin a DHA/paciltaxel conjugate), cisplatin, carboplatin, Nedaplatin, oxaliplatin (Eloxatin), Satraplatin, Camptosar, capecitabine (Xeloda), oxaliplatin (Eloxatin), Taxotere alitretinoin, Canfosfamide (Telcyta®), DMXAA (Antisoma), ibandronic acid, L-asparaginase, pegaspargase (Oncaspar®), Efaproxiral (Efaproxyn®--radiation therapy)), bexarotene (Targretin®), Tesmilifene (DPPE--enhances efficacy of cytotoxics)), Theratope® (Biomira), Tretinoin (Vesanoid®), tirapazamine (Trizaone®), motexafin gadolinium (Xcytrin®) Cotara® (mAb), and NBI-3001 (Protox Therapeutics), polyglutamate-paclitaxel (Xyotax®) and combinations thereof.

Further examples of classical antineoplastic agents that may be used according to the methods disclosed herein include, but are not limited to, as Advexin (ING 201), TNFerade (GeneVec, one or more compounds which express TNFalpha in response to radiotherapy), RB94 (Baylor College of Medicine), Genasense (Oblimersen, Genta), Combretastatin A4P (CA4P), Oxi-4503, AVE-8062, ZD-6126, TZT-1027, Atorvastatin (Lipitor, Pfizer Inc.), Provastatin (Pravachol, Bristol-Myers Squibb), Lovastatin (Mevacor, Merck Inc.), Simvastatin (Zocor, Merck Inc.), Fluvastatin (Lescol, Novartis), Cerivastatin (Baycol, Bayer), Rosuvastatin (Crestor, Astra-Zeneca), Lovostatin, Niacin (Advicor, Kos Pharmaceuticals), Caduet, Lipitor, torcetrapib, and combinations thereof.

Adjuvant chemotherapy for the treatment of breast cancer in a subject in need of such treatment may comprise one or more (preferably one to three) anti-cancer agents selected from the group consisting of trastuzumab, tamoxifen, docetaxel, paclitaxel, capecitabine, gemcitabine, vinorelbine, exemestane, letrozole and anastrozole.

Adjuvant chemotherapy for the treatment of colorectal cancer in a subject in need of such treatment may comprise one or more (preferably one to three) anti-cancer agents. Examples of particular anti-cancer agents include those typically used in adjuvant chemotherapy, such as FOLFOX, a combination of 5-fluorouracil (5-FU) or capecitabine (Xeloda), leucovorin and oxaliplatin (Eloxatin). Further examples of particular anti-cancer agents include those typically used in chemotherapy for metastatic disease, such as FOLFOX or FOLFOX in combination with bevacizumab (Avastin); and FOLFIRI, a combination of 5-FU or capecitabine, leucovorin and irinotecan (Camptosar). Further examples include 17-DMAG, ABX-EFR, AMG-706, AMT-2003, ANX-510 (CoFactor), aplidine (plitidepsin, Aplidin), Aroplatin, axitinib (AG-13736), AZD-0530, AZD-2171, bacillus Calmette-Guerin (BCG), bevacizumab (Avastin), BIO-117, BIO-145, BMS-184476, BMS-275183, BMS-528664, bortezomib (Velcade), C-1311 (Symadex), cantuzumab mertansine, capecitabine (Xeloda), cetuximab (Erbitux), clofarabine (Clofarex), CMD-193, combretastatin, Cotara, CT-2106, CV-247, decitabine (Dacogen), E-7070, E-7820, edotecarin, EMD-273066, enzastaurin (LY-317615) epothilone B (EPO-906), erlotinib (Tarceva), flavopyridol, GCAN-101, gefitinib (Iressa), huA33, huC242-DM4, imatinib (Gleevec), indisulam, ING-1, irinotecan (CPT-11, Camptosar) ISIS 2503, ixabepilone, lapatinib (Tykerb), mapatumumab (HGS-ETR1), MBT-0206, MEDI-522 (Abregrin), Mitomycin, MK-0457 (VX-680), MLN-8054, NB-1011, NGR-TNF, NV-1020, oblimersen (Genasense, G3139), OncoVex, ONYX 015 (CI-1042), oxaliplatin (Eloxatin), panitumumab (ABX-EGF, Vectibix), pelitinib (EKB-569), pemetrexed (Alimta), PD-325901, PF-0337210, PF-2341066, RAD-001 (Everolimus), RAV-12, Resveratrol, Rexin-G, S-1 (TS-1), seliciclib, SN-38 liposome, Sodium stibogluconate (SSG), sorafenib (Nexavar), SU-14813, sunitinib (Sutent), temsirolimus (CCI 779), tetrathiomolybdate, thalomide, TLK-286 (Telcyta), topotecan (Hycamtin), trabectedin (Yondelis), vatalanib (PTK-787), vorinostat (SAHA, Zolinza), WX-UK1, and ZYC300, wherein the amounts of the anticancer agents are effective in treating colorectal cancer.

Adjuvant chemotherapy for the treatment of renal cell carcinoma in a subject in need of such treatment may comprise one or more (preferably one to three) anti-cancer agents selected from the group consisting of capecitabine (Xeloda), interferon alpha, interleukin-2, bevacizumab (Avastin), gemcitabine (Gemzar), thalidomide, cetuximab (Erbitux), vatalanib (PTK-787), Sutent, AG-13736, SU-11248, Tarceva, Iressa, Lapatinib and Gleevec, wherein the amounts of the anticancer agents are effective in treating renal cell carcinoma.

Adjuvant chemotherapy for the treatment of melanoma in a subject in need of such treatment may comprise one or more (preferably one to three) anti-cancer agents selected from the group consisting of interferon alpha, interleukin-2, temozolomide (Temodar), docetaxel (Taxotere), paclitaxel, Dacarbazine (DTIC), carmustine (also known as BCNU), Cisplatin, vinblastine, tamoxifen, PD-325,901, Axitinib, bevacizumab (Avastin), thalidomide, sorafanib, vatalanib (PTK-787), Sutent, CpG-7909, AG-13736, Iressa, Lapatinib and Gleevec, wherein the amounts of the anticancer agents are effective in treating melanoma.

Adjuvant chemotherapy for the treatment of lung cancer in a subject in need of such treatment may comprise one or more (preferably one to three) anti-cancer agents selected from the group consisting of capecitabine (Xeloda), bevacizumab (Avastin), gemcitabine (Gemzar), docetaxel (Taxotere), paclitaxel, premetrexed disodium (Alimta), Tarceva, Iressa, Vinorelbine, Irinotecan, Etoposide, Vinblastine, and Paraplatin (carboplatin), wherein the amounts of the agents are effective in treating lung cancer.

Adjuvant chemotherapy for the treatment of renal cell carcinoma in a subject in need of such treatment may comprise one or more additional medicinal or pharmaceutical agents selected from 5-fluorouracil, vismodegib, sonidegib, and imiquimod. In some embodiments, the one additional medicinal or pharmaceutical agent is 5-fluorouracil. In some embodiments, the one additional medicinal or pharmaceutical agent is vismodegib. In some embodiments, the one additional medicinal or pharmaceutical agent is sonidegib. In some embodiments, the one additional medicinal or pharmaceutical agent is imiquimod.

EXAMPLES

**Example 1. Sample preparation, imaging, and analysis of imaging for melanoma tissue samples from human patients**

*Sample preparation.* Formalin fixed paraffin embedded (FFPE) tissue samples were dewaxed. The slides were then rehydrated through a series of xylene to alcohol washes before incubating in distilled water. Heat-induced antigen retrieval was then performed using elevated pressure and temperature conditions, allowed to cool, and transferred to Tris-buffered saline. Staining was then performed where the following steps were carried out. First, endogenous peroxidase was blocked followed by incubation with a protein-blocking solution to reduce nonspecific antibody staining. Next, the slides were stained with a mouse anti-PD-1 primary antibody. Slides were then washed before incubation with an anti-mouse HRP secondary antibody. Slides were washed and then PD-1 staining was detected using TSA+ Cy® 3.5 (Perkin Elmer). Any residual HRP was then quenched using two washes of fresh 100 mM benzhydrazide with 50 mM hydrogen peroxide. The slides were again washed before staining with a rabbit anti-PD-LI primary antibody. Slides were washed and then incubated with a cocktail of anti-rabbit HRP secondary antibody plus mouse anti-S 100 directly labeled with 488 dye and 4',6-diamidino-2-phenylindole (DAPI). Slides were washed and then PD-L1 staining was detected using TSA-Cy® 5 (Perkin Elmer). Slides were washed a final time before they were cover-slipped with mounting media and allowed to dry overnight at room temperature. A schematic overview of the antibodies and detection reagents is shown in **FIG 1.**

*Sample imaging and analysis.* Fluorescence images were then acquired using the Vectra 2 Intelligent Slide Analysis System using the Vectra software version 2.0.8 (Perkin Elmer). First, monochrome imaging of the slide at 4x magnification using DAPI was conducted. An automated algorithm (developed using inForm) was used to identify areas of the slide containing tissue.

The areas of the slide identified as containing tissue were imaged at 4x magnification for channels associated with DAPI (blue), FITC (green), and Cy® 5 (red) to create RGB images. These 4x images were processed using an automated enrichment algorithm (developed using inForm) in field of view selector 104 to identify and rank possible 20x magnification fields of view according to the highest Cy® 5 expression.

The top 40 fields of view were imaged at 20x magnification across DAPI, FITC, Texas Red, and Cy® 5 wavelengths. Raw images were reviewed for acceptability, and images that were out of focus, lacked any tumor cells, were highly necrotic, or contained high levels of fluorescence signal not associated with expected antibody localization (*i.e.,* background staining) were rejected prior to analysis. Accepted images were processed using AQUAduct (Perkin Elmer), wherein each fluorophore was spectrally unmixed by spectral unmixer 210 into individual channels and saved as a separate file.

The processed files were further analyzed using AQUAnalysis™ or through a fully automated process using AQUAserve™. Details were as follows.

Each DAPI image was processed by nuclei masker 212 to identify all cell nuclei within that image **(FIG. 2a),** and then dilated by 3 pixels to represent the approximate size of an entire cell. This resulting mask represented all cells within that image **(FIG. 2b).**

S100 (tumor cell marker for melanoma) detected with 488 dye **(FIG. 3a)** was processed by tumor masker 216 to create a binary mask of all tumor area within that image **(FIG. 3b).** Overlap between this mask and the mask of all cells created a new mask for tumor cells **(FIG. 3c),** using tumor cell masker 218.

Similarly, absence of the tumor cell marker in combination with the mask of all nuclei created a new mask for all non-tumor cells **(FIG. 3d),** performed using non-tumor cell masker 220.

Each Cy® 5 image **(FIG. 4a)** was processed by first biomarker masker 222 and overlapped with the mask of all cells to create a binary mask of all cells that are PD-L1-positive **(FIG. 4b).** Overlapping the biomarker mask with the mask of all cells eliminated noise pixels that may be falsely identified in the mask as biomarker positive cells.

Each Cy® 3.5 image **(FIG. 5a)** was processed by second biomarker masker 224 to create a binary mask for PD-1-positive cells and overlapped with the mask of all non-tumor cells to create a binary mask of all non-tumor cells that are PD-1-positive **(FIG. 5b).** Overlapping the biomarker mask with the mask of all non-tumor cells eliminated noise pixels that may be falsely identified in the mask as biomarker positive cells.

The binary mask of all PD-L1-positive cells was dilated using second dilator 226 to create an interaction mask encompassing the nearest neighbor cells *(e.g.,* cells with PD-1) **(FIG. 6a).** This interaction mask was combined with a binary mask of all PD-1-positive non-tumor cells using interaction masker 230 to create an interaction compartment of the PD-1-positive cells in close enough proximity to the PD-L1-positive cells such that PD-1 is likely interacting with PD-L1 **(FIG. 6b).**

The total area from all accepted fields (up to 40 fields of view) for the interaction compartment and the total area of the non-tumor cells was calculated in area evaluators 232, 234 respectively. The total area from all accepted fields of view for the interaction compartment was divided by the total area of the non-tumor cells and multiplied by a factor of 10,000, using the interaction calculator 236 to create a whole number representing an interaction score for each specimen. PD-L1 and PD-1 measurements were highly reproducible ($R^2$ = 0.98 and 0.97, respectively). In a cohort of 24 advanced melanoma patients treated with nivolumab (n=5) or pembrolizumab (n=19), the PD-1/PD-L1 interaction score was found to reliably distinguish responders from non-responders (*p* = 0.04) while PD-L1 alone (*p* = 0.22), PD-1 alone (*p* = 0.3) or CD8 alone (*p* = 0.23) did not. Representative scores from the 24 patients are shown in **FIG. 7a.** Based on the data, a threshold of 900 was selected to indicate likelihood of response to treatment.

This data set was then expanded to 142 total metastatic melanoma patients where PD-1/PD-L1 interaction scores were obtained (see **FIG. 7b)** and demonstrated responders had statistically significantly higher PD-1/PD-L1 interaction scores than non-responders (p = 0.02).

To improve the ability of the PD-1/PD-L1 interaction score test to correctly identify patients who respond to anti-PD-1 axis therapies, the same melanoma patient specimens were stained with antibodies to identify phenotypic markers characteristic of myeloid cells (CD11b and HLA-DR) and the biochemical enzyme IDO-1 that renders suppressive function upon these cells. Sample preparation was analogous to that performed for the PD-1/PD-L1 interaction score test where the slides were stained with a rabbit anti-IDO-1 primary antibody. Slides were then washed before incubation with anti-rabbit HRP secondary antibody. Slides were washed and then anti-IDO-1 was detected using TSA+ Cy® 5 (Perkin Elmer). Any residual HRP was then quenched using two washes of fresh 100 mM benzhydrazide with 50 mM hydrogen peroxide. The slides were again washed before staining with a mouse anti-HLA-DR primary antibody. Slides were washed and then incubated with anti-mouse HRP secondary antibody. Slides were washed and then the anti-HLA-DR staining was detected using TSA-Cy® 3 (Perkin Elmer). Primary and secondary antibody reagents were then removed via microwave. The slides were again washed before staining with a rabbit anti-CD 11b antibody. Slides were washed and then incubated with a cocktail of anti-rabbit HRP secondary antibody plus 4',6-diamidino-2-phenylindole (DAPI). Slides were washed and then anti-CD11b staining was detected using TSA-AlexaFluor488 (Life Technologies). Slides were washed a final time before they were cover-slipped with mounting media and allowed to dry overnight at room temperature.

Analogous procedures to the PD-1/PD-L1 interaction test were used for sample imaging and analysis across DAPI, FITC, Cy®3, and Cy®5 wavelengths. 4x magnification images were processed using an automated enrichment algorithm (developed using inForm) in field of view selector 104 to identify and rank possible 20x magnification fields of view according to the highest Cy® 3 and Cy® 5 expression.

Each DAPI image was processed by cell masker 212 to identify all cell nuclei within that image and then dilated to represent the approximate size of an entire cell. This resulting mask represented all cells within that image.

Each AlexaFluor488® image was processed by biomarker masker 222 to create a binary mask of all cells that are CD11b positive.

Each Cy® 3 image was processed by biomarker masker 222 to create a binary mask of all cells that are HLA-DR positive.

Each Cy® 5 image was processed by biomarker masker 222 to create a binary mask of all cells that are IDO-1 positive.

The binary masks for all cells CD11b positive and HLA-DR positive were combined to create a binary mask of all cells that were either double positive for CD11b and HLA-DR or were CD11b positive and HLA-DR negative.

The % biomarker positivity (PBP) for all CD11b cells lacking expression of HLA-DR was derived, using positivity calculator 236, by dividing the total area, measured in pixels and determined by area evaluator 232, of the mask of all CD11b-positive, HLA-DR-negative cells with the total area, measured in pixels and determined by area evaluator 232, of the mask of all CD11b-positive cells.

The binary masks for all cells CD11b positive, IDO-1 positive, and HLA-DR negative were combined to create a binary mask of all cells that are CD11b-positive, HLA-DR-negative, and IDO-1-positive.

The PBP for all CD11b cells expressing IDO-1, but lacking expression of HLA-DR was derived by dividing the total area, measured in pixels, of the mask of all CD11b-positive, HLA-DR-negative, IDO-1-positive cells with the total area, measured in pixels, of the mask of all CD11b-positive cells.

The binary masks for all cells HLA-DR positive and IDO-1 positive were combined to create a binary mask of all cells that are double positive for HLA-DR and IDO-1.

The % biomarker positivity (PBP) for all HLA-DR cells expressing IDO-1 was derived, using positivity calculator 236, by dividing the total area, measured in pixels and determined by area evaluator 232, of the mask of all IDO-1-positive, HLA-DR-positive cells with the total area, measured in pixels and determined by area evaluator 232, of the mask of all HLA-DR-positive cells.

The binary masks for all cells CD11b positive, IDO-1 positive, and HLA-DR positive were combined to create a binary mask of all cells that are CD11b-positive, HLA-DR-positive, and IDO-1-positive.

The PBP for all CD11b cells expressing IDO-1 and HLA-DR was derived by dividing the total area, measured in pixels, of the mask of all CD11b-positive, HLA-DR-positive, IDO-1-positive cells with the total area, measured in pixels, of the mask of all CD11b-positive cells.

The differential expression of the PBP for the phenotypes listed above was compared to response status in metastatic melanoma patients treated with either nivolumab or pembrolizumab (n = 24) to determine which cell subset (if any) predicted response to anti-PD-1 axis therapies (FIG. 8a). Unexpectedly, the PBP of all HLA-DR positive cells expressing IDO-1 was able to distinguish responders from non-responders **(FIG. 7c)** and HLA-DR positive cells expressing IDO-1 were predominantly CD11b negative **(FIG. 7d).**

Additionally, it was observed that the combination of patients high for the PD-1/PD-L1 interaction score (≥900) or the IDO-1$^+$HLA-DR$^+$ PBP (≥5%) was able to improve the ability to correctly identify the greatest number of patients (12/13) who responded to anti-PD-1 axis therapy when compared to either test alone (9/13 or 10/13 respectively, **FIG. 8b).**

This observation was validated in the additional set of samples from metastatic melanoma patients treated with either nivolumab or pembrolizumab (n = 142), where again the values of PBP of IDO-1$^+$HLA-DR$^+$ were also able to distinguish patients who responded to therapy ($p$ = 0.0002) and the combination of the two signatures identified the greatest number of patients who responded to anti-PD-1 therapy (55/78) than either signature alone (PD-1/PD-L1 interaction score 40/78 or IDO-1$^+$HLA-DR$^+$ 38/78 , $p$ = 0.0096,**FIG. 8c).** Additionally, the cohorts were combined and the highest prediction of response (80%) was observed in patients who had both biomarker signatures expressed above the cut points (PD-1/PD-L1 interaction score ≥900 and IDO-1$^+$HLA-DR$^+$ PBP (≥5%) compared to 54% for patients positive for only one signature or 36% for patients negative for both signatures) **(FIG. 8d).**

The patients who were positive for either the PD-1/PD-L1 interaction test or the IDO-1$^+$HLA-DR$^+$ PBP test also had improved progression free survival (PFS, **FIG. 9a.),** hazard ratio = 0.36, $p$ = 0.0037 and overall survival (OS, **FIG. 9b),** hazard ratio = 0.39, $p$ = 0.0011. In contrast, PD-L1 tumor expression was not able to identify patients with improved PFS or OS at 5% **(FIGS. 20a-b).** Additionally, in the combined cohort, patients above the cut point for both biomarker signatures demonstrated the highest overall survival $p$ = 0.0018 **(FIG. 10).**

### Example 2. Alternative sample preparation, imaging, and analysis of imaging for melanoma tissue samples from human patients for HLA-DR and IDO-1

Analogous procedures from Example 1 were performed where melanoma samples were prepared by staining with mouse anti-HLA-DR primary antibody. Slides were washed and then incubated with anti-mouse HRP secondary antibody. Slides were washed and HLA-DR expression was detected with Cy® 3. Any residual HRP was then quenched using two washes of fresh 100 mM benzhydrazide with 50 mM hydrogen peroxide. Slides were then stained with a rabbit anti-IDO-1 primary antibody. Slides were washed and then incubated with a cocktail of anti-rabbit HRP secondary antibody plus mouse anti-S100 directly labeled with 488 dye and 4',6-diamidino-2-phenylindole (DAPI). Slides were washed and then anti-IDO-1 was detected using TSA+ Cy® 5.

Analogous procedures to the PD-1/PD-L1 interaction test were used for sample imaging and analysis across DAPI, FITC, Cy®3, and Cy®5 wavelengths. 4x magnification images were processed using an automated enrichment algorithm (developed using inForm) in field of view selector 104 to identify and rank possible 20x magnification fields of view according to the highest Cy® 3 and Cy® 5 expression.

Each DAPI image was processed by cell masker 212 to identify all cell nuclei within that image and then dilated to represent the approximate size of an entire cell. This resulting mask represented all cells within that image.

Each AlexaFluor488® image was processed by biomarker masker 222 to create a binary mask of all cells that are S100 positive.

Each Cy® 3 image was processed by biomarker masker 222 to create a binary mask of all cells that are HLA-DR positive.

Each Cy® 5 image was processed by biomarker masker 222 to create a binary mask of all cells that are IDO-1 positive.

The binary masks for all cells HLA-DR positive and IDO-1 positive were combined to create a binary mask of all cells that are double positive for HLA-DR and IDO-1.

The % biomarker positivity (PBP) for all HLA-DR cells expressing IDO-1 was derived, using positivity calculator 236, by dividing the total area, measured in pixels and determined by area evaluator 232, of the mask of all IDO-1-positive, HLA-DR-positive cells with the total area, measured in pixels and determined by area evaluator 232, of the mask of all HLA-DR-positive cells.

The method for identification of PBP of HLA-DR$^+$IDO-1$^+$ from Example 1 was compared with that from Example 2 and found to have a high correlation ($R^2$ = 0.8) where all samples greater than or equal to the 5% threshold with the CD11b/HLA-DR/IDO-1 assay configuration remained greater than or equal to the 5% threshold with the S100/HLA-DR/IDO-1 assay configuration **(FIG. 19)**

### Example 3. Sample preparation, imaging, and analysis of imaging for non-small cell lung cancer (NSCLC) tissue samples from human patients

Analogous methods to Example 1 were performed for the PD-1/PD-L1 interaction assay except the mouse anti-SIOO reagent was replaced with mouse anti-Pan Cytokeratin directly labeled with a 488 dye on 463 early stage NSCLC samples from patients with or without adjuvant chemotherapy treatment. High PD-1/PD-L1 interaction scores (greater than or equal to the median interaction score of 734) were found to predict patients who responded to adjuvant chemotherapy **(FIG. 21a).** High PD-1/PD-L1 interaction scores did not show a difference in survival for patients who did not receive adjuvant chemotherapy **(FIG. 21b).** There was also no significant survival benefit according to whether or not patients received adjuvant chemotherapy **(FIG. 22).**

Additionally, analogous methods to Example 1 were peformed where the same NSCLC samples were stained with mouse anti-CD4 antibody detected with Opal520, mouse anti-CD8 antibody detected by Opal620, rabbit anti-FoxP3 antibody detected with Opal540, rabbit anti-CD25 antibody detected with Opal570 and mouse anti-Ki67 antibody detected wtih Opal650 in addition to DAPI. PBP was calculated for %$CD4^+$ of all cells, %$CD8^+$ of all cells, %$CD4^+$ or $CD8^+$ of all cells, %$CD25^+FoxP3^+$ of $CD4^+$, %$CD25^+FoxP3^+$ of CD8 cells, %$CD25^+FoxP3^+$ of all $CD4^+$ or $CD8^+$, %$Ki67^+$ of $CD4^+$, %$Ki67^+$ of $CD8^+$, %$Ki67^+$ of $CD4^+$ or $CD8^+$. Patients who received adjuvant chemotherapy whose tumor samples demonstrated expression greater than or equal to the median expression of 1% $CD25^+FoxP3^+$ of all T cells ($CD4^+$ or $CD8^+$) were found to have statistically significantly higher PFS, $p = 0.0027$ **(FIG. 23a)** and OS, $p = 0.0056$ **(FIG. 23b).** Additionally after optimizing the cut point for both signatures, patients dual positive with PD-1/PD-L1 interaction score ≥643 and $CD25^+FoxP3^+$ ≥1% had improved PFS, $p = 0.003$ **(FIG. 24a)** and OS, $p = 0.004$ **(FIG. 24b).** In contrast, NSCLC patients (n = 328) who were not administered chemotherapy did not experience survival benefit when classified by the same signatures (P>0.5) PFS **(FIG. 25a)** or OS **(FIG. 25b)** with ≥1% $CD25^+FoxP3^+$ of all T cells or in PFS **(FIG. 26a)** or OS **(FIG. 26b)** for the combined signature dual positive with PD-1/PD-L1 interaction score ≥643 and $CD25^+FoxP3^+$ ≥1%. Patients who received adjuvant chemotherapy whose tumor samples demonstrated expression greater than or equal to the median expression of 6% $Ki67^+$ of all T cells were found to have a statistically significantly lower PFS, $p = 0.004$ **(FIG. 27a)** and OS, $p = 0.0006$ **(FIG. 27b).** Additionally, patients dual positive with PD-1/PD-L1 interaction score ≥643 and $Ki67^+$ <6% had improved PFS, $p = 0.022$ **(FIG. 28a)** and OS, $p = 0.029$ **(FIG. 28b).** In contrast, NSCLC patients (n = 328) who were not administered chemotherapy did not experience survival benefit when classified by the same signatures (P>0.5) PFS **(FIG. 29a)** or OS **(FIG. 29b)** with ≥6% $Ki67^+$ of all T cells or in PFS **(FIG. 30a)** or OS **(FIG. 30b)** for the combined signature dual positive with PD-1/PD-L1 interaction score ≥643 and $Ki67^+$ <1%.

Para. A. A method of predicting a likelihood that a cancer patient will respond positively to immunotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all HLA-DR positive cells in a field of view of the sample expressing $HLA\text{-}DR^+IDO\text{-}1^+$, and recording the value for PBP; and

(C) comparing the interaction score to a first threshold value and comparing the value for PBP to a second threshold value;

wherein if either the interaction score is greater than or equal to the first threshold value or the value for PBP is greater than or equal to the second threshold value, then the cancer patient is likely to respond positively to immunotherapy.

Para. B. A method of predicting a likelihood that a cancer patient will respond positively to immunotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all HLA-DR positive cells in a field of view of the sample expressing $HLA\text{-}DR^+IDO\text{-}1^+$, and recording the value for PBP; and

(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy.

Para. C. The method of Para. A or Para. B, wherein the immunotherapy targets the PD-1 and/or PD-L1 axis.

Para. D. The method of Para. A or Para. B, wherein the immunotherapy comprises anti-PD-1 treatment, anti-PD-L1 treatment, IDO-1 inhibiting treatment, or combinations thereof.

Para. E. The method of any one of Paras. A-D, wherein the spatial proximity is assessed on a pixel scale.

Para. F. The method of any one of Paras. A-E, wherein the spatial proximity between the at least one pair of cells ranges from about 1 pixel to about 100 pixels.

Para. G. The method of any one of Paras. A-F, wherein the spatial proximity between the at least one pair of cells ranges from about 0.5 $\mu$m to about 50 $\mu$m.
Para. H. The method of any one of Paras. A-G, wherein the first threshold value ranges from about 500 to about 5000.
Para. I. The method of any one of Paras. A-H, wherein the first threshold value is about 900 plus or minus 100.
Para. J. The method of any one of Paras. A-I, wherein the second threshold value ranges from about 2% to about 10%.
Para. K. The method of any one of Paras. A-J, wherein the second threshold value is about 5% plus or minus 1%.
Para. L. The method of any one of Paras. A-K, wherein the cancer patient is a melanoma cancer patient or a lung cancer patient.
Para. M. The method of any one of Paras. A-L, wherein the cancer patient is a melanoma cancer patient.
Para. N. The method of any one of Paras. A-L, wherein the cancer patient is a non-small cell lung cancer patient.
Para. O. A method of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to respond positively to immunotherapy.
Para. P. A method of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

Para. Q. The method of Para. O or Para. P, wherein the immunotherapy targets the PD-1 and/or PD-L1 axis.

Para. R. The method of Para. O or Para. P, wherein the immunotherapy comprises anti-PD-1 treatment, anti-PD-LI treatment, anti-IDO-1 treatment, or combinations thereof.

Para. S. The method of any one of Paras. O-R, wherein the total area of the fourth mask and the total area of the sixth mask from step (B) are measured in pixels.

Para. T. The method of any one of Paras. O-S, wherein the spatial proximity is assessed on a pixel scale.

Para. U. The method of any one of Paras. O-T, wherein each of the fluorescence tags is directed to a specific biomarker.

Para. V. The method of any one of Paras. O-U, wherein the plurality of fluorescence tags comprises a first fluorescence tag for PD-1 and a second fluorescence tag for PD-L1.

Para. W. The method of any one of Paras. O-V, wherein the margin ranges from about 1 to about 100 pixels.

Para. X. The method of any one of Paras. O-W, wherein the proximally located cells expressing PD-L1 are within about 0.5 to about 50 $\mu$m of a plasma membrane of the cells that express PD-1.

Para. Y. The method of any one of Paras. O-X, wherein the first total area for all cells from each of the selected fields of view from step (A) is measured in pixels.

Para. Z. The method of any one of Paras. O-Y, wherein the normalization factor is a second total area for all non-tumor cells from each of the selected fields of view.

Para. AA. The method of any one of Paras. O-Z, wherein the predetermined factor is $10^4$.

Para. AB. The method of any one of Paras. O-AA, wherein the first threshold value ranges from about 500 to about 5000.

Para. AC. The method of any one of Paras. O-AB, wherein the first threshold value is about 900 plus or minus 100.

Para. AD. The method of any one of Paras. O-AC, wherein the spatial proximity score (SPS) is determined by the following equation:

$$SPS = \frac{A_I}{A_C} \times 10^4$$

wherein $A_I$ is a total interaction area (total area of cells expressing PD-1 and encompassed by dilated fluorescence signals attributable to cells expressing PD-L1) and $A_C$ is the total area of cells that have a capacity to express the PD-1.

Para. AE. The method of any one of Paras. O-AD, wherein the second threshold value ranges from about 2% to about 10%.

Para. AF. The method of any one of Paras. O-AE, wherein the second threshold value is about 5% plus or minus 1%.

Para. AG. The method of any one of Paras. O-AF, wherein the cancer patient is a melanoma cancer patient or a lung cancer patient.

Para. AH. The method of any one of Paras. O-AG, wherein the cancer patient is a melanoma cancer patient.

Para. AI. The method of any one of Paras. O-AG, wherein the cancer patient is a non-small cell lung cancer patient.

Para. AJ. A method of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;

for each of the selected fields of view, dilating fluorescence signals attributable to PD-1 by a margin sufficient to encompass proximally located cells expressing PD-L1;

dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1 $^+$;

deriving the value for PBP for all cells present in the field of view expressing HLA-DR$^+$IDO-1$^+$ by dividing the total area of the sixth mask by the total area of the fourth mask; and

recording the value for PBP; and

C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to respond positively to immunotherapy.

Para. AK. A method of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;
for each of the selected fields of view, dilating fluorescence signals attributable to PD-1 by a margin sufficient to encompass proximally located cells expressing PD-L1;
dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1$^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

Para. AL. The method of Para. AJ or Para. AK, wherein the immunotherapy targets the PD-1 and/or PD-L1 axis.

Para. AM. The method of Para. AJ or Para. AK, wherein the immunotherapy comprises anti-PD-1 treatment, anti-PD-Ll treatment, anti-IDO-1 treatment, or combinations thereof.

Para. AN. The method of any one of Paras. AJ-AM, wherein the total area of the fourth mask and the total area of the sixth mask from step (B) are measured in pixels.

Para. AO. The method of any one of Paras. AJ-AN, wherein the spatial proximity is assessed on a pixel scale.

Para. AP. The method of any one of Paras. AJ-AO, wherein each of the fluorescence tags is directed to a specific biomarker.

Para. AQ. The method of any one of Paras. AJ-AP, wherein the plurality of fluorescence tags comprises a first fluorescence tag for PD-1 and a second fluorescence tag for PD-L1.

Para. AR. The method of any one of Paras. AJ-AQ, wherein the margin ranges from about 1 to about 100 pixels.

Para. AS. The method of any one of Paras. AJ-AR, wherein the proximally located cells expressing PD-L1 are within about 0.5 to about 50 $\mu$m of a plasma membrane of the cells that express PD-1.

Para. AT. The method of any one of Paras. AJ-AS, wherein the first total area for all cells from each of the selected fields of view from step (A) is measured in pixels.

Para. AU. The method of any one of Paras. AJ-AT, wherein the normalization factor is a second total area for all non-tumor cells from each of the selected fields of view.

Para. AV. The method of any one of Paras. AJ-AU, wherein the predetermined factor is $10^4$.

Para. AW. The method of any one of Paras. AJ-AV, wherein the first threshold value ranges from about 500 to about 5000.

Para. AX. The method of any one of Paras. AJ-AW, wherein the first threshold value is about 900 plus or minus 100.

Para. AY. The method of any one of Paras. AJ-AX, wherein the spatial proximity score (SPS) is determined by the following equation:

$$SPS = \frac{A_I}{A_C} \times 10^4$$

wherein $A_I$ is a total interaction area (total area of cells expressing PD-L1 and encompassed by dilated fluorescence signals attributable to cells expressing PD-1) and $A_C$ is the total area of cells that have a capacity to express the PD-L1.

Para. AZ. The method of any one of Paras. AJ-AY, wherein the second threshold value ranges from about 2% to about 10%.

Para. BA. The method of any one of Paras. AJ-AZ, wherein the second threshold value is about 5% plus or minus 1%.

Para. BB. The method of any one of Paras. AJ-BA, wherein the cancer patient is a melanoma cancer patient or a lung cancer patient.

Para. BC. The method of any one of Paras. AJ-BB, wherein the cancer patient is a melanoma cancer patient.

Para. BD. The method of any one of Paras. AJ-BB, wherein the cancer patient is a non-small cell lung cancer patient.

Para. BE. A method of predicting a likelihood that a cancer patient will respond positively to immunotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if either the interaction score is greater than or equal to the first threshold value or the value for PBP is greater than or equal to the second threshold value, then the cancer patient is likely to respond positively to immunotherapy.

Para. BF. A method of predicting a likelihood that a cancer patient will respond positively to immunotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy.

Para. BG. The method of Para. BE or Para. BF, wherein the immunotherapy targets the PD-1 and/or PD-L1 axis.

Para. BH. The method of Para. BE or Para. BF, wherein the immunotherapy comprises anti-PD-1 treatment, anti-PD-LI treatment, IDO-1 inhibiting treatment, or combinations thereof.

Para. BI. The method of any one of Paras. BE-BH, wherein the spatial proximity is assessed on a pixel scale.

Para. BJ. The method of any one of Paras. BE-BI, wherein the spatial proximity between the at least one pair of cells ranges from about 1 pixel to about 100 pixels.

Para. BK. The method of any one of Paras. BE-BJ, wherein the spatial proximity between the at least one pair of cells ranges from about 0.5 μm to about

50 $\mu$m.

Para. BL. The method of any one of Paras. BE-BK, wherein the first threshold value ranges from about 500 to about 5000.

Para. BM. The method of any one of Paras. BE-BL, wherein the first threshold value is about 900 plus or minus 100.

Para. BN. The method of any one of Paras. BE-BM, wherein the second threshold value ranges from about 2% to about 10%.

Para. BO. The method of any one of Paras. BE-BN, wherein the second threshold value is about 5% plus or minus 1%.

Para. BP. The method of any one of Paras. BE-BO, wherein the cancer patient is a melanoma cancer patient or a lung cancer patient.

Para. BQ. The method of any one of Paras. BE-BP, wherein the cancer patient is a melanoma cancer patient.

Para. BR. The method of any one of Paras. BE-BP, wherein the cancer patient is a non-small cell lung cancer patient.

Para. BS. The method of any one of Paras. BE-BR, wherein the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof.

Para. BT. A method of predicting a likelihood that a cancer patient will respond positively to adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if either the interaction score is greater than or equal to the first threshold value or the value for PBP is greater than or equal to the second threshold value, then the cancer patient is likely to respond positively to adjuvant chemotherapy.

Para. BU. A method of predicting a likelihood that a cancer patient will respond positively to adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to adjuvant chemotherapy.

Para. BV. The method of Para. BT or Para. BU, wherein the adjuvant chemotherapy targets the PD-1 and/or PD-L1 axis.

Para. BW. The method of Para. BT or Para. BU, wherein the adjuvant chemotherapy comprises anti-PD-1 treatment, anti-PD-Ll treatment, IDO-1 inhibiting treatment, or combinations thereof.

Para. BX. The method of any one of Paras. BT-BW, wherein the spatial proximity is assessed on a pixel scale.

Para. BY. The method of any one of Paras. BT-BX, wherein the spatial proximity between the at least one pair of cells ranges from about 1 pixel to about 100 pixels.

Para. BZ. The method of any one of Paras. BT-BY, wherein the spatial proximity between the at least one pair of cells ranges from about 0.5 $\mu$m to about 50 $\mu$m.

Para. CA. The method of any one of Paras. BT-BZ, wherein the first threshold value ranges from about 500 to about 5000.

Para. CB. The method of any one of Paras. BT-CA, wherein the first threshold value is about 700 plus or minus 100.

Para. CC. The method of any one of Paras. BT-CB, wherein the second threshold value ranges from about 2% to about 10%.

Para. CD. The method of any one of Paras. BT-CC, wherein the second threshold value is about 5% plus or minus 1%.

Para. CE. The method of any one of Paras. BT-CD, wherein the cancer patient is a melanoma cancer patient or a lung cancer patient.

Para. CF. The method of any one of Paras. BT-CE, wherein the cancer patient is a melanoma cancer patient.

Para. CG. The method of any one of Paras. BT-CE, wherein the cancer patient is a non-small cell lung cancer patient.

Para. CH. The method of any one of Paras. BT-CG, wherein the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof.

Para. CI. A method of treating cancer in a patient in need thereof, the method comprising

(A) predicting a likelihood that the patient will respond positively to immunotherapy using the method of any one of Paras. A-BS; and

(B) if the patient is likely to respond positively to immunotherapy, then administering immunotherapy to the patient.

Para. CJ. A method of treating cancer in a patient in need thereof, the method comprising

(A) predicting a likelihood that the patient will respond positively to adjuvant chemotherapy using the method of any one of Paras. BT-CH; and

(B) if the patient is likely to respond positively to adjuvant chemotherapy, then administering adjuvant chemotherapy to the patient.

Para. CK. A method of selecting a cancer patient who is likely to benefit from an immunotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all HLA-DR positive cells in a field of view of the sample expressing HLA-DR$^+$IDO-1$^+$, and recording the value for PBP; and

(C) comparing the interaction score to a first threshold value and comparing the value for PBP to a second threshold value;

wherein if either the interaction score is greater than or equal to the first threshold value or the value for PBP is greater than or equal to the second threshold value, then the cancer patient is likely to benefit from the immunotherapy.

Para. CL. A method of selecting a cancer patient who is likely to benefit from an immunotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all HLA-DR positive cells in a field of view of the sample expressing HLA-DR$^+$IDO-1$^+$, and recording the value for PBP; and

(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from the immunotherapy.

Para. CM. The method of Para. CK or Para. CL, wherein the immunotherapy targets the PD-1 and/or PD-L1 axis.

Para. CN. The method of Para. CK or Para. CL, wherein the immunotherapy comprises anti-PD-1 treatment, anti-PD-LI treatment, IDO-1 inhibiting treatment, or combinations thereof.

Para. CO. The method of any one of Paras. CK-CN, wherein the spatial proximity is assessed on a pixel scale.

Para. CP. The method of any one of Paras. CK-CO, wherein the spatial proximity between the at least one pair of cells ranges from about 1 pixel to about 100 pixels.

Para. CQ. The method of any one of Paras. CK-CP, wherein the spatial proximity between the at least one pair of cells ranges from about 0.5 $\mu$m to about 50 $\mu$m.

Para. CR. The method of any one of Paras. CK-CQ, wherein the first threshold value ranges from about 500 to about 5000.

Para. CS. The method of any one of Paras. CK-CR, wherein the first threshold value is about 900 plus or minus 100.

Para. CT. The method of any one of Paras. CK-CS, wherein the second threshold value ranges from about 2% to about 10%.

Para. CU. The method of any one of Paras. CK-CT, wherein the second threshold value is about 5% plus or minus 1%.

Para. CV. The method of any one of Paras. CK-CU, wherein the cancer patient is a melanoma cancer patient or a lung cancer patient.

Para. CW. The method of any one of Paras. CK-CV, wherein the cancer patient is a melanoma cancer patient.

Para. CX. A method of selecting a cancer patient who is likely to benefit from an immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-$DR^+$ cells present in a field of view expressing HLA-$DR^+$IDO-$1^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-$DR^+$;
constructing a third mask of third fluorescence signals representative of all areas present in the field of view, which express IDO-$1^+$;
combining said first and second masks in a manner that provides a fourth mask comprising fluorescence signals representative of all cells in the field of view, which also express HLA-$DR^+$;
combining said first and third masks in a manner that provides a fifth mask comprising fluorescence signals representative of all cells in the field of view, which also express IDO-$1^+$;
combining said fourth and fifth masks in a manner that provides a sixth mask comprising fluorescence signals representative of all cells in the field of view, which express HLA-$DR^+$ and IDO-$1^+$;
deriving the value for PBP for all cells present in the field of view expressing HLA-$DR^+$IDO-$1^+$ by dividing the total area of the sixth mask by the total area of the fourth mask; and
recording the value for PBP; and

C) comparing the spatial proximity score to a first threshold value and comparing the value for PBP to a second threshold value;

wherein if either the spatial proximity score is greater than or equal to the first threshold value or the value for PBP is greater than or equal to the second threshold value, then the cancer patient is likely to benefit from the immunotherapy.

Para. CY. A method of selecting a cancer patient who is likely to benefit from an immunotherapy, the method comprising:

(A) determining a score representative of a spatial proximity between at least one pair of cells selected from among a plurality of cells present in a predetermined number of fields of view available from a sample comprising tumor tissue, which sample is taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
for each of the selected fields of view, dilating fluorescence signals attributable to PD-L1 by a margin sufficient to encompass proximally located cells expressing PD-1;
dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-DR$^+$;
constructing a third mask of third fluorescence signals representative of all areas present in the field of view, which express IDO-1$^+$;
combining said first and second masks in a manner that provides a fourth mask comprising fluorescence signals representative of all cells in the field of view, which also express HLA-DR$^+$;
combining said first and third masks in a manner that provides a fifth mask comprising fluorescence signals representative of all cells in the field of view, which also express IDO-1$^+$;
combining said fourth and fifth masks in a manner that provides a sixth mask comprising fluorescence signals representative of all cells in the field of view, which express HLA-DR$^+$ and IDO-1 $^+$;
deriving the value for PBP for all cells present in the field of view expressing HLA-DR$^+$IDO-1$^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to benefit from the immunotherapy.

Para. CZ. The method of Para. CX or Para. CY, wherein the immunotherapy targets the PD-1 and/or PD-L1 axis.

Para. DA. The method of Para. CX or Para. CY, wherein the immunotherapy comprises anti-PD-1 treatment, anti-PD-LI treatment, anti-IDO-1 treatment, or combinations thereof.

Para. DB. The method of any one of Paras. CX-DA, wherein the total area of the fourth mask and the total area of the sixth mask from step (B) are measured in pixels.

Para. DC. The method of any one of Paras. CX-DB, wherein the spatial proximity is assessed on a pixel scale.

Para. DD. The method of any one of Paras. CX-DC, wherein each of the fluorescence tags is directed to a specific biomarker.

Para. DE. The method of any one of Paras. CX-DD, wherein the plurality of fluorescence tags comprises a first fluorescence tag for PD-1 and a second fluorescence tag for PD-L1.

Para. DF. The method of any one of Paras. CX-DE, wherein the margin ranges from about 1 to about 100 pixels.

Para. DG. The method of any one of Paras. CX-DF, wherein the proximally located cells expressing PD-L1 are within about 0.5 to about 50 μm of a plasma membrane of the cells that express PD-1.

Para. DH. The method of any one of Paras. CX-DG, wherein the first total area for all cells from each of the selected fields of view from step (A) is measured in pixels.

Para. DI. The method of any one of Paras. CX-DH, wherein the normalization factor is a second total area for all non-tumor cells from each of the selected fields of view.

Para. DJ. The method of any one of Paras. CX-DI, wherein the predetermined factor is $10^4$.

Para. DK. The method of any one of Paras. CX-DJ, wherein the first threshold value ranges from about 500 to about 5000.

Para. DL. The method of any one of Paras. CX-DK, wherein the first threshold value is about 900 plus or minus 100.

Para. DM. The method of any one of Paras. CX-DL, wherein the spatial proximity score (SPS) is determined by the following equation:

$$SPS = \frac{A_I}{A_C} \times 10^4$$

wherein $A_I$ is a total interaction area (total area of cells expressing PD-1 and encompassed by dilated fluorescence signals attributable to cells expressing PD-L1) and $A_C$ is the total area of cells that have a capacity to express the PD-1.

Para. DN. The method of any one of Paras. CX-DM, wherein the second threshold value ranges from about 2% to about 10%.

Para. DO. The method of any one of Paras. CX-DN, wherein the second threshold value is about 5% plus or minus 1%.

Para. DP. The method of any one of Paras. CX-DO, wherein the cancer patient is a melanoma cancer patient or a lung cancer patient.

Para. DQ. The method of any one of Paras. CX-DP, wherein the cancer patient is a melanoma cancer patient.

Para. DR. A method of selecting a cancer patient who is likely to benefit from an adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if either the interaction score is greater than or equal to the first threshold value or the value for PBP is greater than or equal to the second threshold value, then the cancer patient is likely to benefit from adjuvant chemotherapy.

Para. DS. A method of selecting a cancer patient who is likely to benefit from an adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all biomarker positive cells in a field of view of the sample, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from adjuvant chemotherapy.

Para. DT. The method of Para. DR or Para. DS, wherein the adjuvant chemotherapy targets the PD-1 and/or PD-L1 axis.

Para. DU. The method of Para. DR or Para. DS, wherein the adjuvant chemotherapy comprises anti-PD-1 treatment, anti-PD-Ll treatment, IDO-1 inhibiting treatment, or combinations thereof.

Para. DV. The method of any one of Paras. DR-DU, wherein the spatial proximity is assessed on a pixel scale.

Para. DW. The method of any one of Paras. DR-DV, wherein the spatial proximity between the at least one pair of cells ranges from about 1 pixel to about 100 pixels.

Para. DX. The method of any one of Paras. DR-DW, wherein the spatial proximity between the at least one pair of cells ranges from about 0.5 $\mu$m to about 50 $\mu$m.

Para. DY. The method of any one of Paras. DR-DX, wherein the first threshold value ranges from about 500 to about 5000.

Para. DZ. The method of any one of Paras. DR-DY, wherein the first threshold value is about 700 plus or minus 100.

Para. EA. The method of any one of Paras. DR-DZ, wherein the second threshold value ranges from about 2% to about 10%.

Para. EB. The method of any one of Paras. DR-EA, wherein the second threshold value is about 5% plus or minus 1%.

Para. EC. The method of any one of Paras. DR-EB, wherein the cancer patient is a melanoma cancer patient or a lung cancer patient.

Para. ED. The method of any one of Paras. DR-EC, wherein the cancer patient is a melanoma cancer patient.

Para. EE. The method of any one of Paras. DR-EC, wherein the cancer patient is a non-small cell lung cancer patient.

Para. EF. The method of any one of Paras. DR-EE, wherein the biomarker of step (B) is selected from CD11b, HLA-DR, Arginase 1, IDO-1, CD25, FoxP3, Granzyme B, CD56, CD68, CD163, Ki67, Tim3, Lag3, CD4, CD8, and a combination of two or more thereof.

Para. EG. A method of predicting a likelihood that a cancer patient will respond positively to adjuvant chemotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $CD25^+FoxP3^+$, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to adjuvant chemotherapy.

Para. EH. A method of selecting a cancer patient who is likely to benefit from an adjuvant chemotherapy, the method comprising:

(A) scoring a sample comprising tumor tissue taken from the cancer patient comprising: using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
(B) deriving a value for % biomarker positivity (PBP) for all T cells ($CD4^+$ or $CD8^+$) in a field of view of the sample expressing $CD25^+FoxP3^+$, and recording the value for PBP; and
(C) comparing the interaction score to a first threshold value and comparing the value for PBP to a second threshold value;

wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to benefit from adjuvant chemotherapy.

Para. EI. A method of treating cancer in a patient in need thereof, the method comprising

(A) predicting a likelihood that the patient will respond positively to immunotherapy using the method of any one of Paras. ; and
(B) if the patient is likely to respond positively to immunotherapy, then administering immunotherapy to the patient; or
(C) if the patient is unlikely to respond positively to immunotherapy, then administering to the patient (1) targeted therapy if a BRAF mutation is present, or (2) palliative surgery and/or radiation therapy and best supportive care if BRAF mutation is absent.

Para. EJ. A method of treating cancer in a patient in need thereof, the method comprising

(A) predicting a likelihood that the patient will respond positively to adjuvant chemotherapy using the method of any one of Paras. BT-CH; and
(B) if the patient is likely to respond positively to adjuvant chemotherapy, then administering adjuvant chemotherapy to the patient; or
(C) if the patient is unlikely to respond positively to adjuvant chemotherapy, then proceeding to mutation testing and (1) administering to the patient targeted therapy if the mutation testing is positive or (2) administering to the patient best supportive care if the mutation testing is negative.

Para. EK. A method of predicting a likelihood that a melanoma patient will respond positively to immunotherapy, the method comprising:

(A) deriving a value for % biomarker positivity (PBP) for all HLA-DR positive cells in a field of view of the sample expressing $HLA\text{-}DR^+IDO\text{-}1^+$, and recording the value for PBP; and
(B) comparing the value for PBP to a threshold value;

wherein if the value for PBP is greater than or equal to the threshold value, then the melanoma patient is likely to respond positively to immunotherapy.

Para. EL. A method of predicting a likelihood that a melanoma patient will respond positively to immunotherapy, the method comprising:

(A) deriving a value for % biomarker positivity (PBP) for all HLA-$DR^+$ cells present in a field of view expressing HLA-$DR^+$IDO-$1^+$, comprising:

generating an image of first fluorescence signals representative of nuclei of all cells present in a field of view, and dilating the first fluorescence signals to a diameter of that of an entire cell to construct a first mask of all cells present in the field of view;
constructing a second mask of second fluorescence signals representative of all areas present in the field of view, which express HLA-$DR^+$;
constructing a third mask of third fluorescence signals representative of all areas present in the field of view, which express IDO-$1^+$;
combining said first and second masks in a manner that provides a fourth mask comprising fluorescence signals representative of all cells in the field of view, which also express HLA-$DR^+$;
combining said first and third masks in a manner that provides a fifth mask comprising fluorescence signals representative of all cells in the field of view, which also express IDO-$1^+$;
combining said fourth and fifth masks in a manner that provides a sixth mask comprising fluorescence signals representative of all cells in the field of view, which express HLA-$DR^+$ and IDO-$1^+$;
deriving the value for PBP for all cells present in the field of view expressing HLA-$DR^+$IDO-$1^+$ by dividing the total area of the sixth mask by the total area of the fourth mask; and
recording the value for PBP; and

(B) comparing the value for PBP to a threshold value;

wherein if the value for PBP is greater than or equal to the threshold value, then the melanoma patient is likely to respond positively to immunotherapy.

Para. EM. A method of predicting a likelihood that a non-small cell lung cancer patient will respond positively to adjuvant chemotherapy, the method comprising:

(A) deriving a value for % biomarker positivity (PBP) for all T cells ($CD4^+$ or $CD8^+$) in a field of view of the sample expressing $CD25^+FoxP3^+$, and recording the value for PBP; and
(B) comparing the value for PBP to a threshold value;

wherein if the value for PBP is greater than or equal to the threshold value, then the non-small cell lung cancer patient is likely to respond positively to adjuvant chemotherapy.

Para. EN. A method of selecting a non-small cell lung cancer patient who is likely to benefit from an adjuvant chemotherapy, the method comprising:

(A) deriving a value for % biomarker positivity (PBP) for all T cells ($CD4^+$ or $CD8^+$) in a field of view of the sample expressing $CD25^+FoxP3^+$, and recording the value for PBP; and
(B) comparing the value for PBP to a threshold value;

wherein if the value for PBP is greater than or equal to the threshold value, then the non-small cell lung cancer patient is likely to benefit from adjuvant chemotherapy.

Para. EO. A method of predicting a likelihood that a non-small cell lung cancer patient will respond positively to adjuvant chemotherapy, the method comprising:

(A) deriving a value for % biomarker positivity (PBP) for all T cells ($CD4^+$ or $CD8^+$) in a field of view of the sample expressing $Ki67^+$, and recording the value for PBP; and
(B) comparing the value for PBP to a threshold value;

wherein if the value for PBP is less than the threshold value, then the non-small cell lung cancer patient is likely to respond positively to adjuvant chemotherapy.

Para. EP. A method of selecting a non-small cell lung cancer patient who is likely to benefit from an adjuvant chemotherapy, the method comprising:

(A) deriving a value for % biomarker positivity (PBP) for all T cells ($CD4^+$ or $CD8^+$) in a field of view of the sample expressing $Ki67^+$, and recording the value for PBP; and
(B) comparing the value for PBP to a threshold value;

wherein if the value for PBP is less than the threshold value, then the non-small cell lung cancer patient is likely to benefit from adjuvant chemotherapy.

Para. EQ. A method of predicting a likelihood that a cancer patient will respond positively to adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all T cells ($CD4^+$ or $CD8^+$) in a field of view of the sample expressing $Ki67^+$, and recording the value for PBP; and

(C) comparing the interaction score to a first threshold value and comparing the value for PBP to a second threshold value;

wherein if (1) either the interaction score is greater than or equal to the first threshold value or the value for PBP is less than the second threshold value, or (2) the interaction score is greater than or equal to the first threshold value and the value for PBP is less than the second threshold value, then the cancer patient is likely to respond positively to adjuvant chemotherapy.

Para. ER. A method of selecting a cancer patient who is likely to benefit from an adjuvant chemotherapy, the method comprising:

(A) scoring a sample comprising tumor tissue taken from the cancer patient comprising:
using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;

(B) deriving a value for % biomarker positivity (PBP) for all T cells ($CD4^+$ or $CD8^+$) in a field of view of the sample expressing $Ki67^+$, and recording the value for PBP; and

(C) comparing the interaction score to a first threshold value and comparing the value for PBP to a second threshold value;

wherein if (1) either the interaction score is greater than or equal to the first threshold value or the value for PBP is less than the second threshold value, or (2) the interaction score is greater than or equal to the first threshold value and the value for PBP is less than the second threshold value, then the cancer patient is likely to benefit from adjuvant chemotherapy.

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of' will be understood to include those elements specifically recited and those additional elements that do not materially affect the basic and novel characteristics of the claimed technology. The phrase "consisting of' excludes any element not specified.

The present disclosure is not to be limited in terms of the particular embodiments described in this application. Many modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can of course vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a nonlimiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

The scope of protection is defined in the following claims.

## Claims

1. A method of predicting a likelihood that a cancer patient will respond positively to immunotherapy, the method comprising:

   (A) scoring a sample comprising tumor tissue previously taken from the cancer patient comprising:
   using the sample comprising tumor tissue taken from the cancer patient, determining an interaction score representative of a spatial proximity between at least one pair of cells, a first member of the at least one pair of cells expressing PD-1 and a second member of the at least one pair of cells expressing PD-L1; and recording the interaction score;
   (B) deriving a value for % biomarker positivity (PBP) for all HLA-DR positive cells in a field of view of the sample expressing HLA-$DR^+$IDO-$1^+$, and recording the value for PBP; and
   (C) comparing the interaction score to a first threshold value and comparing the value for PBP to a second threshold value;

   wherein if (1) either the interaction score is greater than or equal to the first threshold value or the value for PBP is greater than or equal to the second threshold value, or (2) the interaction score is greater than or equal to the first threshold value and the value for PBP is greater than or equal to the second threshold value, then the cancer patient is likely to respond positively to immunotherapy.

2. The method of claim 1, wherein the immunotherapy targets the PD-1 and/or PD-L1 axis.

3. The method of claim 1, wherein the immunotherapy comprises anti-PD-1 treatment, anti-PD-LI treatment, IDO-1 inhibiting treatment, or combinations thereof.

4. The method of any one of claims 1-3, wherein the spatial proximity between the at least one pair of cells ranges from about 1 pixel to about 100 pixels, or wherein the spatial proximity between the at elast one pair of cells ranges from about 0.5 $\mu$m to about 50 $\mu$m.

5. The method of any one of claims 1-4, wherein the first threshold value ranges from about 500 to about 5000, preferably wherein the first threshold value is about 900 plus or minus 100.

6. The method of any one of claims 1-5, wherein the second threshold value ranges from about 2% to about 10%, preferably wherein the second threshold value is about 5% plus or minus 1%.

7. The method of any one of claims 1-6, wherein the cancer patient is a melanoma cancer patient or a lung cancer patient, preferably wherein the cancer patient is a non-small cell lung cancer patient.

8. A method of predicting a likelihood that a cancer patient will respond positively to immunotherapy, the method comprising:

   (A) determining a score representative of a spatial proximity between at least one pair of cells selected from among a plurality of cells present in a predetermined number of fields of view available from a sample comprising tumor tissue, which sample was previously taken from the cancer patient, the method comprising:

      selecting a predetermined number of fields of view available from the sample comprising tumor tissue taken from the cancer patient, which is stained with a plurality of fluorescence tags, which selection is biased toward selecting fields of view that contain a greater number of cells that express PD-L1 relative to other fields of view;
      for each of the selected fields of view, dilating fluorescence signals attributable to PD-L1 by a margin sufficient to encompass proximally located cells expressing PD-1;
      dividing a first total area for all cells from each of the selected fields of view, which express PD-1 and are encompassed within the dilated fluorescence signals attributable to the cells expressing PD-L1, with a normalization factor, and multiplying the resulting quotient by a predetermined factor to arrive at a spatial proximity score; and
      recording the spatial proximity score;

   (B) deriving a value for % biomarker positivity (PBP) for all HLA-$DR^+$ cells present in a field of view expressing HLA-$DR^+$IDO-$1^+$, comprising:

      generating an image of first fluorescence signals representative of nuclei of all cells present in a field of view, and dilating the first fluorescence signals to a diameter of that of an entire cell to construct a first mask of all cells present in the field of view;
      constructing a second mask of second fluorescence signals representative of all areas present in the field of view, which express HLA-$DR^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-DR$^+$ and IDO-1$^+$;

deriving the value for PBP for all cells present in the field of view expressing HLA-DR$^+$IDO-1$^+$ by dividing the total area of the sixth mask by the total area of the fourth mask; and

recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

**9.** The method of claim 8, wherein the predetermined factor is $10^4$.

**10.** The method of claim 8 or 9, wherein the first threshold value ranges from about 500 to about 5000, preferably wherein the first threshold value is about 900 plus or minus 100.

**11.** The method of any one of claims 8-10, wherein the second threshold value ranges from about 2% to about 10%, preferably wherein the second threshold value is about 5% plus or minus 1%.

**12.** A method of predicting a likelihood that a cancer patient will respond positively to immunotherapy, the method comprising:

(A) determining a score representative of a spatial proximity between at least one pair of cells selected from among a plurality of cells present in a predetermined number of fields of view available from a sample comprising tumor tissue, which sample was previously taken from the cancer patient, the method comprising:

selecting a predetermined number of fields of view available from the sample comprising tumor tissue taken from the cancer patient, which is stained with a plurality of fluorescence tags, which selection is biased toward selecting fields of view that contain a greater number of cells that express PD-1 relative to other fields of view;

for each of the selected fields of view, dilating fluorescence signals attributable to PD-1 by a margin sufficient to encompass proximally located cells expressing PD-L1;

dividing a first total area for all cells from each of the selected fields of view, which express PD-L1 and are encompassed within the dilated fluorescence signals attributable to the cells expressing PD-1, with a normalization factor, and multiplying the resulting quotient by a predetermined factor to arrive at a spatial proximity score; and

recording the spatial proximity score;

(B) deriving a value for % biomarker positivity (PBP) for all HLA-DR$^+$ cells present in a field of view expressing HLA-DR$^+$IDO-1$^+$, comprising:

generating an image of first fluorescence signals representative of nuclei of all cells present in a field of view, and dilating the first fluorescence signals to a diameter of that of an entire cell to construct a first mask of all cells present in the field of view;

constructing a second mask of second fluorescence signals representative of all areas present in the field of view, which express HLA-DR$^+$;

constructing a third mask of third fluorescence signals representative of all areas present in the field of view, which express IDO-1$^+$;

combining said first and second masks in a manner that provides a fourth mask comprising fluorescence signals representative of all cells in the field of view, which also express HLA-DR$^+$;

combining said first and third masks in a manner that provides a fifth mask comprising fluorescence signals representative of all cells in the field of view, which also express IDO-1$^+$;

combining said fourth and fifth masks in a manner that provides a sixth mask comprising fluorescence signals representative of all cells in the field of view, which express HLA-$DR^+$ and IDO-$1^+$;
deriving the value for PBP for all cells present in the field of view expressing HLA-$DR^+$IDO-$1^+$ by dividing the total area of the sixth mask by the total area of the fourth mask; and
recording the value for PBP; and

(C) comparing the spatial proximity score to a first threshold value and comparing the value for PBP to a second threshold value;

wherein if (1) either the spatial proximity score is greater than or equal to the first threshold value or the value for PBP is greater than or equal to the second threshold value, or (2) the spatial proximity score is greater than or equal to the first threshold value and the value for PBP is greater than or equal to the second threshold value, then the cancer patient is likely to respond positively to immunotherapy.

**13.** The method of claim 12, wherein the predetermined factor is $10^4$.

**14.** The method of claim 12 or 13, wherein the first threshold value ranges from about 500 to about 5000, preferably wherein the first threshold value is about 900 plus or minus 100.

**15.** The method of any one of claims 12-14, wherein the second threshold value ranges from about 2% to about 10%, preferably wherein the second threshold value is about 5% plus or minus 1%.

## Patentansprüche

**1.** Verfahren zum Vorhersagen einer Wahrscheinlichkeit, dass ein Krebspatient positiv auf eine Immuntherapie ansprechen wird, wobei das Verfahren Folgendes aufweist:

(A) Bewerten einer Tumorgewebe aufweisenden Probe, die dem Krebspatienten zuvor entnommen wurde, was Folgendes aufweist:

Verwenden der Tumorgewebe aufweisenden Probe, die dem Krebspatienten entnommen wurde, Bestimmen eines Interaktionsscores bzw. Interaktionswertes, der für eine räumliche Nähe zwischen mindestens einem Paar von Zellen repräsentativ ist, wobei ein erstes Teil des mindestens einen Paares von Zellen PD-1 exprimiert, und wobei ein zweites Teil des mindestens einen Paares von Zellen PD-L1 exprimiert; und
Aufzeichnen des Interaktionswertes;

(B) Ableiten eines Wertes für die prozentuale Biomarker-Positivität (PBP) für alle HLA-DR-positiven Zellen in einem Sichtfeld der Probe, die HLA-$DR^+$IDO-$1^+$ exprimiert, und Aufzeichnen des Wertes für die PBP; und
(C) Vergleichen des Interaktionswertes mit einem ersten Schwellenwert und Vergleichen des Werts für die PBP mit einem zweiten Schwellenwert;
wobei, wenn (1) entweder der Interaktionswert größer als oder gleich dem ersten Schwellenwert ist oder der Wert für PBP größer als oder gleich dem zweiten Schwellenwert ist, oder (2) der Interaktionswert größer als oder gleich dem ersten Schwellenwert ist und der Wert für PBP größer als oder gleich dem zweiten Schwellenwert ist, der Krebspatient dann wahrscheinlich positiv auf die Immuntherapie ansprechen wird.

**2.** Verfahren nach Anspruch 1, wobei die Immuntherapie auf die PD-1 und/oder die PD-L1-Achse abzielt.

**3.** Verfahren nach Anspruch 1, wobei die Immuntherapie eine Anti-PD-1-Behandlung, eine Anti-PD-L1-Behandlung, eine IDO-1-inhibierende Behandlung oder Kombinationen davon aufweist.

**4.** Verfahren nach einem der Ansprüche 1-3, wobei die räumliche Nähe zwischen dem mindestens einen Paar von Zellen im Bereich von ungefähr 1 Pixel bis ungefähr 100 Pixel liegt, oder wobei die räumliche Nähe zwischen dem mindesten einen Paar von Zellen im Bereich von ungefähr 0,5 $\mu$m bis ungefähr 50 $\mu$m liegt.

**5.** Verfahren nach einem der Ansprüche 1-4, wobei der erste Schwellenwert im Bereich von ungefähr 500 bis ungefähr 5000 liegt, vorzugsweise wobei der erste Schwellenwert ungefähr 900 plus oder minus 100 beträgt.

**6.** Verfahren nach einem der Ansprüche 1-5, wobei der zweite Schwellenwert im Bereich von ungefähr 2 % bis ungefähr 10 % liegt, vorzugsweise wobei der zweite Schwellenwert ungefähr 5 % plus oder minus 1 % ist.

**7.** Verfahren nach einem der Ansprüche 1-6, wobei der Krebspatient ein Patient mit Melanom oder ein Patient mit einem Lungenkarzinom ist, wobei vorzugsweise der Krebspatient ein Patient mit nicht-kleinzelligem Bronchialkarzinom ist.

**8.** Verfahren zum Vorhersagen der Wahrscheinlichkeit, dass ein Krebspatient positiv auf eine Immuntherapie ansprechen wird, wobei das Verfahren Folgendes aufweist:

(A) Bestimmen eines Wertes, der repräsentativ ist für eine räumliche Nähe zwischen mindestens einem Paar von Zellen, das aus einer Vielzahl von Zellen ausgewählt ist, die in einer vorbestimmten Anzahl von Sichtfeldern vorhanden sind, die von einer ein Tumorgewebe aufweisenden Probe verfügbar sind, wobei diese Probe dem Krebspatienten zuvor entnommen wurde, wobei das Verfahren Folgendes aufweist:

Auswählen einer vorbestimmten Anzahl von Sichtfeldern, die aus der Probe verfügbar sind, die Tumorgewebe, das dem Krebspatienten entnommen wurde, aufweist, die mit einer Vielzahl von Fluoreszenzmarkern gefärbt ist, wobei die Auswahl auf die Auswahl von Sichtfeldern ausgerichtet ist, die relativ zu anderen Sichtfeldern eine größere Anzahl von Zellen aufweisen, die PD-L1 exprimieren;
Erweitern der Fluoreszenzsignale für jedes der ausgewählten Sichtfelder, die PD-L1 zuzuordnen sind, um einen Rand, der ausreicht, um proximal gelegene Zellen, die PD-1 exprimieren, zu erfassen;
Dividieren einer ersten Gesamtfläche für alle Zellen aus jedem der ausgewählten Sichtfelder, die PD-1 exprimieren und die innerhalb der erweiterten Fluoreszenzsignale erfasst sind, die den Zellen zugeordnet sind, die PD-L1 exprimieren, durch einen Normalisierungsfaktor bzw. Normierungsfaktor und Multiplizieren des resultierenden Quotienten mit einem vorbestimmten Faktor, um zu einem Wert der räumlichen Nähe zu gelangen; und Aufzeichnen des Wertes der räumlichen Nähe;

B) Ableiten eines Wertes für die prozentuale Biomarker-Positivität (PBP) für alle HLA-DR$^+$-Zellen, die in einem Sichtfeld vorhanden sind, das HLA-DR$^+$IDO-1$^+$ exprimiert, was Folgendes aufweist:

Erzeugen eines Bildes von ersten Fluoreszenzsignalen, die für Kerne aller in einem Sichtfeld vorhandenen Zellen repräsentativ sind, und Erweitern der ersten Fluoreszenzsignale auf einen Durchmesser von einer ganzen Zelle, um eine erste Maske aller im Sichtfeld vorhandenen Zellen zu konstruieren;
Konstruieren einer zweiten Maske von zweiten Fluoreszenzsignalen, die für alle im Sichtfeld vorhandenen Bereiche repräsentativ sind, welche HLA-DR$^+$ exprimieren;
Konstruieren einer dritten Maske aus dritten Fluoreszenzsignalen, die für alle im Sichtfeld vorhandenen Bereiche repräsentativ sind, welche IDO-1$^+$ exprimieren;
Kombinieren der ersten und zweiten Maske auf eine Weise, die eine vierte Maske liefert, die Fluoreszenzsignale aufweist, die für alle Zellen im Sichtfeld repräsentativ sind, die auch HLA-DR$^+$ exprimieren;
Kombinieren der ersten und dritten Maske auf eine Weise, die eine fünfte Maske liefert, die Fluoreszenzsignale aufweist, die für alle Zellen im Sichtfeld repräsentativ sind, die auch IDO-1$^+$ exprimieren;
Kombinieren der vierten und fünften Maske auf eine Weise, die eine sechste Maske liefert, die Fluoreszenzsignale aufweist, die für alle Zellen im Sichtfeld repräsentativ sind, die HLA-DR$^+$ und IDO-1$^+$ exprimieren;
Ableiten des Wertes für PBP für alle im Sichtfeld vorhandenen Zellen, die HLA-DR$^+$IDO-1$^+$ exprimieren, durch Dividieren der Gesamtfläche der sechsten Maske durch die Gesamtfläche der vierten Maske; und
Aufzeichnen des Wertes für PBP; und

(C) Vergleichen des Wertes für räumliche Nähe mit einem ersten Schwellenwert und Vergleichen des Werts für PBP mit einem zweiten Schwellenwert;
wobei, wenn (1) entweder der Wert für räumliche Nähe größer als oder gleich dem ersten Schwellenwert ist oder der Wert für PBP größer als oder gleich dem zweiten Schwellenwert ist, oder (2) der Wert für die räumliche Nähe größer als oder gleich dem ersten Schwellenwert ist und der Wert für PBP größer als oder gleich dem zweiten Schwellenwert ist, dann der Krebspatient wahrscheinlich positiv auf die Immuntherapie ansprechen wird.

**9.** Verfahren nach Anspruch 8, wobei der vorbestimmte Faktor $10^4$ ist.

**10.** Verfahren nach Anspruch 8 oder 9, wobei der erste Schwellenwert im Bereich von ungefähr 500 bis ungefähr 5000 liegt, vorzugsweise wobei der erste Schwellenwert ungefähr 900 plus oder minus 100 ist.

**11.** Verfahren nach einem der Ansprüche 8-10, wobei der zweite Schwellenwert im Bereich von ungefähr 2 % bis ungefähr 10 % liegt, vorzugsweise wobei der zweite Schwellenwert ungefähr 5 % plus oder minus 1 % ist.

**12.** Verfahren zum Vorhersagen der Wahrscheinlichkeit, dass ein Krebspatient positiv auf eine Immuntherapie ansprechen wird, wobei das Verfahren Folgendes aufweist:

(A) Bestimmen eines Wertes, der für eine räumliche Nähe zwischen mindestens einem Paar von Zellen repräsentativ ist, das aus einer Vielzahl von Zellen ausgewählt ist, die in einer vorbestimmten Anzahl von Sichtfeldern vorhanden sind, die aus einer Tumorgewebe aufweisenden Probe verfügbar sind, wobei diese Probe dem Krebspatienten zuvor entnommen wurde, wobei das Verfahren Folgendes aufweist:

Auswählen einer vorbestimmten Anzahl von Sichtfeldern, die aus der Probe, die Tumorgewebe aufweist, verfügbar sind, welches dem Krebspatienten entnommen wurde, die mit einer Vielzahl von Fluoreszenzmarkern gefärbt ist, wobei diese Auswahl auf das Auswählen von Sichtfeldern ausgerichtet ist, die relativ zu anderen Sichtfeldern eine größere Anzahl von Zellen aufweisen, die PD-1 exprimieren;
Ausweiten der Fluoreszenzsignale für jedes der ausgewählten Sichtfelder, die PD-1 zuzuschreiben sind, um einen Rand, der ausreicht, um proximal gelegene Zellen, die PD-L1 exprimieren, zu erfassen;
Dividieren einer ersten Gesamtfläche für alle Zellen von jedem der ausgewählten Sichtfelder, die PD-L1 exprimieren und innerhalb der erweiterten Fluoreszenzsignalen erfasst sind, die den Zellen zugeordnet sind, die PD-1 exprimieren, durch einen Normalisierungsfaktor und Multiplizieren des resultierenden Quotienten mit einem vorbestimmten Faktor, um zu einem Wert für räumliche Nähe zu gelangen; und
Aufzeichnen des Wertes für räumlichen Nähe;

(B) Ableiten eines Wertes für die prozentuale Biomarker-Positivität (PBP) für alle HLA-$DR^+$-Zellen, die in einem Sichtfeld vorhanden sind, das HLA-$DR^+$IDO-$1^+$ exprimiert, was Folgendes aufweist:

Erzeugen eines Bildes von ersten Fluoreszenzsignalen, die für Kerne aller in einem Sichtfeld vorhandenen Zellen repräsentativ sind, und Erweitern der ersten Fluoreszenzsignale auf einen Durchmesser von einer ganzen Zelle, um eine erste Maske aller im Sichtfeld vorhandenen Zellen zu konstruieren;
Konstruieren einer zweiten Maske von zweiten Fluoreszenzsignalen, die für alle im Sichtfeld vorhandenen Bereiche repräsentativ sind, welche HLA-$DR^+$ exprimieren;
Konstruieren einer dritten Maske aus dritten Fluoreszenzsignalen, die für alle im Sichtfeld vorhandenen Bereiche repräsentativ sind, welche IDO-$1^+$ exprimieren;
Kombinieren der ersten und zweiten Maske auf eine Weise, die eine vierte Maske liefert, die Fluoreszenzsignale aufweist, die für alle Zellen im Sichtfeld repräsentativ sind, die auch HLA-$DR^+$ exprimieren;
Kombinieren der ersten und dritten Maske auf eine Weise, die eine fünfte Maske liefert, die Fluoreszenzsignale aufweist, die für alle Zellen im Sichtfeld repräsentativ sind, die auch IDO-$1^+$ exprimieren;
Kombinieren der vierten und fünften Maske auf eine Weise, die eine sechste Maske vorsieht, die Fluoreszenzsignale aufweist, die für alle Zellen im Sichtfeld repräsentativ sind, die HLA-$DR^+$ und IDO-$1^+$ exprimieren;
Ableiten des Wertes für PBP für alle im Sichtfeld vorhandenen Zellen, die HLA-$DR^+$ IDO-$1^+$ exprimieren, durch Dividieren der Gesamtfläche der sechsten Maske durch die Gesamtfläche der vierten Maske; und
Aufzeichnen des Wertes für PBP; und

(C) Vergleichen des Wertes für räumliche Nähe mit einem ersten Schwellenwert und Vergleichen des Werts für PBP mit einem zweiten Schwellenwert;
wobei, wenn (1) entweder der Wert für räumliche Nähe größer als oder gleich dem ersten Schwellenwert ist oder der Wert für PBP größer als oder gleich dem zweiten Schwellenwert ist, oder (2) der Wert für die räumliche Nähe größer als oder gleich dem ersten Schwellenwert ist und der Wert für PBP größer als oder gleich dem zweiten Schwellenwert ist, der Krebspatient dann wahrscheinlich positiv auf die Immuntherapie ansprechen wird.

**13.** Verfahren nach Anspruch 12, wobei der vorbestimmte Faktor $10^4$ ist.

**14.** Verfahren nach Anspruch 12 oder 13, wobei der erste Schwellenwert im Bereich von ungefähr 500 bis ungefähr 5000 liegt, vorzugsweise wobei der erste Schwellenwert ungefähr 900 plus oder minus 100 ist.

**15.** Verfahren nach einem der Ansprüche 12-14, wobei der zweite Schwellenwert im Bereich von ungefähr 2 % bis ungefähr 10 %, vorzugsweise wobei der zweite Schwellenwert ungefähr 5 % plus oder minus 1 % ist.

**Revendications**

**1.** Procédé de prédiction de la probabilité qu'un patient atteint de cancer réponde positivement à une immunothérapie, ledit procédé comprenant :

(A) l'évaluation du score d'un échantillon comprenant un tissu tumoral antérieurement prélevé chez le patient atteint de cancer, comprenant : l'utilisation de l'échantillon comprenant le tissu tumoral prélevé chez le patient atteint de cancer, la détermination d'un score d'interaction représentatif d'une proximité spatiale entre au moins une paire de cellules, un premier membre de ladite au moins une paire de cellules exprimant PD-1 et un second membre de ladite au moins une paire de cellules exprimant PD-L1; et l'enregistrement du score d'interaction ;
(B) la déduction d'une valeur pour le % de positivité du biomarqueur (PBP) pour toutes les cellules positives au HLA-DR dans un champ de vision de l'échantillon exprimant HLA-$DR^{+}$IDO-$1^{+}$, et l'enregistrement de la valeur du PBP ; et
(C) la comparaison du score d'interaction à une première valeur seuil et la comparaison de la valeur du PBP à une deuxième valeur seuil ;

dans lequel si (1) soit le score d'interaction est supérieur ou égal à la première valeur seuil ou la valeur du PBP est supérieure ou égale à la deuxième valeur seuil, (2) soit le score d'interaction est supérieur ou égal à la première valeur seuil et la valeur du PBP est supérieure ou égale à la deuxième valeur seuil, alors le patient atteint de cancer est susceptible de répondre positivement à l'immunothérapie.

**2.** Procédé selon la revendication 1, dans lequel l'immunothérapie cible l'axe PD-1 et/ou PD-L1.

**3.** Procédé selon la revendication 1, dans lequel l'immunothérapie comprend un traitement anti-PD-1, un traitement anti-PD-L1, un traitement d'inhibition d'IDO-1, ou des combinaisons de ceux-ci.

**4.** Procédé selon l'une quelconque des revendications 1-3, dans lequel la proximité spatiale entre ladite au moins une paire de cellules s'étend d'environ 1 pixel à environ 100 pixels, ou dans lequel la proximité spatiale entre ladite au moins une paire de cellules s'étend d'environ 0,5 $\mu$m à environ 50 $\mu$m.

**5.** Procédé selon l'une quelconque des revendications 1-4, dans lequel la première valeur seuil s'étend d'environ 500 à environ 5000, de préférence, dans lequel la première valeur seuil est d'environ 900 plus ou moins 100.

**6.** Procédé selon l'une quelconque des revendications 1-5, dans lequel la deuxième valeur seuil s'étend d'environ 2% à environ 10%, de préférence, dans lequel la deuxième valeur seuil est d'environ 5% plus ou moins 1%.

**7.** Procédé selon l'une quelconque des revendications 1-6, dans lequel le patient atteint de cancer est un patient atteint de cancer du mélanome ou un patient atteint de cancer du poumon, de préférence, dans lequel le patient atteint de cancer est un patient atteint de cancer du poumon non à petites cellules.

**8.** Procédé de prédiction de la probabilité qu'un patient atteint de cancer réponde positivement à une immunothérapie, ledit procédé comprenant:

(A) la détermination d'un score représentatif d'une proximité spatiale entre au moins une paire de cellules choisies parmi une pluralité de cellules présentes dans un nombre prédéterminé de champs de vision disponibles à partir d'un échantillon comprenant un tissu tumoral, lequel échantillon a été antérieurement prélevé chez le patient atteint de cancer, ledit procédé comprenant :

la sélection d'un nombre prédéterminé de champs de vision disponibles à partir de l'échantillon comprenant un tissu tumoral prélevé chez le patient atteint de cancer, qui est coloré à l'aide d'une pluralité d'étiquettes fluorescentes, laquelle sélection est biaisée en faveur de la sélection des champs de vision qui contiennent un nombre plus élevé de cellules qui expriment PD-L1 par rapport à d'autres champs de vision ;
pour chacun des champs de vision sélectionnés, la dilatation des signaux de fluorescence attribuables à PD-L1 par une marge suffisante pour englober les cellules situées à proximité, exprimant PD-1 ;
la division d'une première surface totale pour toutes les cellules de chacun des champs de vision sélectionnés, qui expriment PD-1 et sont englobées dans les signaux de fluorescence dilatés attribuables aux cellules exprimant PD-L1, par un facteur de normalisation, et la multiplication du quotient résultant par un facteur prédéterminé pour arriver à un score de proximité spatiale ; et
l'enregistrement du score de proximité spatiale ;

(B) la déduction d'une valeur pour le % de positivité du biomarqueur (PBP) pour toutes les cellules HLA-$DR^{+}$ présentes dans un champ de vision exprimant HLA-DR⁺IDO-1⁺, comprenant :

la génération d'une image de premiers signaux de fluorescence représentatifs des noyaux de toutes les cellules présentes dans un champ de vision, et la dilatation des premiers signaux de fluorescence à un diamètre de celui d'une cellule entière pour construire un premier masque de toutes les cellules présentes dans le champ de vision ;
la construction d'un deuxième masque des deuxièmes signaux de fluorescence représentatifs de toutes les régions présentes dans le champ de vision, qui expriment HLA-DR⁺ ;
la construction d'un troisième masque des troisièmes signaux de fluorescence représentatifs de toutes les régions présentes dans le champ de vision, qui expriment IDO-1⁺;
la combinaison desdits premier et deuxième masques de manière à obtenir un quatrième masque comprenant les signaux de fluorescence représentatifs de toutes les cellules dans le champ de vision, qui expriment également HLA-DR⁺ ;
la combinaison desdits premier et troisième masques de manière à obtenir un cinquième masque comprenant les signaux de fluorescence représentatifs de toutes les cellules dans le champ de vision, qui expriment également IDO-1⁺;
la combinaison desdits quatrième et cinquième masques de manière à obtenir un sixième masque comprenant les signaux de fluorescence représentatifs de toutes les cellules dans le champ de vision, qui expriment HLA-DR⁺ et IDO-1⁺ ;
la déduction de la valeur du PBP pour toutes les cellules présentes dans le champ de vision exprimant HLA-DR⁺IDO-1⁺ en divisant la surface totale du sixième masque par la surface totale du quatrième masque ; et
l'enregistrement de la valeur du PBP ; et

(C) la comparaison du score de proximité spatiale à une première valeur seuil et la comparaison de la valeur du PBP à une deuxième valeur seuil ; dans lequel si (1) soit le score de proximité spatiale est supérieur ou égal à la première valeur seuil ou la valeur du PBP est supérieure ou égale à la deuxième valeur seuil, (2) soit le score de proximité spatiale est supérieur ou égal à la première valeur seuil et la valeur du PBP est supérieure ou égale à la deuxième valeur seuil, alors le patient atteint de cancer est susceptible de répondre positivement à l'immunothérapie.

**9.** Procédé selon la revendication 8, dans lequel le facteur prédéterminé est de $10^4$.

**10.** Procédé selon la revendication 8 ou 9, dans lequel la première valeur seuil s'étend d'environ 500 à environ 5000, de préférence, dans lequel la première valeur seuil est d'environ 900 plus ou moins 100.

**11.** Procédé selon l'une quelconque des revendications 8-10, dans lequel la deuxième valeur seuil s'étend d'environ 2% à environ 10%, de préférence, dans lequel la deuxième valeur seuil est d'environ 5% plus ou moins 1%.

**12.** Procédé de prédiction de la probabilité qu'un patient atteint de cancer réponde positivement à une immunothérapie, ledit procédé comprenant:

(A) la détermination d'un score représentatif d'une proximité spatiale entre au moins une paire de cellules choisies parmi une pluralité de cellules présentes dans un nombre prédéterminé de champs de vision disponibles à partir d'un échantillon comprenant un tissu tumoral, lequel échantillon a été antérieurement prélevé chez le patient atteint de cancer, ledit procédé comprenant :

la sélection d'un nombre prédéterminé de champs de vision disponibles à partir de l'échantillon comprenant un tissu tumoral prélevé chez le patient atteint de cancer, qui est coloré à l'aide d'une pluralité d'étiquettes fluorescentes, laquelle sélection est biaisée en faveur de la sélection des champs de vision qui contiennent un nombre plus élevé de cellules qui expriment PD-1 par rapport à d'autres champs de vision ;
pour chacun des champs de vision sélectionnés, la dilatation des signaux de fluorescence attribuables à PD-1 par une marge suffisante pour englober les cellules situées à proximité, exprimant PD-L1;
la division d'une première surface totale pour toutes les cellules de chacun des champs de vision sélectionnés, qui expriment PD-L1 et sont englobées dans les signaux de fluorescence dilatés attribuables aux cellules exprimant PD-1, par un facteur de normalisation, et la multiplication du quotient résultant par un facteur prédéterminé pour arriver à un score de proximité spatiale ; et
l'enregistrement du score de proximité spatiale ;

(B) la déduction d'une valeur pour le % de positivité du biomarqueur (PBP) pour toutes les cellules HLA-DR+ présentes dans un champ de vision exprimant HLA-DR+IDO-1+, comprenant :

la génération d'une image de premiers signaux de fluorescence représentatifs des noyaux de toutes les cellules présentes dans un champ de vision, et la dilatation des premiers signaux de fluorescence à un diamètre de celui d'une cellule entière pour construire un premier masque de toutes les cellules présentes dans le champ de vision ;
la construction d'un deuxième masque des deuxièmes signaux de fluorescence représentatifs de toutes les régions présentes dans le champ de vision, qui expriment HLA-DR+ ;
la construction d'un troisième masque des troisièmes signaux de fluorescence représentatifs de toutes les régions présentes dans le champ de vision, qui expriment IDO-1+;
la combinaison desdits premier et deuxième masques de manière à obtenir un quatrième masque comprenant les signaux de fluorescence représentatifs de toutes les cellules dans le champ de vision, qui expriment également HLA-DR+ ;
la combinaison desdits premier et troisième masques de manière à obtenir un cinquième masque comprenant les signaux de fluorescence représentatifs de toutes les cellules dans le champ de vision, qui expriment également IDO-1+;
la combinaison desdits quatrième et cinquième masques de manière à obtenir un sixième masque comprenant les signaux de fluorescence représentatifs de toutes les cellules dans le champ de vision, qui expriment HLA-DR+ et IDO-1+ ;
la déduction de la valeur du PBP pour toutes les cellules présentes dans le champ de vision exprimant HLA-DR+IDO-1+ en divisant la surface totale du sixième masque par la surface totale du quatrième masque ; et
l'enregistrement de la valeur du PBP ; et

(C) la comparaison du score de proximité spatiale à une première valeur seuil et la comparaison de la valeur du PBP à une deuxième valeur seuil ; dans lequel si (1) soit le score de proximité spatiale est supérieur ou égal à la première valeur seuil ou la valeur du PBP est supérieure ou égale à la deuxième valeur seuil, (2) soit le score de proximité spatiale est supérieur ou égal à la première valeur seuil et la valeur du PBP est supérieure ou égale à la deuxième valeur seuil, alors le patient atteint de cancer est susceptible de répondre positivement à l'immunothérapie.

**13.** Procédé selon la revendication 12, dans lequel le facteur prédéterminé est de $10^4$.

**14.** Procédé selon la revendication 12 ou 13, dans lequel la première valeur seuil s'étend d'environ 500 à environ 5000, de préférence, dans lequel la première valeur seuil est d'environ 900 plus ou moins 100.

**15.** Procédé selon l'une quelconque des revendications 12-14, dans lequel la deuxième valeur seuil s'étend d'environ 2% à environ 10%, de préférence, dans lequel la deuxième valeur seuil est d'environ 5% plus ou moins 1%.

FIG. 1

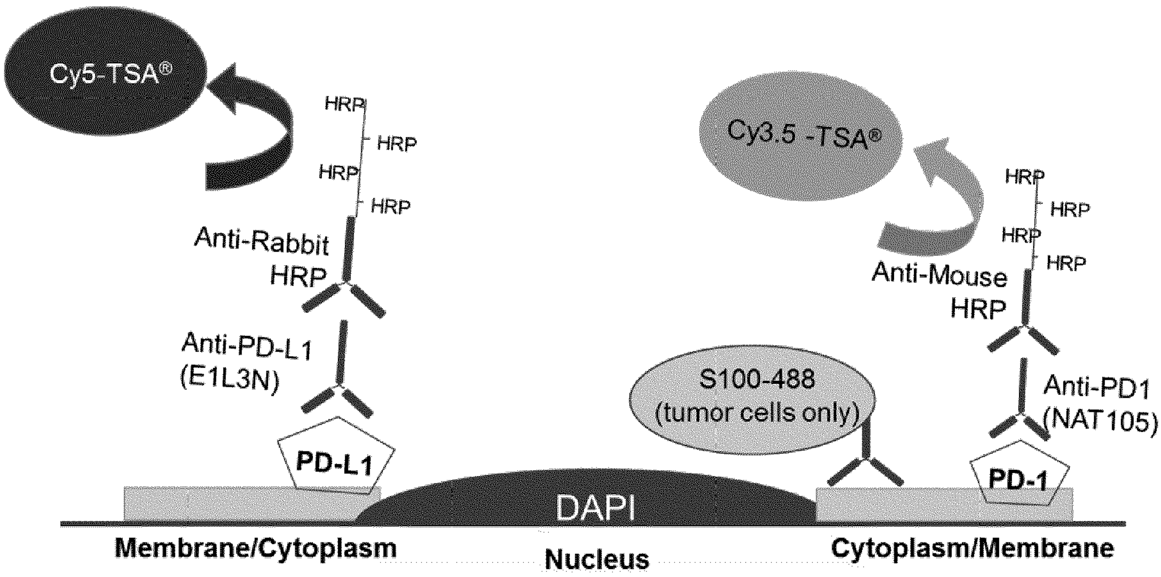
Cy5-TSA®
HRP
HRP
HRP
HRP
Anti-Rabbit HRP
Anti-PD-L1 (E1L3N)
PD-L1
Cy3.5 -TSA®
HRP
HRP
HRP
HRP
Anti-Mouse HRP
S100-488 (tumor cells only)
Anti-PD1 (NAT105)
PD-1
DAPI
Membrane/Cytoplasm
Nucleus
Cytoplasm/Membrane

FIG. 2a

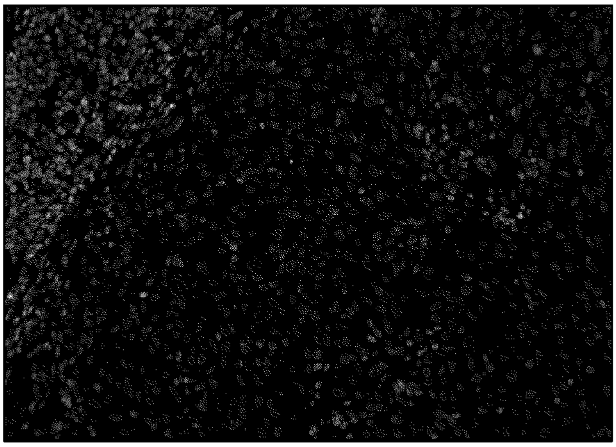

FIG. 2b

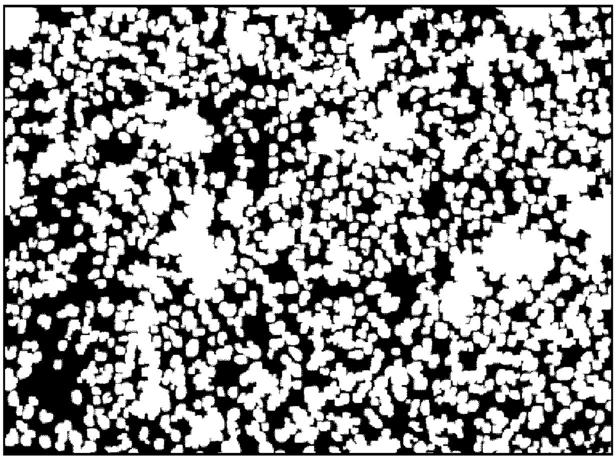

FIG. 3a

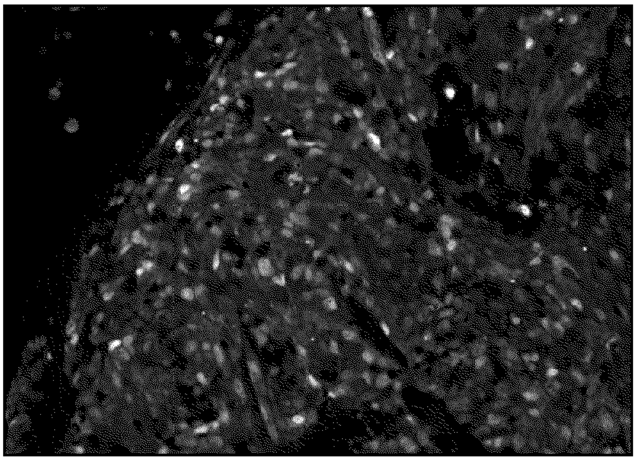

FIG. 3b

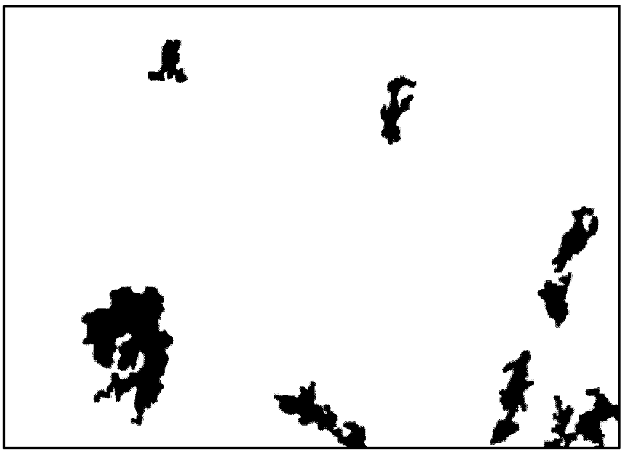

FIG. 3c

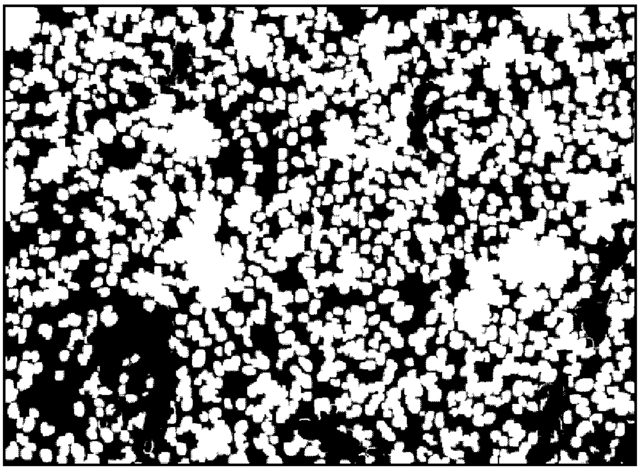

FIG. 3d

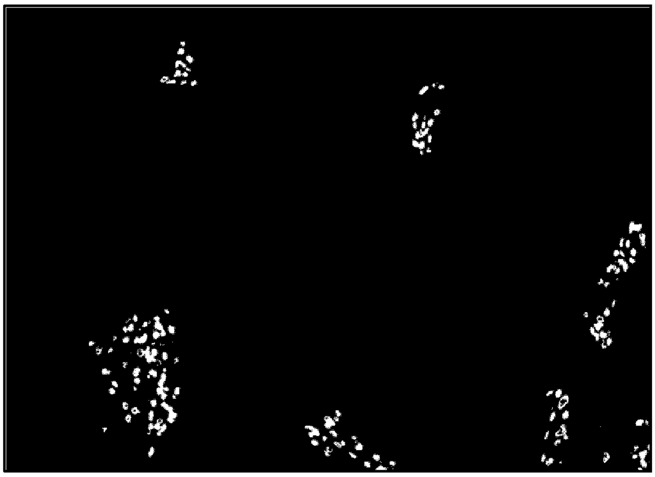

FIG. 4a

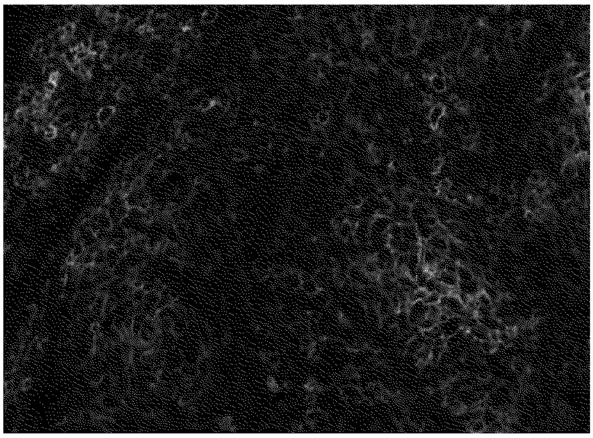

FIG. 4b

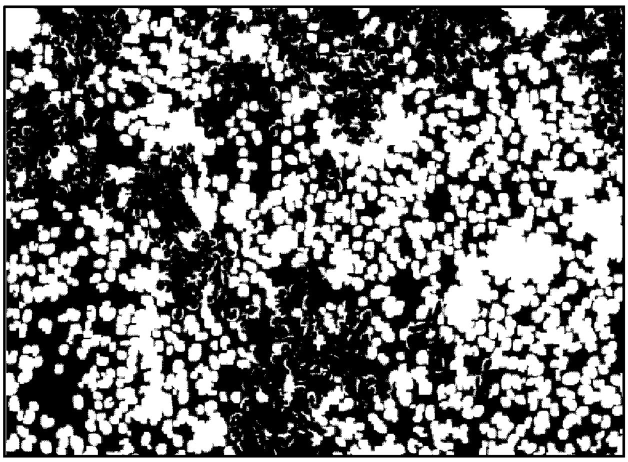

FIG. 5a

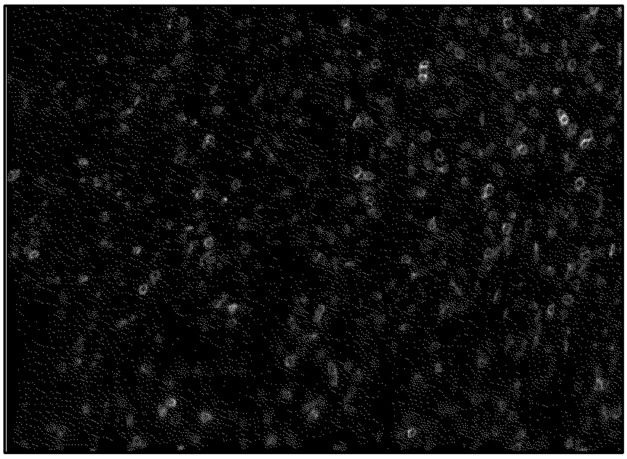

FIG. 5b

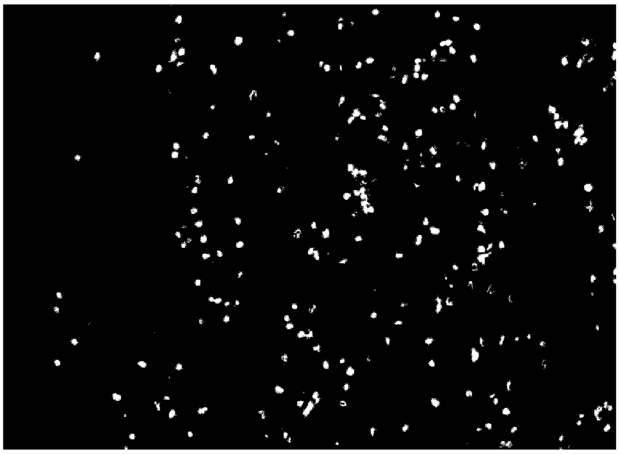

FIG. 6a

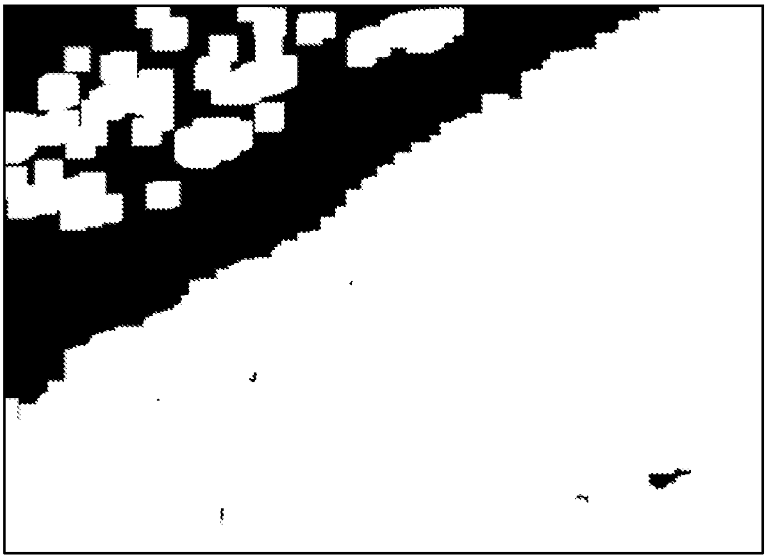

FIG. 6b

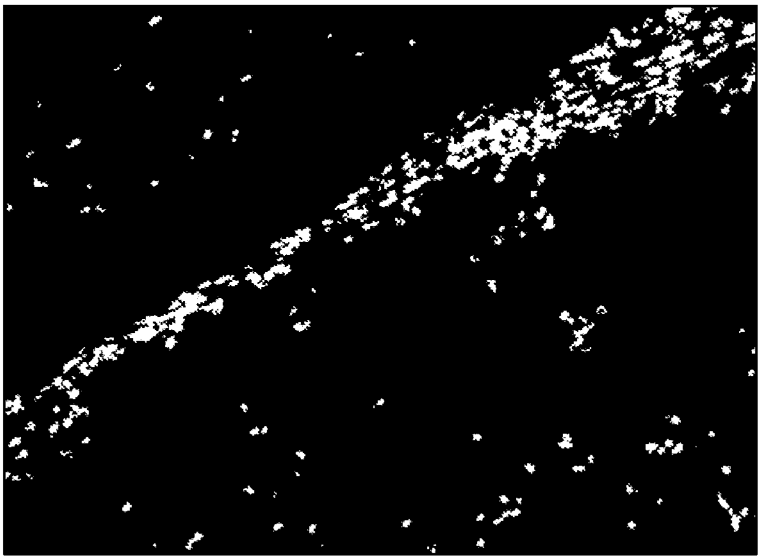

FIG. 7a

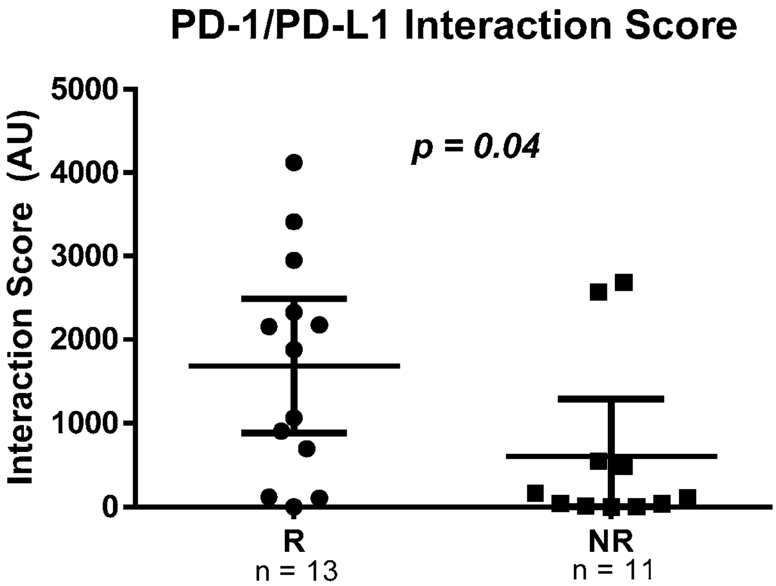
PD-1/PD-L1 Interaction Score
Interaction Score (AU)
5000
4000
3000
2000
1000
0
p = 0.04
R
n = 13
NR
n = 11

FIG. 7b

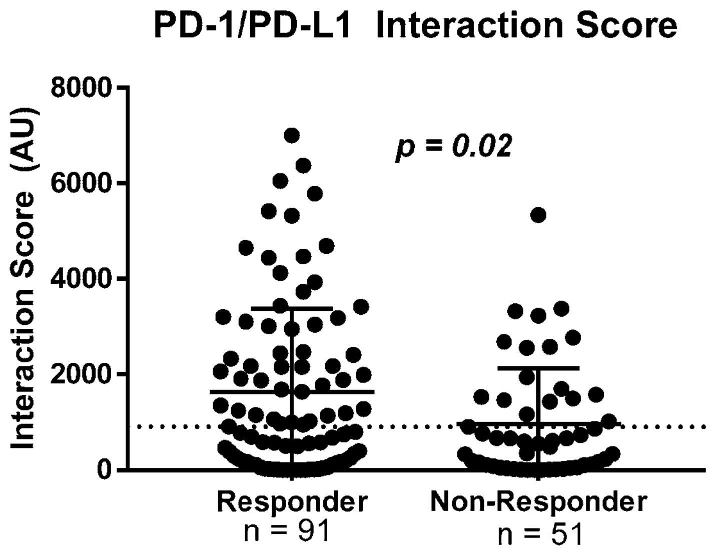
PD-1/PD-L1 Interaction Score
Interaction Score (AU)
8000
6000
4000
2000
0
p = 0.02
Responder
n = 91
Non-Responder
n = 51

FIG. 7c

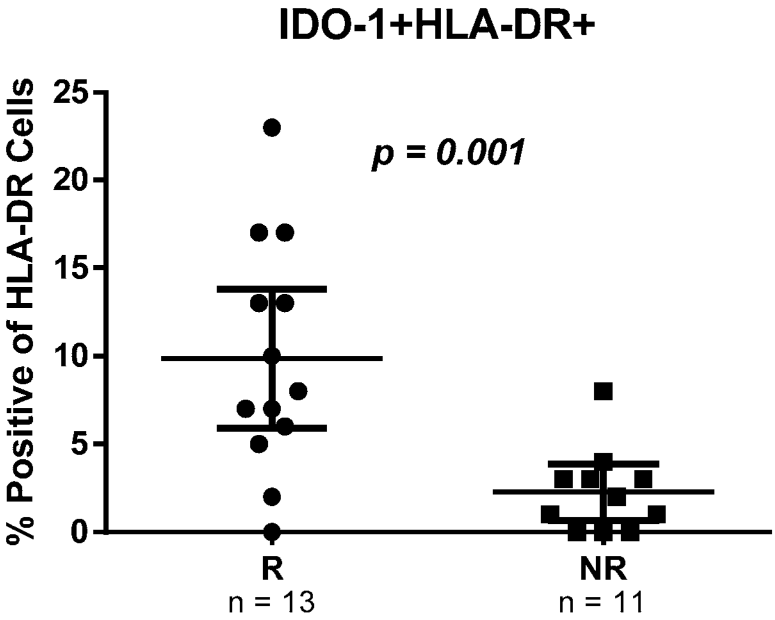
IDO-1+HLA-DR+
% Positive of HLA-DR Cells
25
20
15
10
5
0
p = 0.001
R
n = 13
NR
n = 11

FIG. 7d

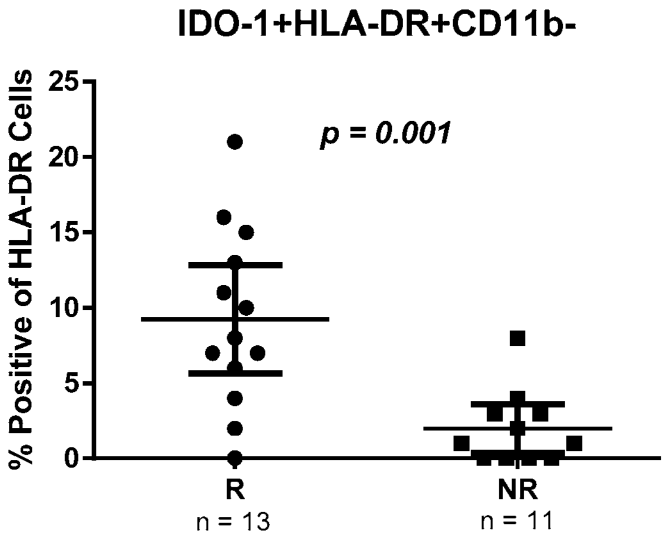
IDO-1+HLA-DR+CD11b-
% Positive of HLA-DR Cells
25
20
15
10
5
0
p = 0.001
R
n = 13
NR
n = 11

**FIG. 8a**

| | Responders | | Non-Responders | | |
|---|---|---|---|---|---|
| Phenotype | Mean | Standard Deviation | Mean | Standard Deviation | P-value |
| CD11b (of all cells) | 0.09 | 0.16 | 0.06 | 0.09 | 0.56 |
| HLA-DR (of all cells) | 0.07 | 0.05 | 0.05 | 0.06 | 0.58 |
| IDO-1 (of all cells) | 0.05 | 0.06 | 0.02 | 0.02 | 0.07 |
| CD11b+IDO-1+HLA-DR+ (of CD11b) | 0.009 | 0.008 | 0.003 | 0.005 | 0.047 |
| CD11b+DR- (of CD11b) | 0.84 | 0.16 | 0.88 | 0.11 | 0.55 |
| **IDO-1+HLA-DR+ (of HLA-DR)** | 0.10 | 0.07 | 0.02 | 0.02 | **0.0017** |
| CD11b+IDO-1+HLA-DR- (of CD11b) | 0.21 | 0.30 | 0.04 | 0.03 | 0.07 |
| CD11b+HLA-DR+ (of CD11b) | 0.16 | 0.16 | 0.12 | 0.11 | 0.55 |

**FIG. 8b**

| | **Responders** | **Non-responders** | **P-value** |
|---|---|---|---|
| Interaction Score <900 | 4 | 9 | 0.02 |
| Interaction Score ≥900 | 9 | 2 | |
| IDO-1+ HLA-DR<5% | 2 | 10 | 0.0006 |
| IDO-1+ HLA-DR ≥5% | 11 | 1 | |
| Dual Signature Negative | 1 | 9 | 0.0005 |
| Single or Dual Signature Positive | 12 | 2 | |

**FIG. 8c**

| | | **Responders** | **Non-responders** | **P-value** |
|---|---|---|---|---|
| Interaction Score | <900 | 38 | 42 | 0.06 |
| | ≥900 | 40 | 22 | |
| IDO-1+HLA-DR+ | <5% | 31 | 46 | 0.0002 |
| | ≥5% | 47 | 18 | |
| Biomarker Signature | Dual Negative | 23 | 33 | 0.0096 |
| | Single or Dual Positive | 55 | 31 | |

**FIG. 8d**

| | | **Responders** | **Non-responders** | **P-value** |
|---|---|---|---|---|
| Biomarker Signature | Dual Positive | 40 | 10 | **0.000004** |
| | Single Positive | 27 | 23 | 0.06 |
| | Dual Negative | 24 | 42 | n/a |

FIG. 9a

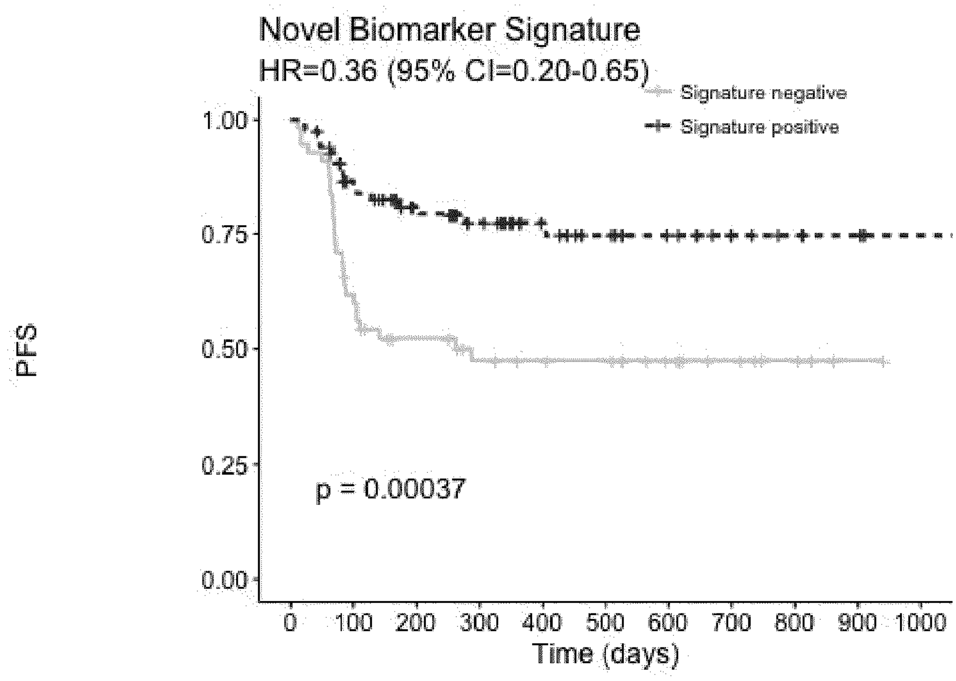
Novel Biomarker Signature
HR=0.36 (95% CI=0.20-0.65)
Signature negative
Signature positive
PFS
1.00
0.75
0.50
0.25
0.00
p = 0.00037
0
100
200
300
400
500
600
700
800
900
1000
Time (days)

FIG. 9b

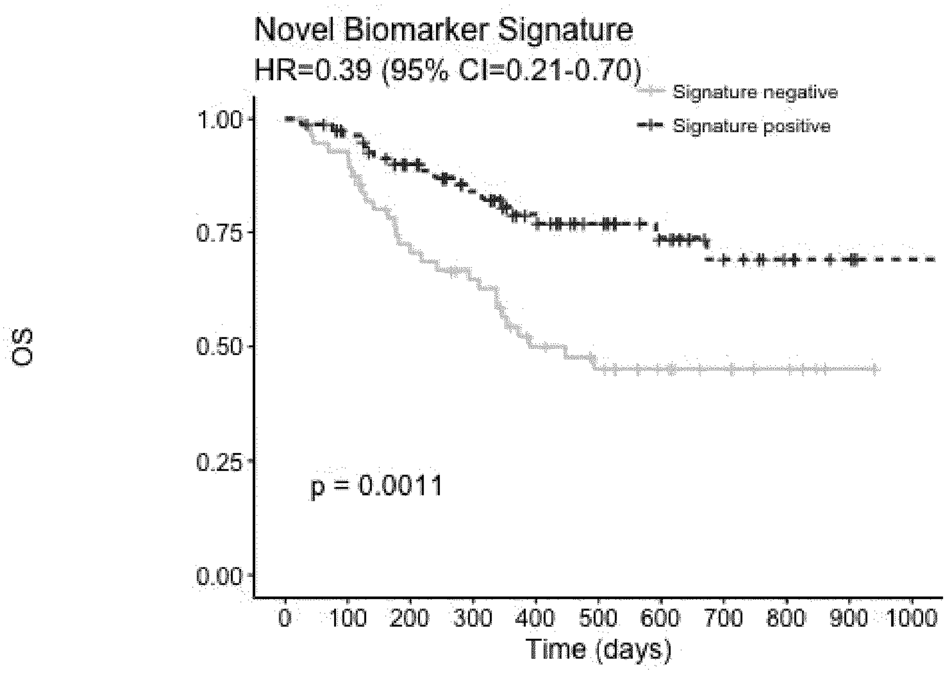
Novel Biomarker Signature
HR=0.39 (95% CI=0.21-0.70)
Signature negative
Signature positive
1.00
0.75
0.50
0.25
0.00
OS
p = 0.0011
0
100
200
300
400
500
600
700
800
900
1000
Time (days)

FIG. 10

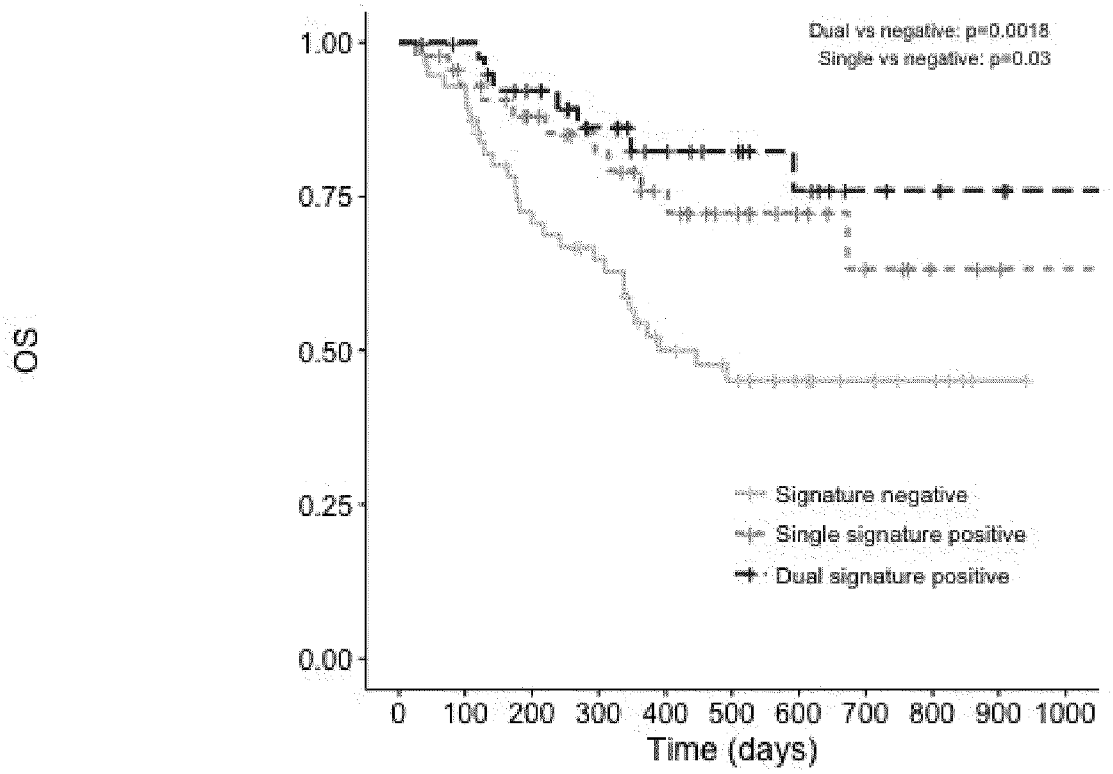
Dual vs negative: p=0.0018
Single vs negative: p=0.03
OS
1.00
0.75
0.50
0.25
0.00
0 100 200 300 400 500 600 700 800 900 1000
Time (days)
Signature negative
Single signature positive
Dual signature positive

FIG. 11

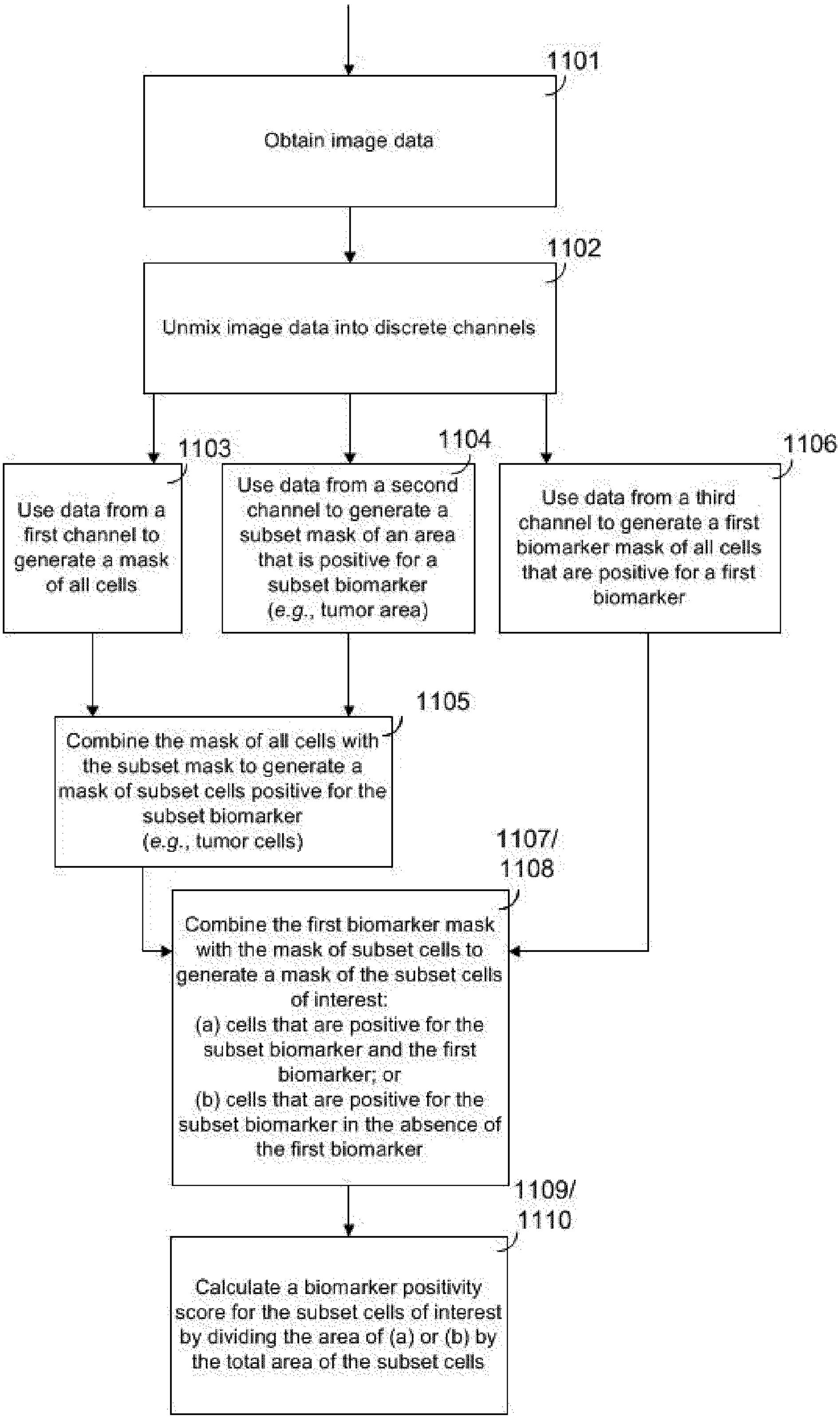
1101
Obtain image data
1102
Unmix image data into discrete channels
1103
Use data from a first channel to generate a mask of all cells
1104
Use data from a second channel to generate a subset mask of an area that is positive for a subset biomarker (e.g., tumor area)
1106
Use data from a third channel to generate a first biomarker mask of all cells that are positive for a first biomarker
1105
Combine the mask of all cells with the subset mask to generate a mask of subset cells positive for the subset biomarker (e.g., tumor cells)
1107/ 1108
Combine the first biomarker mask with the mask of subset cells to generate a mask of the subset cells of interest:
(a) cells that are positive for the subset biomarker and the first biomarker; or
(b) cells that are positive for the subset biomarker in the absence of the first biomarker
1109/ 1110
Calculate a biomarker positivity score for the subset cells of interest by dividing the area of (a) or (b) by the total area of the subset cells

FIG. 12

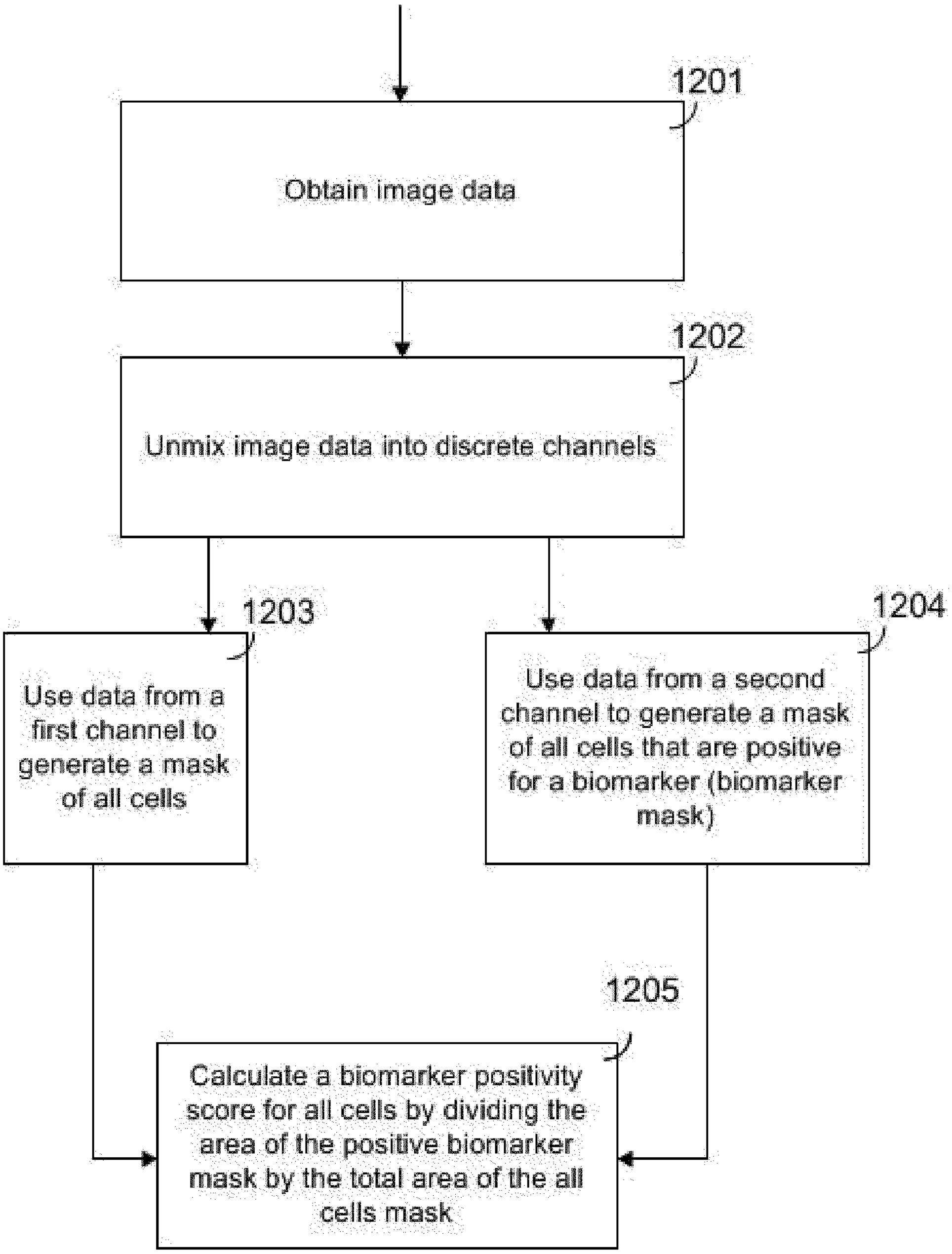
1201
Obtain image data
1202
Unmix image data into discrete channels
1203
Use data from a first channel to generate a mask of all cells
1204
Use data from a second channel to generate a mask of all cells that are positive for a biomarker (biomarker mask)
1205
Calculate a biomarker positivity score for all cells by dividing the area of the positive biomarker mask by the total area of the all cells mask

FIG. 13

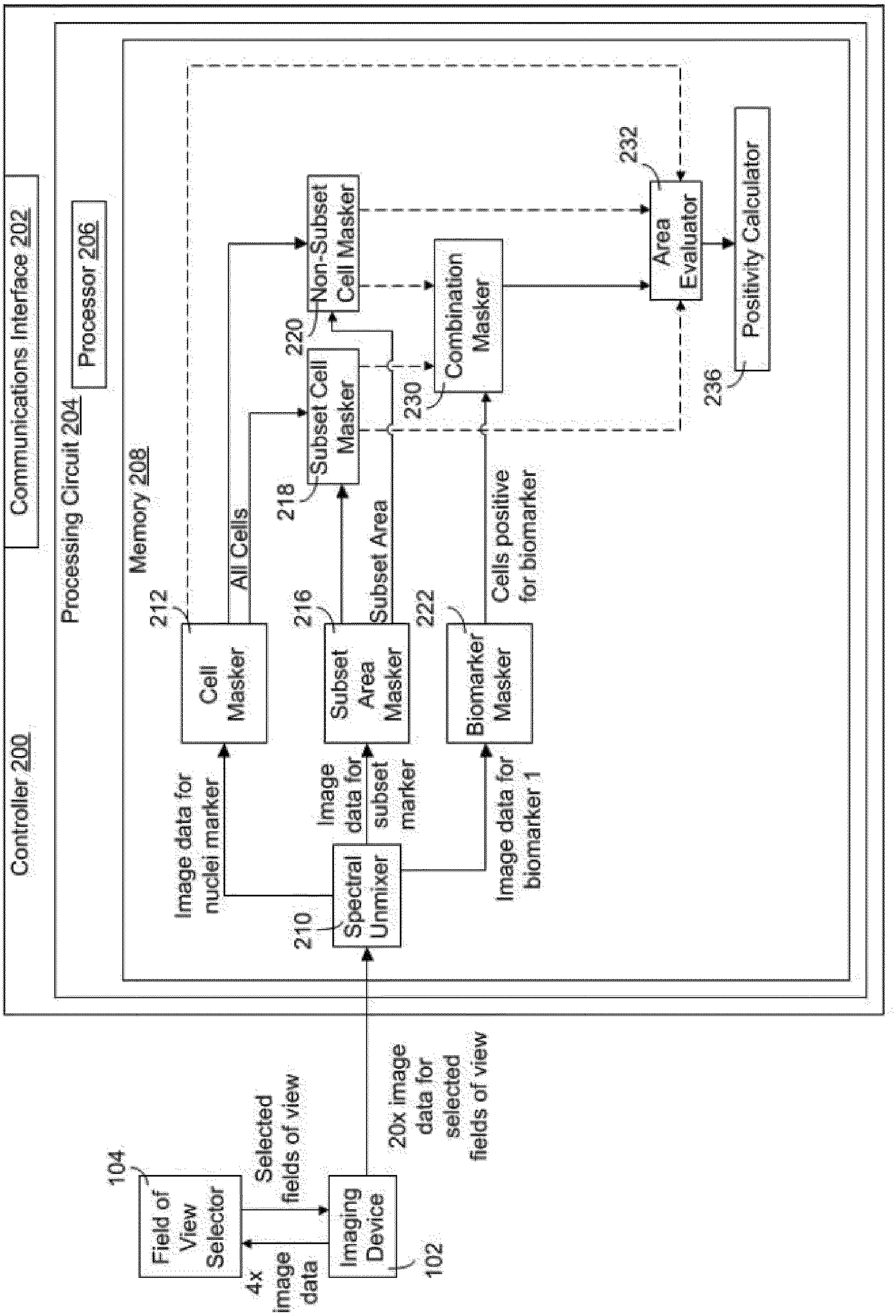
Controller 200
Communications Interface 202
Processing Circuit 204
Processor 206
Memory 208
Field of View Selector
104
4x image data
Selected fields of view
Imaging Device
102
20x image data for selected fields of view
Spectral Unmixer
210
Image data for nuclei marker
Image data for subset marker
Image data for biomarker 1
Cell Masker
212
Subset Area Masker
216
Biomarker Masker
222
All Cells
Subset Area
Cells positive for biomarker
Subset Cell Masker
218
Non-Subset Cell Masker
220
Combination Masker
230
Area Evaluator
232
Positivity Calculator
236

FIG. 14

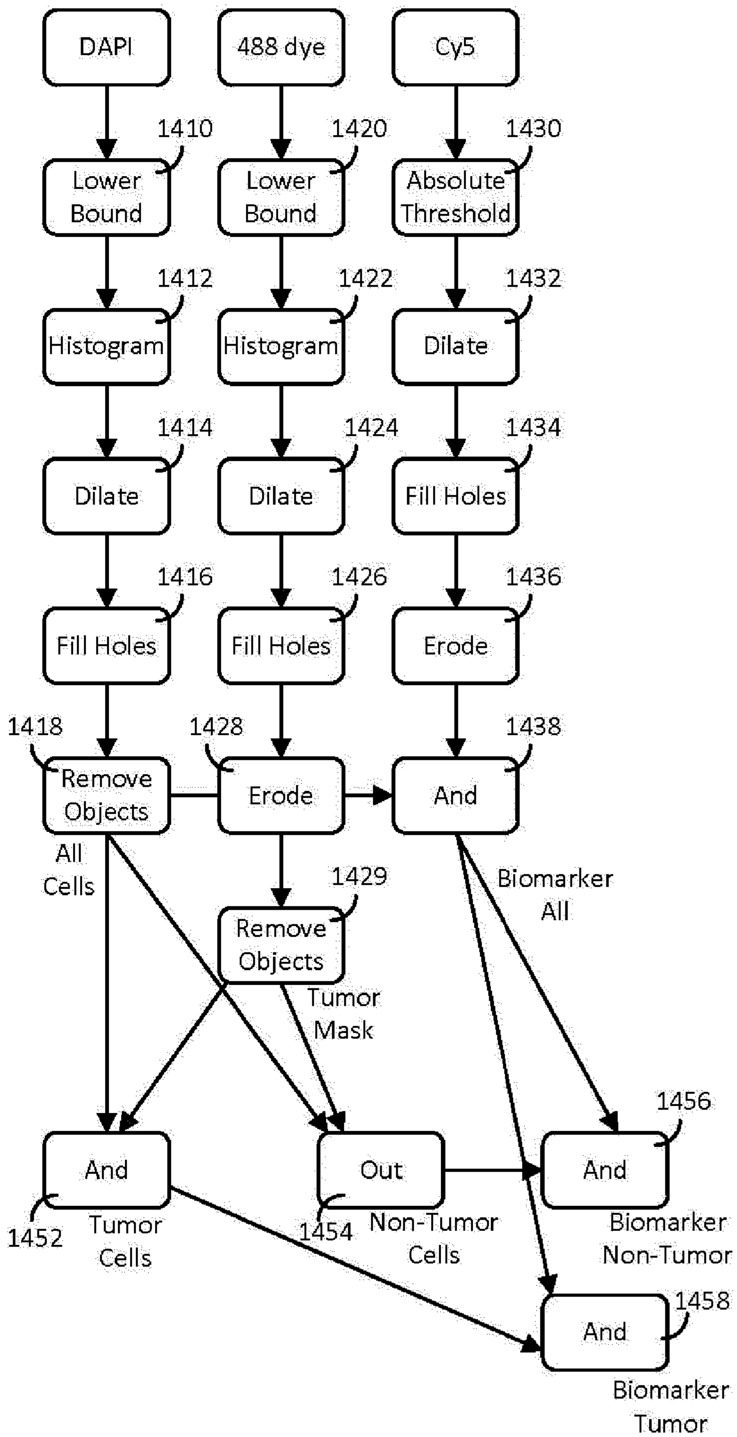
DAPI
488 dye
Cy5
1410
Lower Bound
1420
Lower Bound
1430
Absolute Threshold
1412
Histogram
1422
Histogram
1432
Dilate
1414
Dilate
1424
Dilate
1434
Fill Holes
1416
Fill Holes
1426
Fill Holes
1436
Erode
1418
Remove Objects
1428
Erode
1438
And
All Cells
1429
Remove Objects
Biomarker All
Tumor Mask
1456
And
Out
And
1452
Tumor Cells
1454
Non-Tumor Cells
Biomarker Non-Tumor
1458
And
Biomarker Tumor

FIG. 15

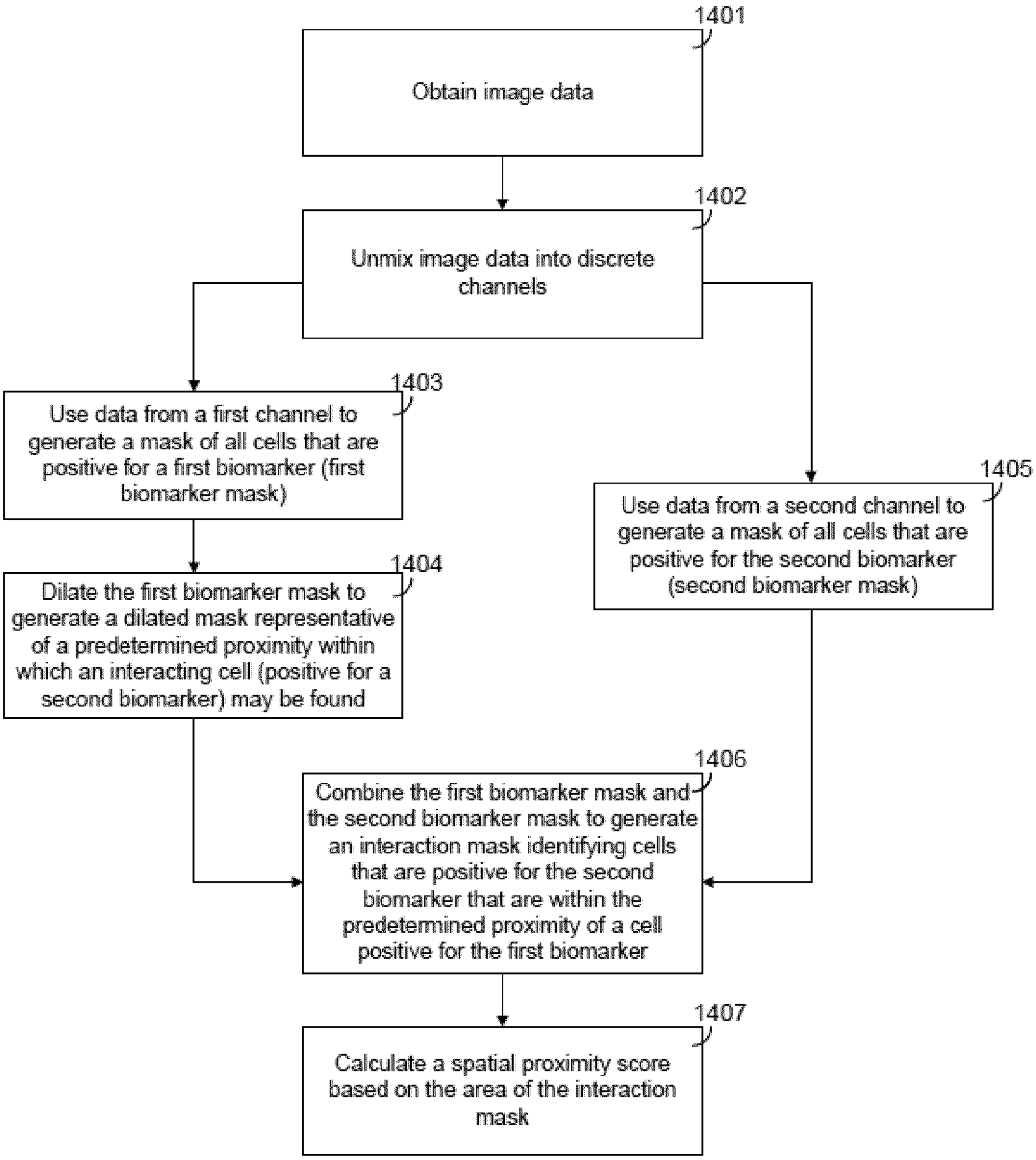
1401
Obtain image data
1402
Unmix image data into discrete channels
1403
Use data from a first channel to generate a mask of all cells that are positive for a first biomarker (first biomarker mask)
1404
Dilate the first biomarker mask to generate a dilated mask representative of a predetermined proximity within which an interacting cell (positive for a second biomarker) may be found
1405
Use data from a second channel to generate a mask of all cells that are positive for the second biomarker (second biomarker mask)
1406
Combine the first biomarker mask and the second biomarker mask to generate an interaction mask identifying cells that are positive for the second biomarker that are within the predetermined proximity of a cell positive for the first biomarker
1407
Calculate a spatial proximity score based on the area of the interaction mask

FIG. 16

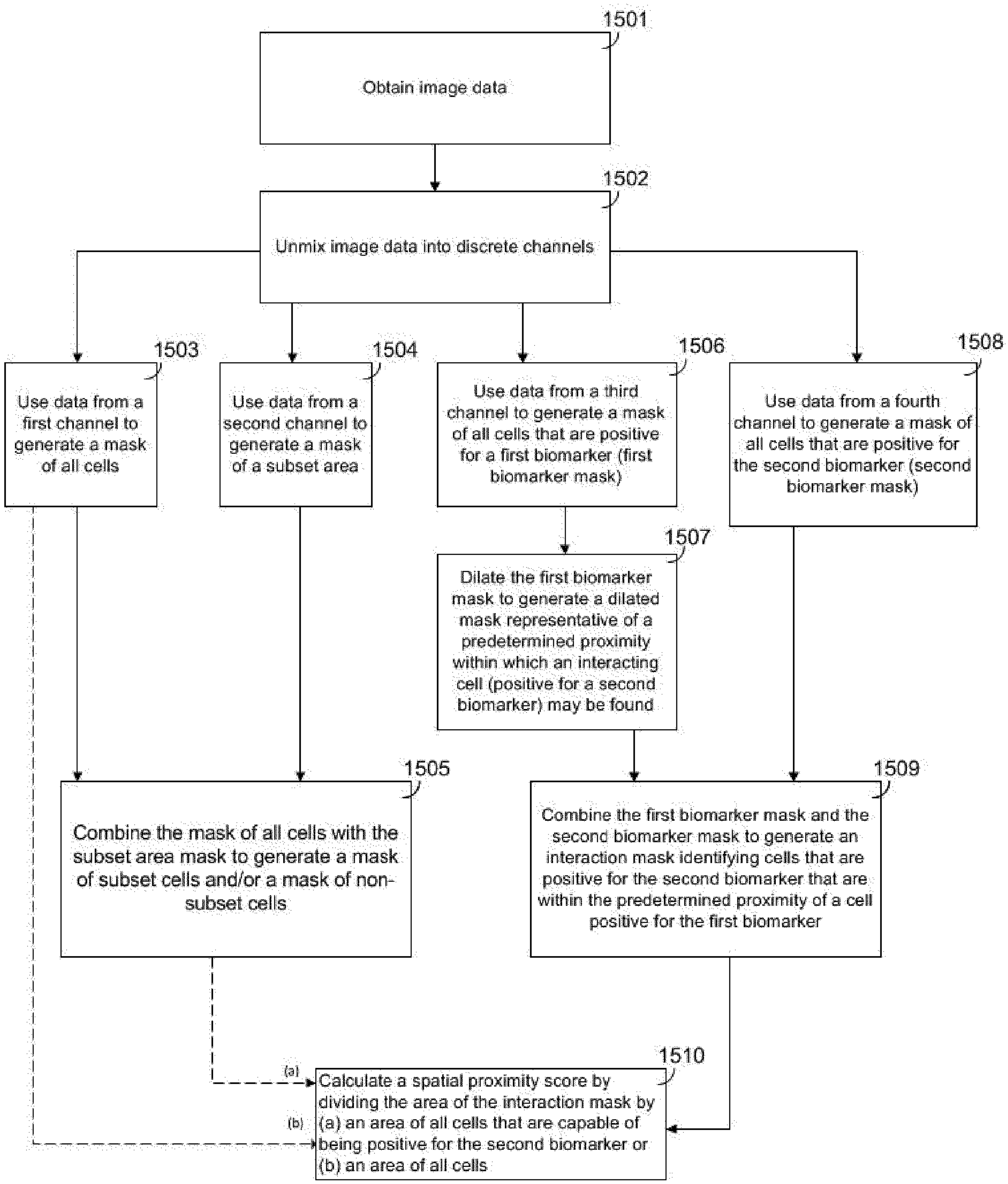
1501
Obtain image data
1502
Unmix image data into discrete channels
1503
Use data from a first channel to generate a mask of all cells
1504
Use data from a second channel to generate a mask of a subset area
1506
Use data from a third channel to generate a mask of all cells that are positive for a first biomarker (first biomarker mask)
1508
Use data from a fourth channel to generate a mask of all cells that are positive for the second biomarker (second biomarker mask)
1507
Dilate the first biomarker mask to generate a dilated mask representative of a predetermined proximity within which an interacting cell (positive for a second biomarker) may be found
1505
Combine the mask of all cells with the subset area mask to generate a mask of subset cells and/or a mask of non-subset cells
1509
Combine the first biomarker mask and the second biomarker mask to generate an interaction mask identifying cells that are positive for the second biomarker that are within the predetermined proximity of a cell positive for the first biomarker
1510
(a)
(b)
Calculate a spatial proximity score by dividing the area of the interaction mask by (a) an area of all cells that are capable of being positive for the second biomarker or (b) an area of all cells

**FIG. 17**

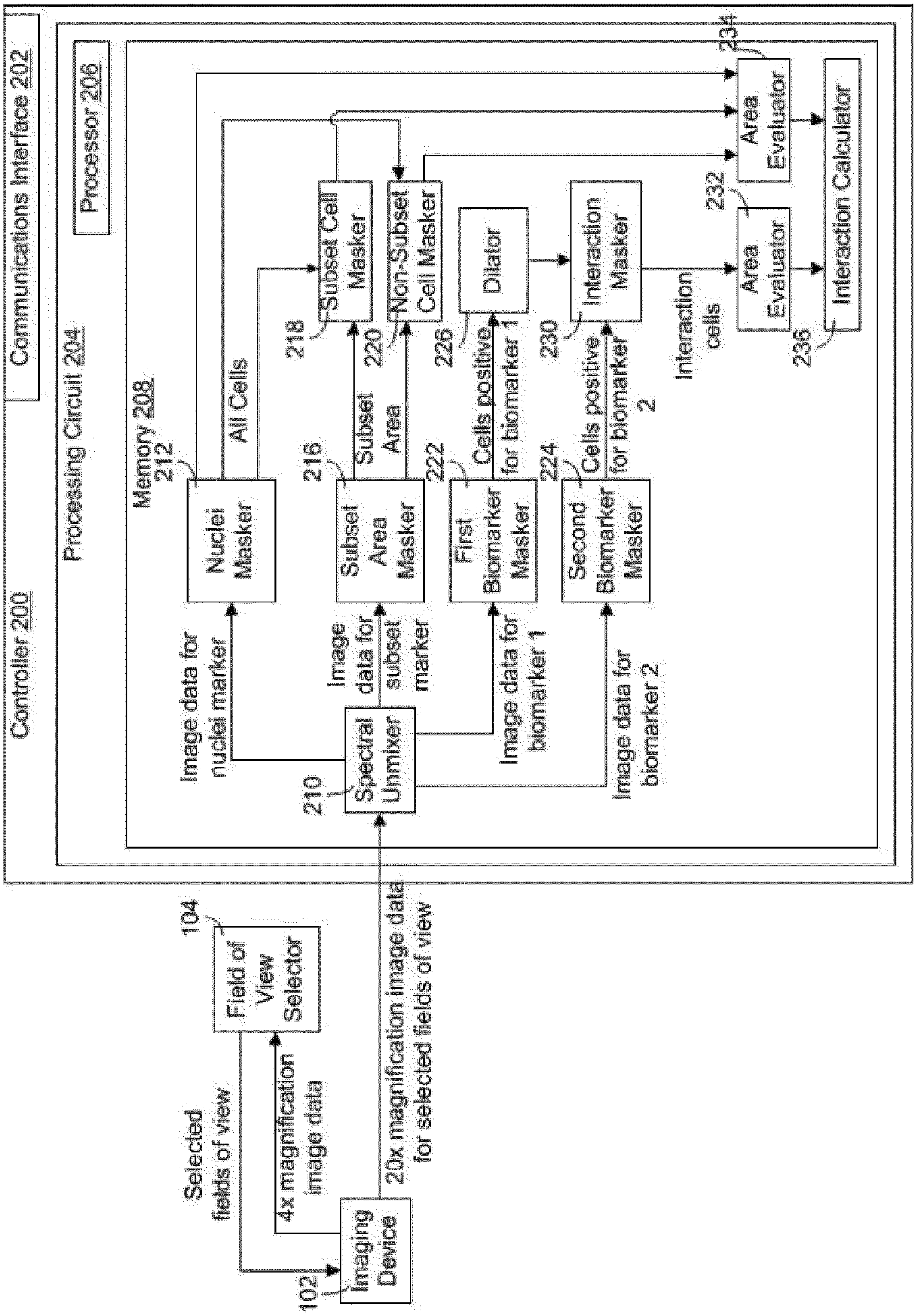
Controller 200
Communications Interface 202
Processing Circuit 204
Processor 206
Memory 208
Spectral Unmixer
210
Image data for nuclei marker
Image data for subset marker
Image data for biomarker 1
Image data for biomarker 2
Nuclei Masker
212
Subset Area Masker
216
First Biomarker Masker
222
Second Biomarker Masker
224
All Cells
Subset Area
Cells positive for biomarker 1
Cells positive for biomarker 2
Subset Cell Masker
218
Non-Subset Cell Masker
220
Dilator
226
Interaction Masker
230
Interaction cells
Area Evaluator
232
Area Evaluator
234
Interaction Calculator
236
Field of View Selector
104
Imaging Device
102
Selected fields of view
4x magnification image data
20x magnification image data for selected fields of view

FIG. 18

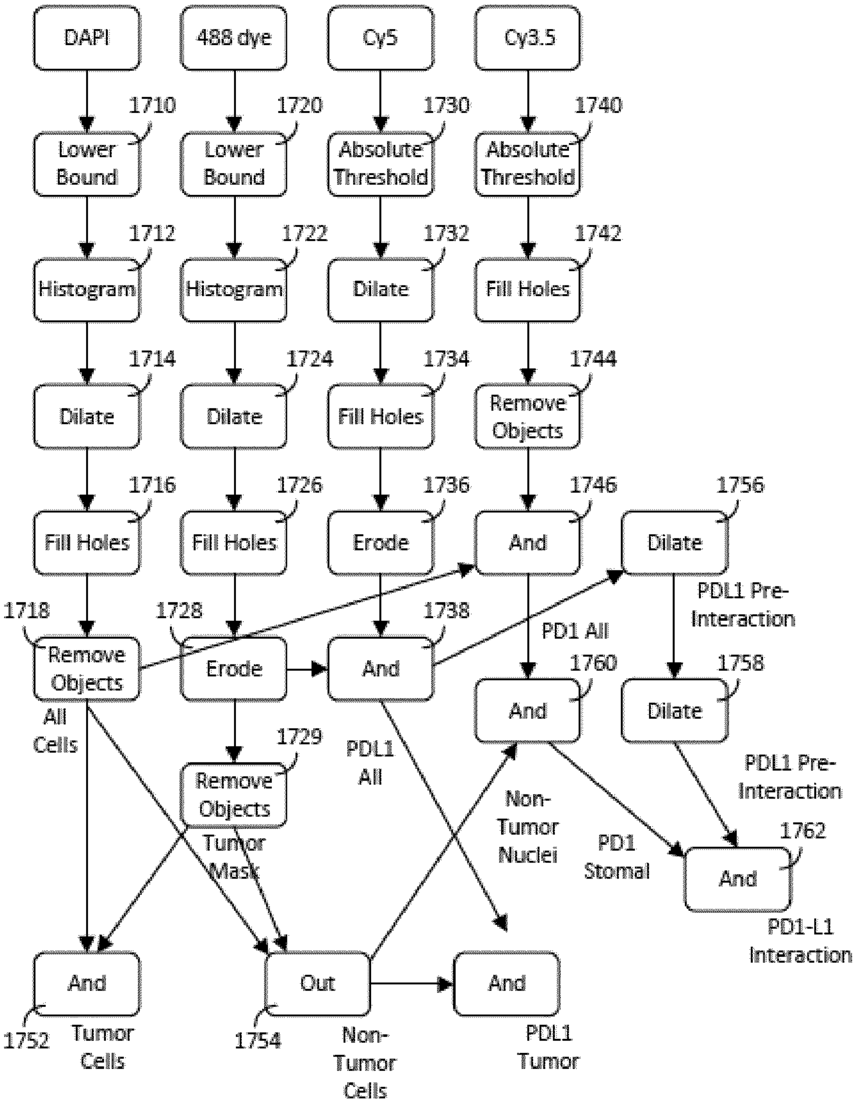
DAPI
488 dye
Cy5
Cy3.5
1710
1720
1730
1740
Lower Bound
Lower Bound
Absolute Threshold
Absolute Threshold
1712
1722
1732
1742
Histogram
Histogram
Dilate
Fill Holes
1714
1724
1734
1744
Dilate
Dilate
Fill Holes
Remove Objects
1716
1726
1736
1746
1756
Fill Holes
Fill Holes
Erode
And
Dilate
PDL1 Pre-Interaction
1718
1728
1738
PD1 All
1760
1758
Remove Objects
Erode
And
And
Dilate
All Cells
1729
PDL1 All
PDL1 Pre-Interaction
Remove Objects
Non-Tumor Nuclei
1762
Tumor Mask
PD1 Stomal
And
PD1-L1 Interaction
And
Out
And
1752
Tumor Cells
1754
Non-Tumor Cells
PDL1 Tumor

FIG. 19

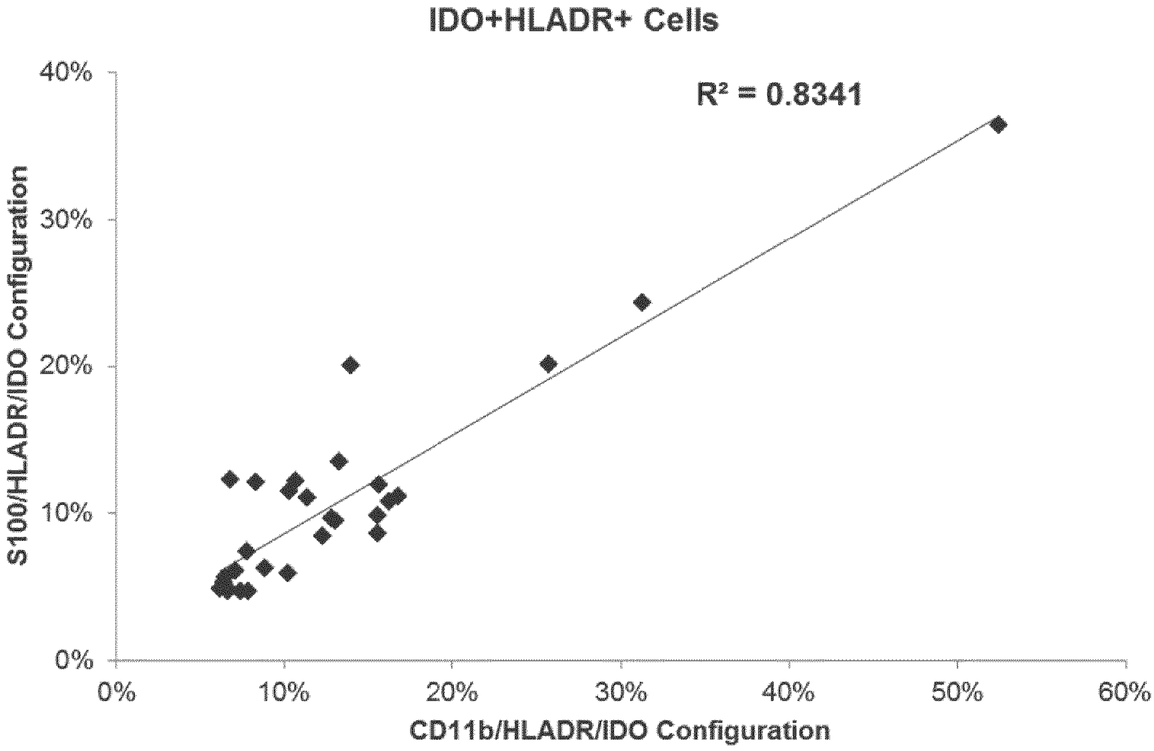
IDO+HLADR+ Cells
R² = 0.8341
40%
30%
20%
10%
0%
0%
10%
20%
30%
40%
50%
60%
S100/HLADR/IDO Configuration
CD11b/HLADR/IDO Configuration

**FIG. 20a**

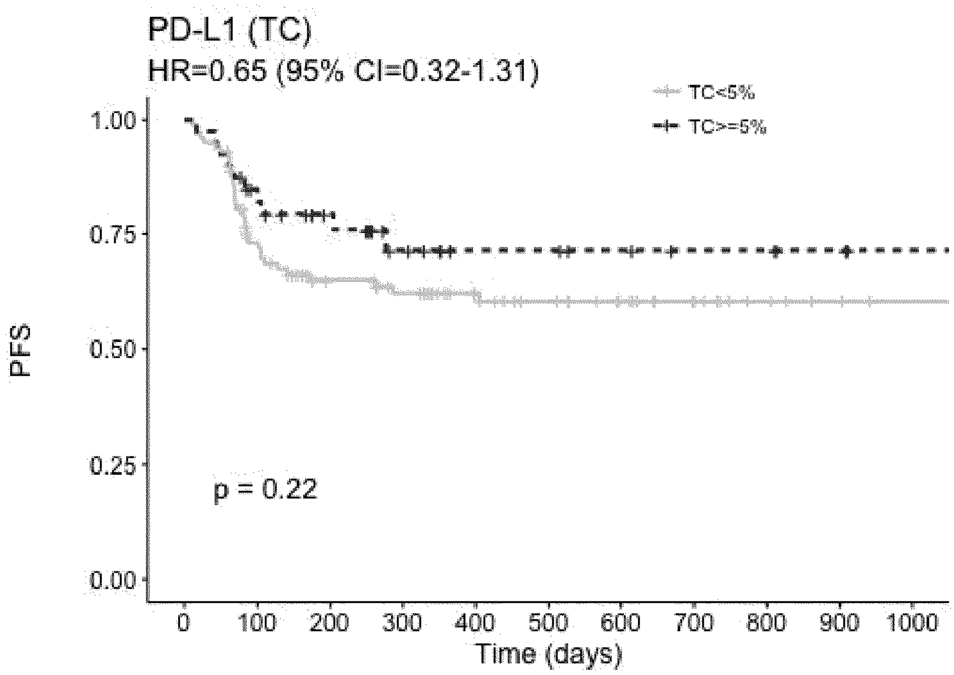
PD-L1 (TC)
HR=0.65 (95% CI=0.32-1.31)
TC<5%
TC>=5%
PFS
1.00
0.75
0.50
0.25
0.00
p = 0.22
0
100
200
300
400
500
600
700
800
900
1000
Time (days)

FIG. 20b

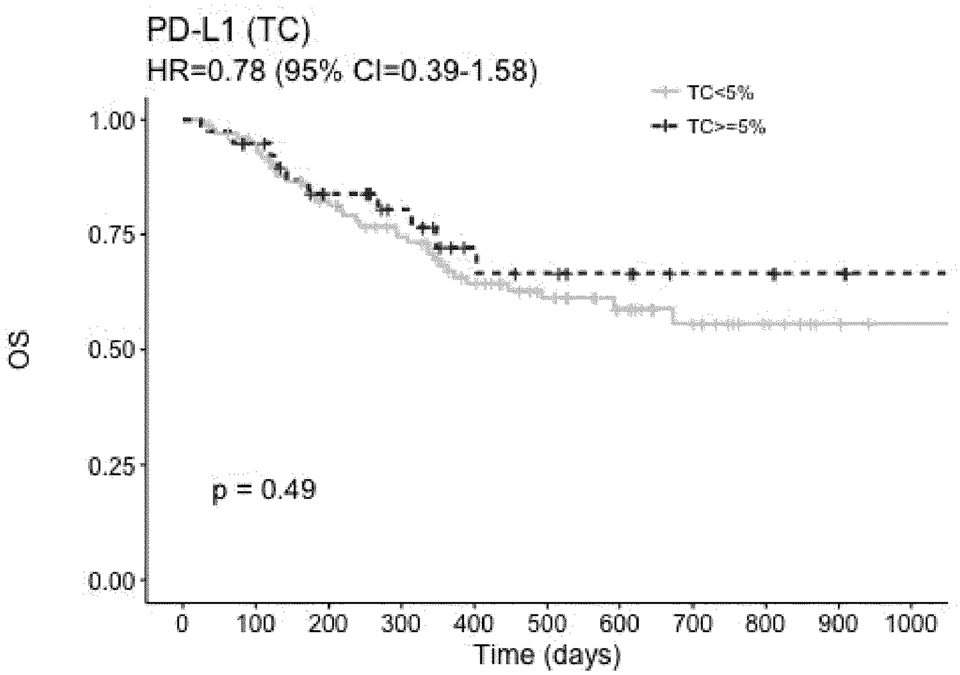
PD-L1 (TC)
HR=0.78 (95% CI=0.39-1.58)
TC<5%
TC>=5%
1.00
0.75
0.50
0.25
0.00
OS
p = 0.49
0
100
200
300
400
500
600
700
800
900
1000
Time (days)

FIG. 21a

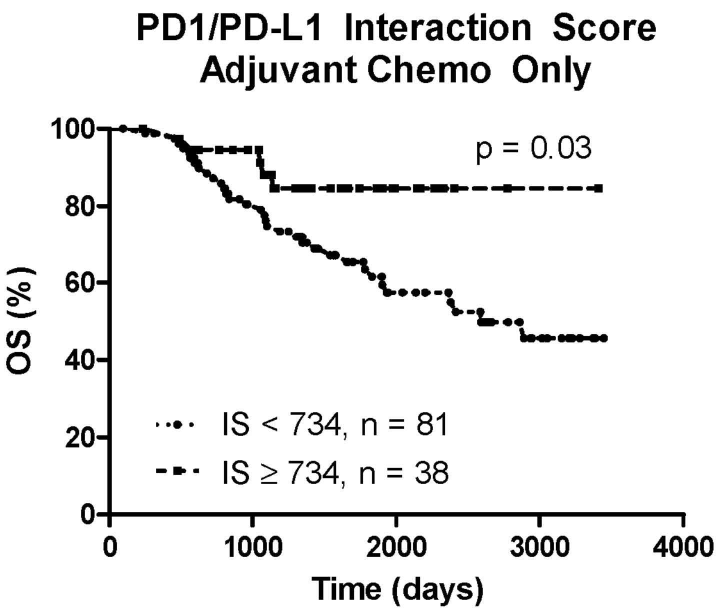


FIG. 21b

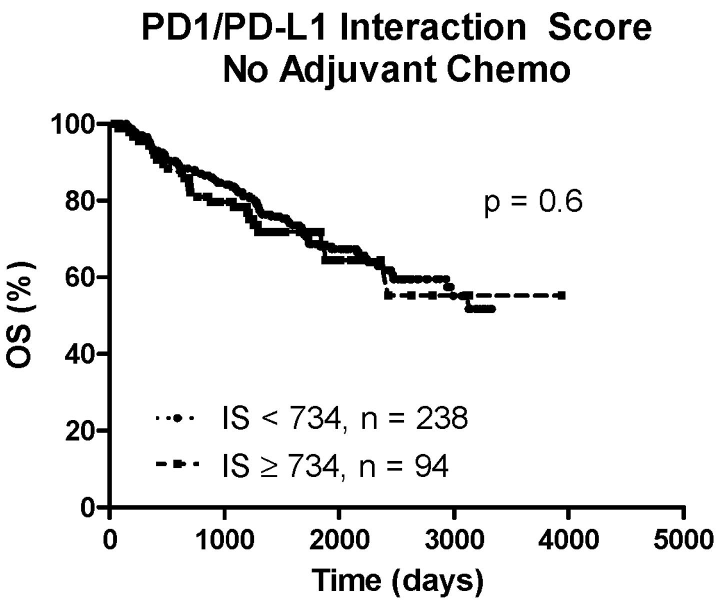

FIG. 22

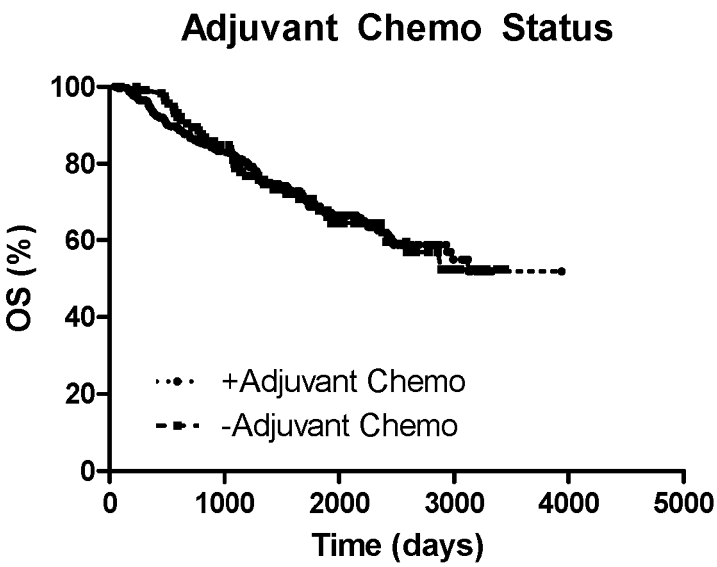
Adjuvant Chemo Status
100
80
60
40
20
0
OS (%)
+Adjuvant Chemo
-Adjuvant Chemo
0
1000
2000
3000
4000
5000
Time (days)

FIG. 23a

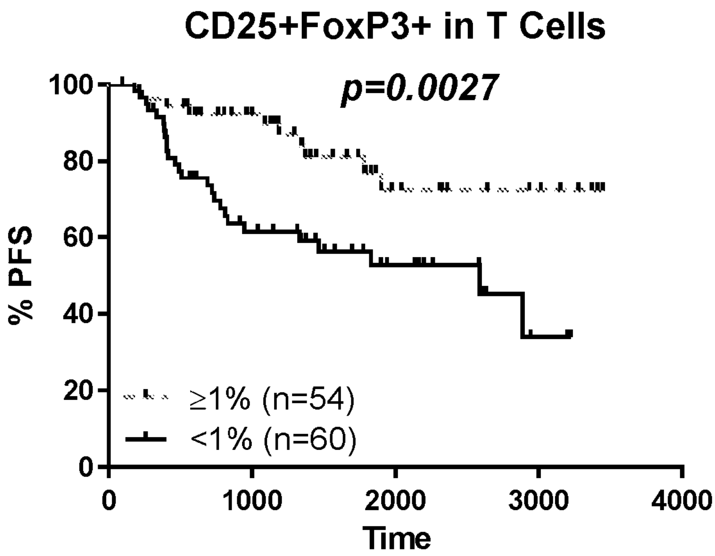


FIG.23b

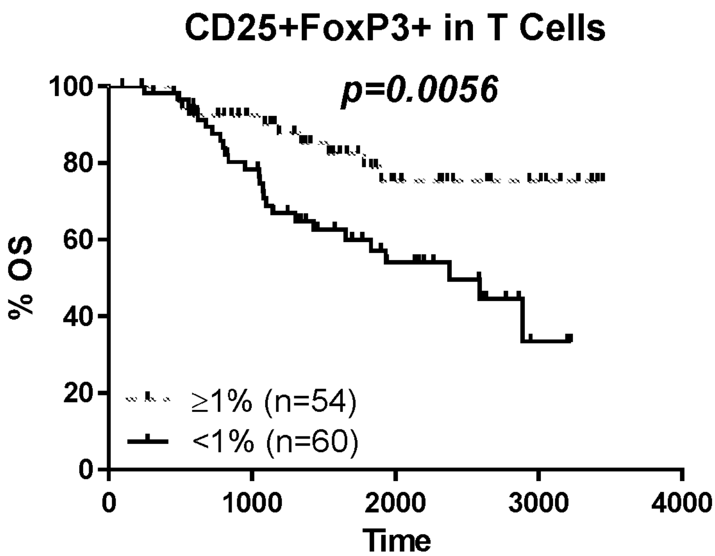

FIG. 24a

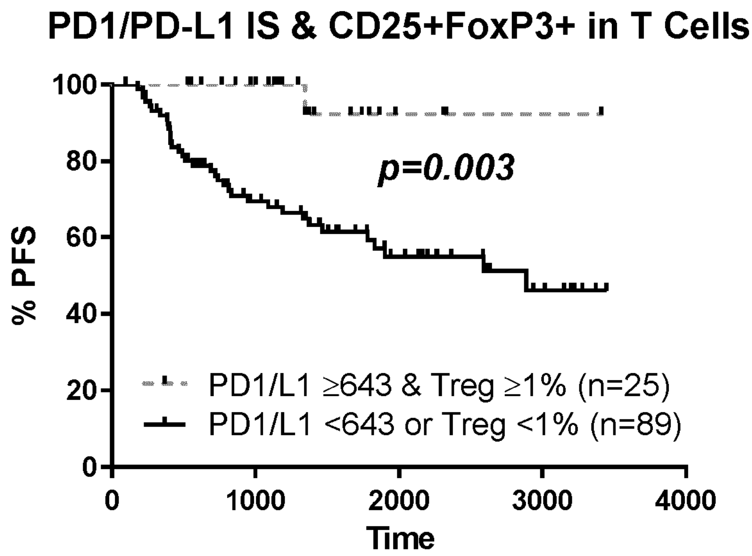


FIG. 24b

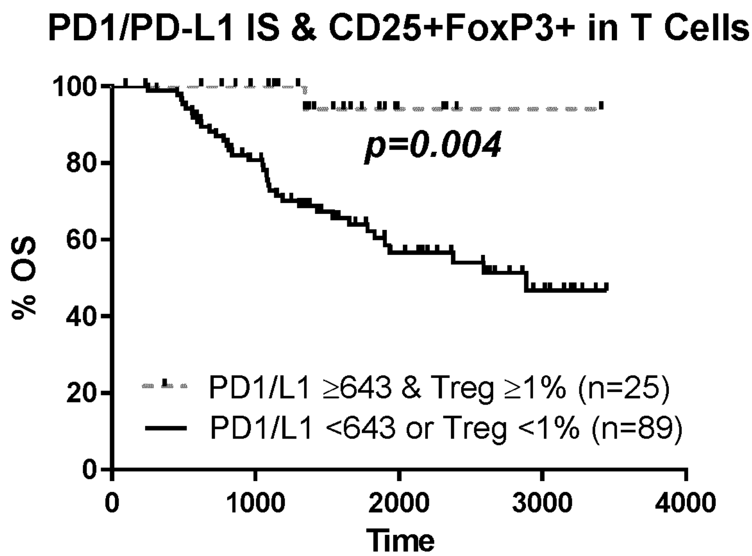

FIG. 25a

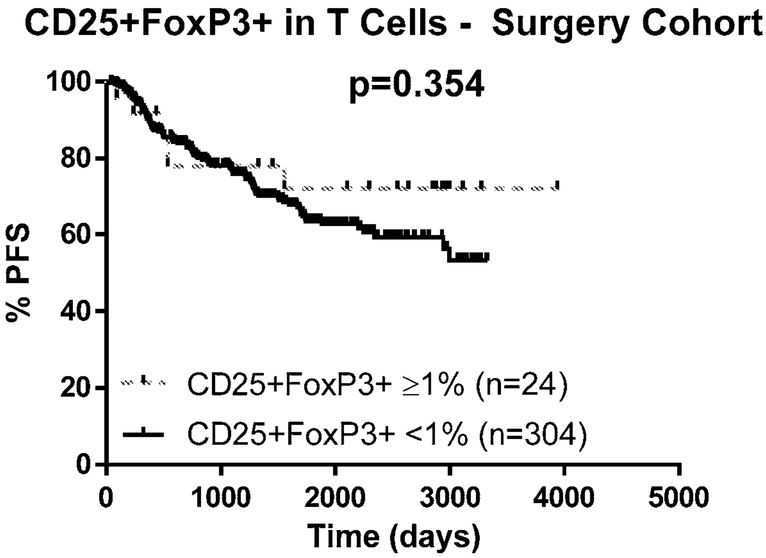


FIG. 25b

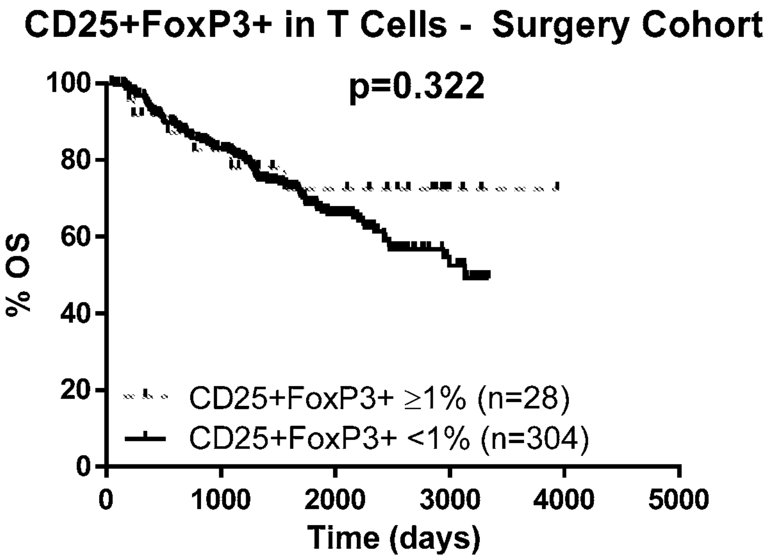

FIG. 26a

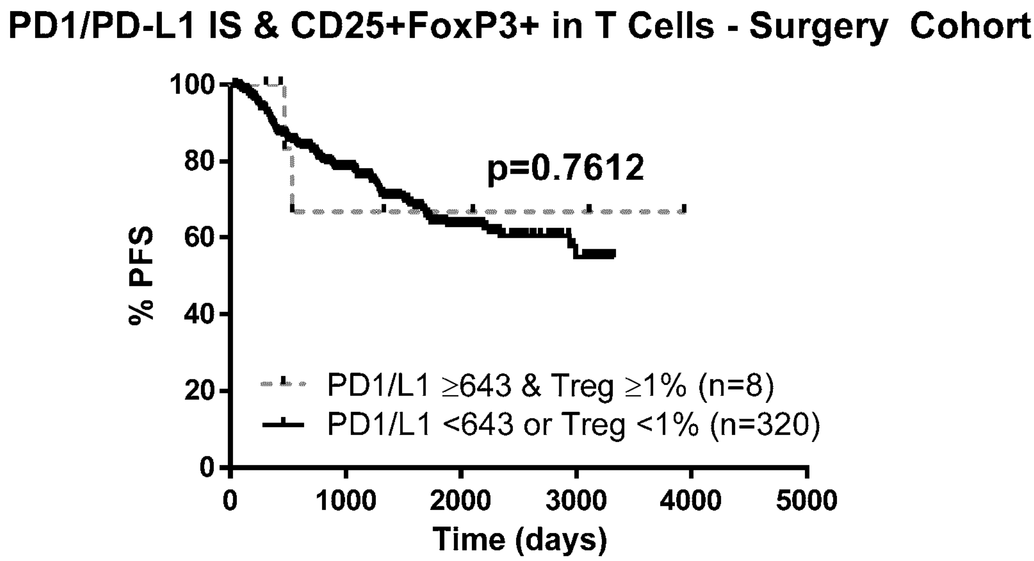


FIG. 26b

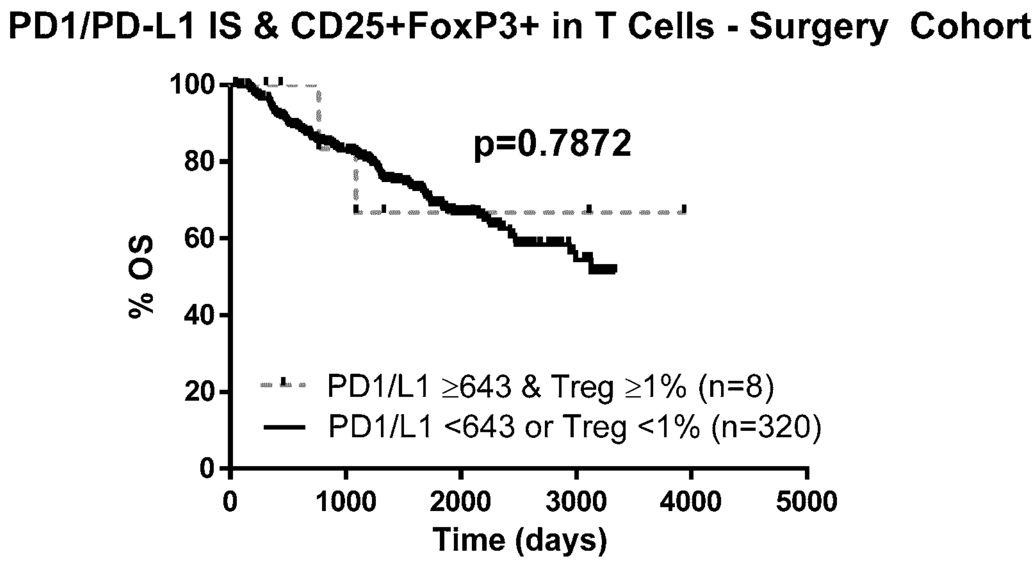

FIG. 27a

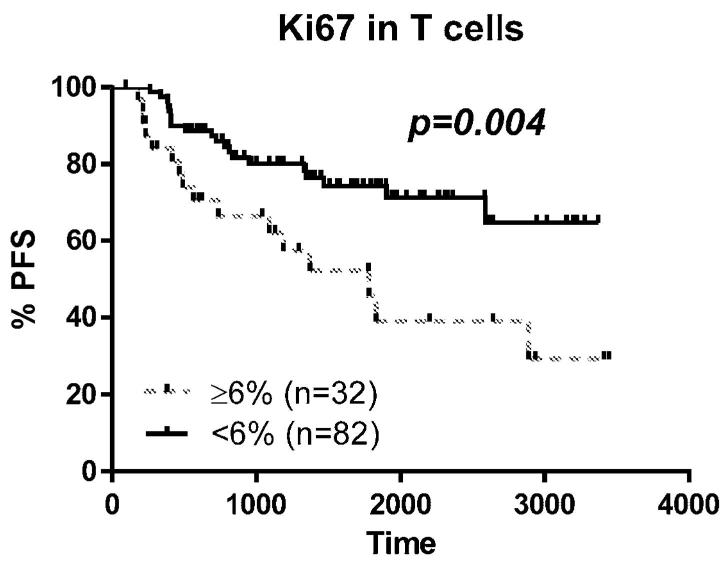


FIG. 27b

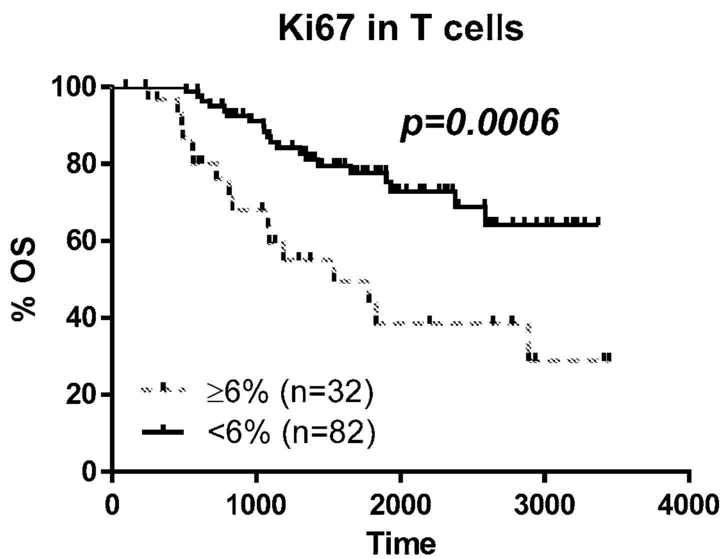

FIG. 28a

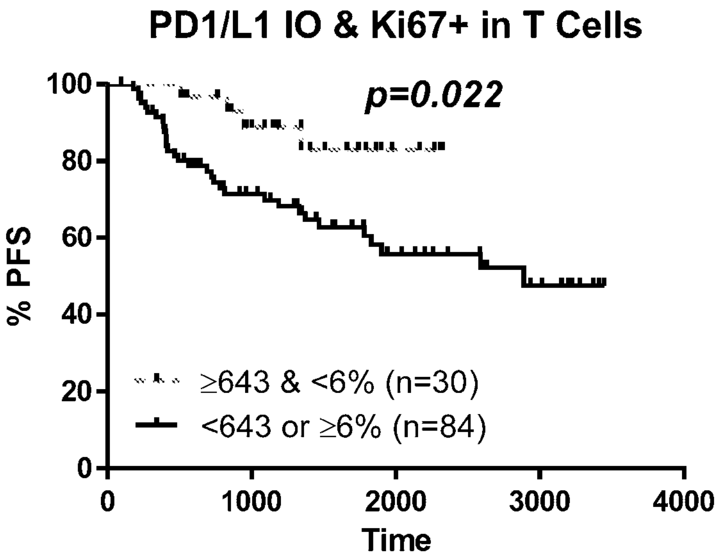


FIG.28b

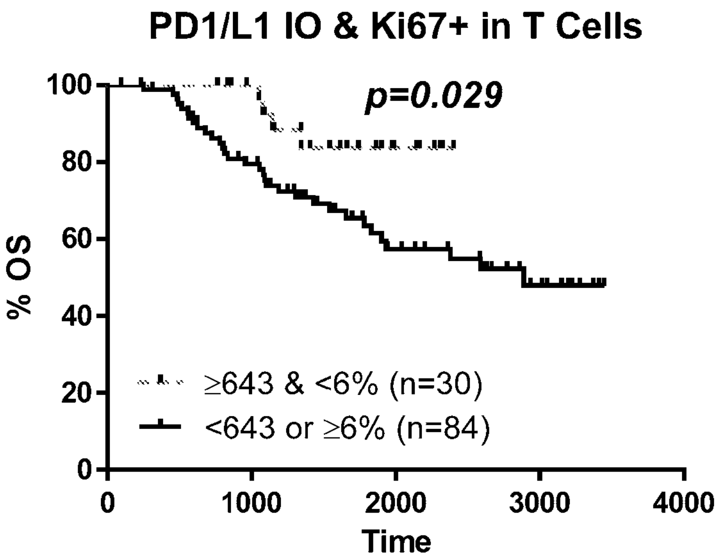

FIG. 29a

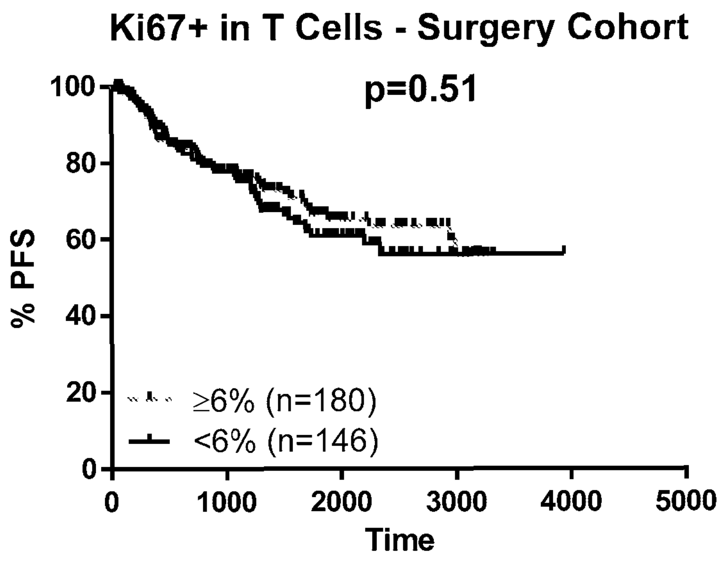


FIG. 29b

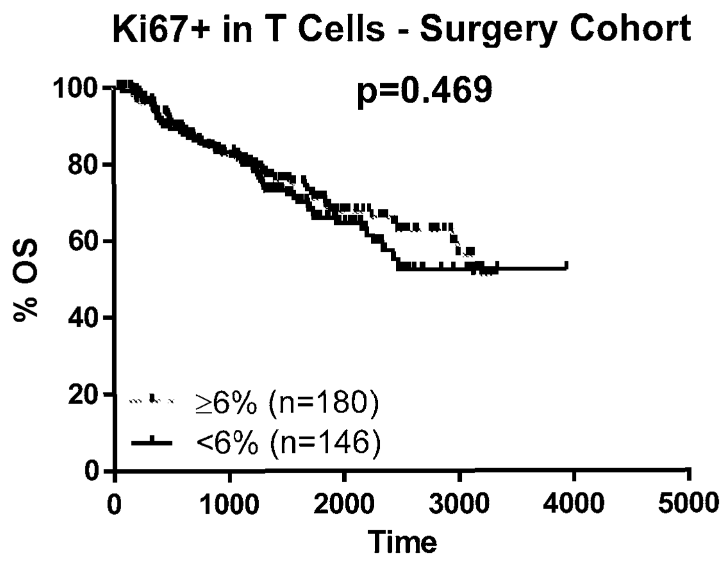

FIG. 30a

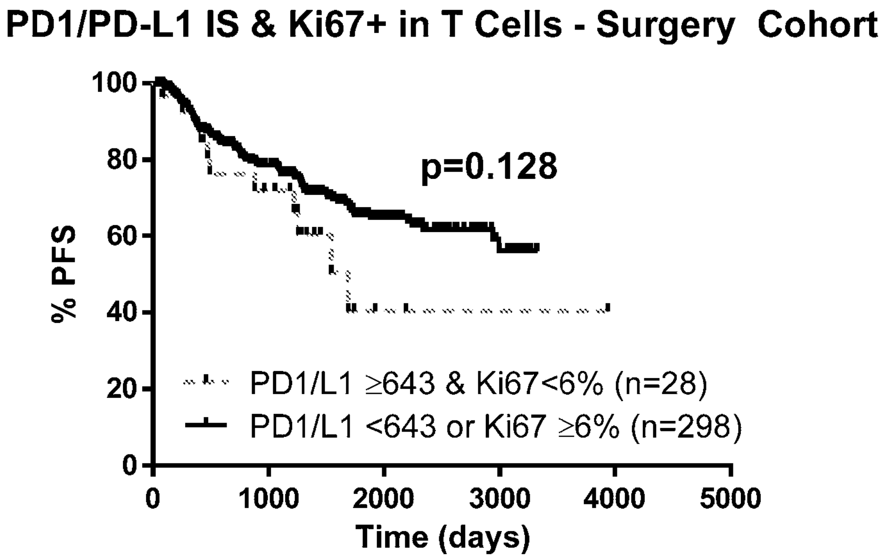


FIG. 30b

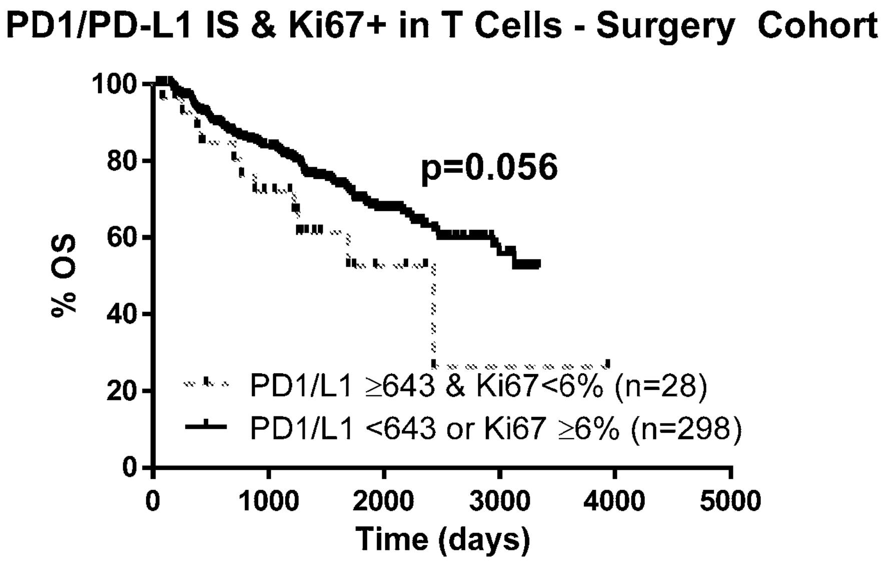

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62455337
- US 62591057
- WO 2016073760 A1
- US 2016058277 W
- US 2016058281 W